# EUROPEAN PATENT APPLICATION

(11) **EP 3 753 932 A1**
(43) Date of publication of application: **23.12.2020**
(21) Application number: 19382498.4
(22) Date of filing: 17.06.2019
(51) Int. Cl.: C07D 401/10, C07D 401/14, A61K 31/496, A61P 29/00

(54) **SUBSTITUTED BICYCLIC DERIVATIVES HAVING MULTIMODAL ACTIVITY AGAINST PAIN**

(71) Applicant: ESTEVE PHARMACEUTICALS, S.A., 08038 Barcelona (ES)
(72) Inventor: ALMANSA-ROSALES, Carmen, 08022 BARCELONA (ES); DIAZ-FERNANDEZ, Jose Luis, 08242 MANRESA (ES); LORENTE-CRIVILLE, Adriana, 08015 Barcelona (ES)

(57) **Abstract**

The present invention relates to substituted bicyclic derivatives (I) having dual pharmacological activity towards both the α2δ subunit, in particular the α2δ-1 subunit, of the voltage-gated calcium channel and the µ-opioid receptor, to processes of preparation of such compounds, to pharmaceutical compositions comprising them, and to their use in therapy, in particular for the treatment of pain.

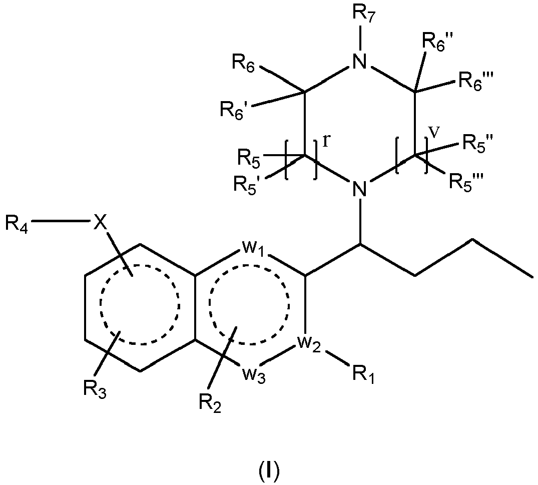

## Description

### FIELD OF THE INVENTION

The present invention relates to compounds having dual pharmacological activity towards both the α₂δ subunit of the voltage-gated calcium channel, and the µ-opioid receptor (MOR or mu-opioid receptor) and more particularly to substituted bicyclic derivatives having this pharmacological activity, to processes of preparation of such compounds, to pharmaceutical compositions comprising them, and to their use in therapy, in particular for the treatment of pain.

### BACKGROUND OF THE INVENTION

The adequate management of pain constitutes an important challenge, since currently available treatments provide in many cases only modest improvements, leaving many patients unrelieved (Turk, D.C., Wilson, H.D., Cahana, A.; 2011; Lancet; 377; 2226-2235). Pain affects a big portion of the population with an estimated prevalence of 20 % and its incidence, particularly in the case of chronic pain, is increasing due to the population ageing. Additionally, pain is clearly related to comorbidities, such as depression, anxiety and insomnia, which leads to important productivity losses and socio-economical burden (Goldberg, D.S., McGee, S.J.; 2011; BMC Public Health; 11; 770). Existing pain therapies include non-steroidal anti-inflammatory drugs (NSAIDs), opioid agonists, calcium channel blockers and antidepressants, but they are much less than optimal regarding their safety ratio. All of them show limited efficacy and a range of secondary effects that preclude their use, especially in chronic settings.

Voltage-gated calcium channels (VGCC) are required for many key functions in the body. Different subtypes of voltage-gated calcium channels have been described (Zamponi et al., Pharmacol Rev. 2015 67:821-70). The VGCC are assembled through interactions of different subunits, namely α₁ (Caᵥα₁), β (Caᵥβ) α₂δ (Caᵥα₂δ) and γ (Caᵥγ). The α₁ subunits are the key porous forming units of the channel complex, being responsible for the Ca²⁺ conduction and generation of Ca²⁺ influx. The α₂δ, β, and γ subunits are auxiliary, although very important for the regulation of the channel, since they increase the expression of the α₁ subunits in the plasma membrane as well as modulate their function, resulting in functional diversity in different cell types. Based on their physiological and pharmacological properties, VGCC can be subdivided into low voltage-activated T-type (Caᵥ3.1, Caᵥ3.2, and Caᵥ3.3), and high voltage-activated L-(Caᵥ1.1 through Caᵥ1.4), N-(Caᵥ2.2), P/Q-(Caᵥ2.1), and R-(Caᵥ2.3) types, depending on the channel forming Cava subunits. All of these five subclasses are found in the central and peripheral nervous systems. Regulation of intracellular calcium through activation of these VGCC plays obligatory roles in: 1) neurotransmitter release, 2) membrane depolarization and hyperpolarization, 3) enzyme activation and inactivation, and 4) gene regulation (Perret and Luo, Neurotherapeutics. 2009 6:679-92; Zamponi et al., 2015 *supra;* Neumaier et al., Prog Neurobiol. 2015 129:1-36.). A large body of data has clearly indicated that VGCC are implicated in mediating various disease states including pain processing. Drugs interacting with the different calcium channel subtypes and subunits have been developed. Current therapeutic agents include drugs targeting L-type Caᵥ1.2 calcium channels, particularly 1,4-dihydropyridines, which are widely used in the treatment of hypertension. T-type (Caᵥ3) channels are the target of ethosuximide, widely used in absence epilepsy. Ziconotide, a peptide blocker of N-type (Caᵥ2.2) calcium channels, has been approved as a treatment of intractable pain. (Perret and Luo, 2009, *supra;* Vink and Alewood, Br J Pharmacol. 2012 167:970-89.).

The Caᵥ1 and Caᵥ2 subfamilies contain an auxiliary α₂δ subunit, which is the therapeutic target of the gabapentinoid drugs of value in certain epilepsies and chronic neuropathic pain. To date, there are four known α₂δ subunits, each encoded by a unique gene and all possessing splice variants. Each α₂δ protein is encoded by a single messenger RNA and is post-translationally cleaved and then linked by disulfide bonds. Four genes encoding α₂δ subunits have now been cloned. α₂δ-1 was initially cloned from skeletal muscle and shows a fairly ubiquitous distribution. The α₂δ-2 and α₂δ-3 subunits were subsequently cloned from brain. The most recently identified subunit, α₂δ-4, is largely non-neuronal. The human α₂δ-4 protein sequence shares 30, 32 and 61% identity with the human α₂δ-1, α₂δ-2 and α₂δ-3 subunits, respectively. The gene structure of all α₂δ subunits is similar. All α₂δ subunits show several splice variants (Davies et al., Trends Pharmacol Sci. 2007 28:220-8.; Dolphin AC, Nat Rev Neurosci. 2012 13:542-55., Biochim Biophys Acta. 2013 1828:1541-9.).

The Caᵥα₂δ-1 subunit may play an important role in neuropathic pain development (Perret and Luo, 2009, *supra;* Vink and Alewood, 2012, *supra).* Biochemical data have indicated a significant Caᵥα₂δ-1, but not Caᵥα₂δ-2, subunit upregulation in the spinal dorsal horn, and DRG (dorsal root ganglia) after nerve injury that correlates with neuropathic pain development. In addition, blocking axonal transport of injury-induced DRG Caᵥα₂δ-1 subunit to the central presynaptic terminals diminishes tactile allodynia in nerve injured animals, suggesting that elevated DRG Caᵥα₂δ-1 subunit contributes to neuropathic allodynia.

The Caᵥα₂δ-1 subunit (and the Caᵥα₂δ-2, but not Caᵥα₂δ-3 and Caᵥα₂δ-4, subunits) is the binding site for gabapentin which has anti-allodynic/ hyperalgesic properties in patients and animal models. Because injury-induced Caᵥα₂δ-1 expression correlates with neuropathic pain development and maintenance, and various calcium channels are known to contribute to spinal synaptic neurotransmission and DRG neuron excitability, injury-induced Caᵥα₂δ-1 subunit upregulation may contribute to the initiation and maintenance of neuropathic pain by altering the properties and/or distribution of VGCC in the subpopulation of DRG neurons and their central terminals, therefore modulating excitability and/or synaptic neuroplasticity in the dorsal horn. Intrathecal antisense oligonucleotides against the Caᵥα₂δ-1 subunit can block nerve injury-induced Caᵥα₂δ-1 upregulation and prevent the onset of allodynia and reserve established allodynia.

As mentioned above, the α₂δ subunits of VGCC form the binding site for gabapentin and pregabalin, which are structural derivatives of the inhibitory neurotransmitter GABA although they do not bind to GABAA, GABAB, or benzodiazepine receptors, or alter GABA regulation in animal brain preparations. The binding of gabapentin and pregabalin to the Caᵥα₂δ subunit results in a reduction in the calcium-dependent release of multiple neurotransmitters, leading to efficacy and tolerability for neuropathic pain management. Gabapentinoids may also reduce excitability by inhibiting synaptogenesis (Perret and Luo, 2009, *supra;* Vink and Alewood, 2012, *supra*, Zamponi et al., 2015, *supra).*

As mentioned before, there are few available therapeutic classes for the treatment of pain, and opioids are among the most effective, especially when addressing severe pain states. They act through three different types of opioid receptors (mu, kappa and delta) which are transmembrane G-protein coupled receptors (GPCRs). Still, the main analgesic action is attributed to the activation of the µ-opioid receptor (MOR). However, the general administration of MOR agonists is limited due to their important side effects, such as constipation, respiratory depression, tolerance, emesis and physical dependence [Meldrum, M.L. (Ed.). Opioids and Pain Relief: A Historical Perspective. Progress in Pain Research and Management, Vol 25. IASP Press, Seattle, 2003]. Additionally, MOR agonists are not optimal for the treatment of chronic pain as indicated by the diminished effectiveness of morphine against chronic pain conditions. This is especially proven for the chronic pain conditions of neuropathic or inflammatory origin, in comparison to its high potency against acute pain. The finding that chronic pain can lead to MOR down-regulation may offer a molecular basis for the relative lack of efficacy of morphine in long-term treatment settings [Dickenson, A.H., Suzuki, R. Opioids in neuropathic pain: Clues from animal studies. Eur J Pain 9, 113-6 (2005)]. Moreover, prolonged treatment with morphine may result in tolerance to its analgesic effects, most likely due to treatment-induced MOR down-regulation, internalization and other regulatory mechanisms. As a consequence, long-term treatment can result in substantial increases in dosing in order to maintain a clinically satisfactory pain relief, but the narrow therapeutic window of MOR agonists finally results in unacceptable side effects and poor patient compliance.

Polypharmacology is a phenomenon in which a drug binds multiple rather than a single target with significant affinity. The effect of polypharmacology on therapy can be positive (effective therapy) and/or negative (side effects). Positive and/or negative effects can be caused by binding to the same or different subsets of targets; binding to some targets may have no effect. Multi-component drugs or multi-targeting drugs can overcome toxicity and other side effects associated with high doses of single drugs by countering biological compensation, allowing reduced dosage of each compound or accessing context-specific multitarget mechanisms. Because multitarget mechanisms require their targets to be available for coordinated action, one would expect synergies to occur in a narrower range of cellular phenotypes given differential expression of the drug targets than would the activities of single agents. In fact, it has been experimentally demonstrated that synergistic drug combinations are generally more specific to particular cellular contexts than are single agent activities, such selectivity is achieved through differential expression of the drugs' targets in cell types associated with therapeutic, but not toxic, effects (Lehar et al., Nat Biotechnol 2009; 27: 659-666.).

In the case of chronic pain, which is a multifactorial disease, multi-targeting drugs may produce concerted pharmacological intervention of multiple targets and signaling pathways that drive pain. Because they actually make use of biological complexity, multi-targeting (or multi-component drugs) approaches are among the most promising avenues toward treating multifactorial diseases such as pain (Gilron et al., Lancet Neurol. 2013 Nov;12(11):1084-95.). In fact, positive synergistic interaction for several compounds, including analgesics, has been described (Schroder et al., J Pharmacol Exp Ther. 2011; 337:312-20. Erratum in: J Pharmacol Exp Ther. 2012; 342:232.; Zhang et al., Cell Death Dis. 2014; 5:e1138.; Gilron et al., 2013, *supra).*

Given the significant differences in pharmacokinetics, metabolisms and bioavailability, reformulation of drug combinations (multi-component drugs) is challenging. Further, two drugs that are generally safe when dosed individually cannot be assumed to be safe in combination. In addition to the possibility of adverse drug-drug interactions, if the theory of network pharmacology indicates that an effect on phenotype may derive from hitting multiple targets, then that combined phenotypic perturbation may be efficacious or deleterious. The major challenge to both drug combination strategies is the regulatory requirement for each individual drug to be shown to be safe as an individual agent and in combination (Hopkins, Nat Chem Biol. 2008; 4:682-90.).

An alternative strategy for multitarget therapy is to design a single compound with selective polypharmacology (multi-targeting drug). It has been shown that many approved drugs act on multiple targets. Dosing with a single compound may have advantages over a drug combination in terms of equitable pharmacokinetics and biodistribution. Indeed, troughs in drug exposure due to incompatible pharmacokinetics between components of a combination therapy may create a low-dose window of opportunity where a reduced selection pressure can lead to drug resistance. In terms of drug registration, approval of a single compound acting on multiple targets faces significantly lower regulatory barriers than approval of a combination of new drugs (Hopkins, 2008, *supra).*

Thus, the present application, relates to the advantages of having dual activity, for µ-receptor and the α₂δ-1 subunit of voltage-gated calcium channels, in the same molecule to treat chronic pain.

In this way, the present invention relates to compounds having a complementary dual mechanism of action (µ-receptor agonist and blocker of the α₂δ subunit, in particular the α₂δ-1 subunit, of voltage-gated calcium channels) which implies a better profile of tolerability than the strong opioids (morphine, oxycodone, fentanyl, etc) and/or better efficacy and tolerability than gabapentinoids (pregabalin and gabapentin).

Pain is multimodal in nature, since in nearly all pain states several mediators, signaling pathways and molecular mechanisms are implicated. Consequently, monomodal therapies fail to provide complete pain relief. Currently, combining existing therapies is a common clinical practice and many efforts are directed to assess the best combination of available drugs in clinical studies (Mao, J., Gold, M.S., Backonja, M.; 2011; J. Pain; 12; 157-166).

Accordingly, there is still a need to find compounds that have an alternative or improved pharmacological activity in the treatment of pain, being both effective and showing the desired selectivity, and having good "drugability" properties, i.e. good pharmaceutical properties related to administration, distribution, metabolism and excretion.

The authors of the present invention, have found a series of compounds that show dual pharmacological activity towards both the α₂δ subunit, in particular the α₂δ-1 subunit, of the voltage-gated calcium channel, and the µ-opioid receptor (MOR) resulting in an innovative, effective and alternative solution for the treatment of pain.

In view of the existing results of the currently available therapies and clinical practices, the present invention offers a solution by combining in a single compound binding to two different targets relevant for the treatment of pain. This was mainly achieved by providing the compounds according to the invention that bind both to the µ-opioid receptor and to the α₂δ subunit, in particular the α₂δ-1 subunit, of the voltage-gated calcium channel.

### SUMMARY OF THE INVENTION

In this invention a family of structurally distinct substituted bicyclic derivatives, encompassed by formula (I), which have a dual pharmacological activity towards both the α₂δ subunit, in particular the α₂δ-1 subunit, of the voltage-gated calcium channel, and the µ-opioid receptor, was identified thus solving the above problem of identifying alternative or improved pain treatments by offering such dual compounds.

The main object of the invention is directed to a compound having a dual activity binding to the α₂δ subunit, in particular the α₂δ-1 subunit, of the voltage-gated calcium channel and the µ-opioid receptor for use in the treatment of pain.

The invention is directed in a main aspect to a compound of general Formula (I), 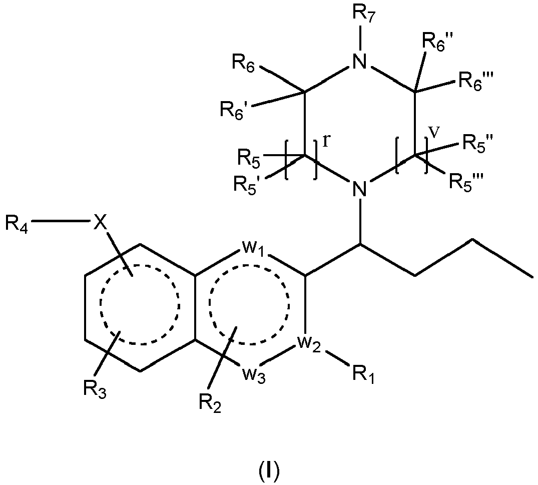 wherein R₁, R₂, R₃, R₄, R₅, R₅', R₅", R₅"', R₆, R₆', R₆", R₆'", R₇, X, w₁, w₂, w₃, r and v are as defined below in the detailed description.

A further object of the invention refers to the processes for preparation of compounds of general formula (I).

A still further object of the invention refers to the use of intermediate compounds for the preparation of a compound of general formula (I).

It is also an object of the invention a pharmaceutical composition comprising a compound of formula (I).

Finally, it is an object of the invention the use of compound as a medicament and more particularly for the treatment of pain and pain related conditions.

### DETAILED DESCRIPTION OF THE INVENTION

In this invention a family of structurally distinct substituted bicyclic derivatives, encompassed by formula (I), which have a dual pharmacological activity towards both the α₂δ subunit, in particular the α₂δ-1 subunit, of the voltage-gated calcium channel, and the µ-opioid receptor was identified, thus solving the above problem of identifying alternative or improved pain treatments by offering such dual compounds.

The main object of the invention is directed to a compound having a dual activity binding to the α₂δ subunit, in particular the α₂δ-1 subunit, of the voltage-gated calcium channel and the µ-opioid receptor, for use in the treatment of pain.

As this invention is aimed at providing a compound or a chemically related series of compounds which act as dual ligands of the α₂δ subunit, in particular the α₂δ-1 subunit, of the voltage-gated calcium channel and the µ-opioid receptor, it is a very preferred embodiment if the compound has a binding expressed as Kᵢ responding to the following scales:
Kᵢ(µ) is preferably < 1000 nM, more preferably < 500 nM, even more preferably < 100 nM.

Preferably, when Kᵢ (µ) > 500 nM, the following scale has been adopted for representing the binding to the µ -receptor:
+ Kᵢ(µ) > 500 nM or inhibition ranges between 1% and 50 %.

Kᵢ(α₂δ-1) is preferably < 10000 nM, more preferably < 5000 nM, even more preferably < 500 nM or even more preferably < 100 nM.

Preferably, when Kᵢ(α₂δ-1) > 5000 nM, the following scale has been adopted for representing the binding to the α₂δ-1 subunit of voltage-gated calcium channels:
+ Kᵢ(α₂δ-1) > 5000 nM or inhibition ranges between 1 % and 50 %

The applicant has surprisingly found that the problem of providing a new effective and alternative for treating pain and pain related disorders can be solved by using a multimodal balanced analgesic approach combining two different synergistic activities in a single drug (i.e., dual ligands which are bifunctional and bind to µ-opioid receptor and to α₂δ subunit, in particular the α₂δ-1 subunit, of the voltage-gated calcium channel), thereby enhancing through the α₂δ blockade without increasing the undesirable side effects. This supports the therapeutic value of a dual agent, whereby the α₂δ binding component acts as an intrinsic adjuvant of the MOR binding component.

A dual compound that possess binding to both the µ-opioid receptor and to the α₂δ subunit of the voltage-gated calcium channel shows a highly valuable therapeutic potential by achieving an outstanding analgesia (enhanced in respect to the potency of the opioid component alone) with a reduced side-effect profile (safety margin increased compared to that of the opioid component alone) versus existing opioid therapies.

Advantageously, the dual compounds according to the present invention would in addition show one or more the following functionalities: blockade of the α₂δ subunit, in particular the α₂δ-1 subunit, of the voltage-gated calcium channel and µ-opioid receptor agonism

It has to be noted, though, that functionalities "antagonism" and "agonism" are also subdivided in their effect into subfunctionalities like partial agonism or inverse agonism. Accordingly, the functionalities of the compound should be considered within a relatively broad bandwidth.

An antagonist blocks or dampens agonist-mediated responses. Known subfunctionalities are neutral antagonists or inverse agonists.

An agonist increases the activity of the receptor above its basal level. Known subfunctionalities are full agonists, or partial agonists.

In addition, the two mechanisms complement each other since MOR agonists are only marginally effective in the treatment of neuropathic pain, while the blockers of the α₂δ subunit, in particular the α₂δ-1 subunit, of voltage-gated calcium channels show outstanding effects in preclinical neuropathic pain models. Thus, the α₂δ component, in particular the α₂δ-1 component, adds unique analgesic actions in opioid-resistant pain. Finally, the dual approach has clear advantages over MOR agonists in the treatment of chronic pain as lower and better tolerated doses would be needed based on the potentiation of analgesia but not of the adverse events of MOR agonists.

A further advantage of using designed multiple ligands is a lower risk of drug-drug interactions compared to cocktails or multi-component drugs, thus involving simpler pharmacokinetics and less variability among patients. Additionally, this approach may improve patient compliance and broaden the therapeutic application in relation to monomechanistic drugs, by addressing more complex aetiologies. It is also seen as a way of improving the R&D output obtained using the "one drug-one target" approach, which has been questioned over the last years [Bornot A, Bauer U, Brown A, Firth M, Hellawell C, Engkvist O. Systematic Exploration of Dual-Acting Modulators from a Combined Medicinal Chemistry and Biology Perspective. J. Med. Chem, 56, 1197-1210 (2013)].

In its broader aspect, the present invention is directed to compounds of general Formula (I): 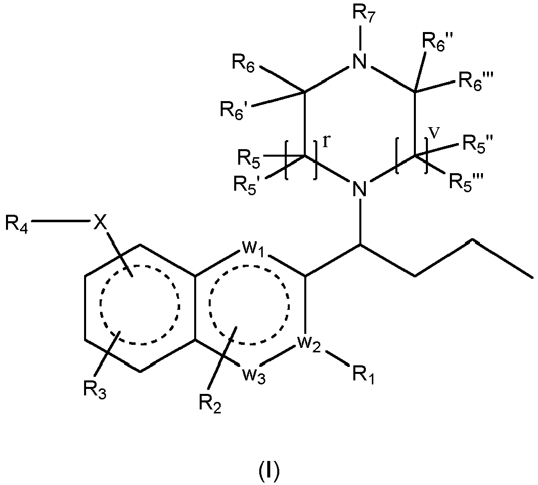 wherein
X is selected from the group consisting of a bond and -[C(RₐR_{b})]ₚ-;
   Rₐ is selected from the group consisting of hydrogen, substituted or unsubstituted C₁₋₆ alkyl, substituted or unsubstituted C₂₋₆ alkenyl and substituted or unsubstituted C₂₋₆ alkynyl;
   R_{b} is selected from the group consisting of hydrogen, substituted or unsubstituted C₁₋₆ alkyl, substituted or unsubstituted C₂₋₆ alkenyl and substituted or unsubstituted C₂₋₆ alkynyl;
      alternatively, Rₐ and R_{b}, taken together with the carbon atom to which they are attached, form a substituted or unsubstituted cycloalkyl;
   p is 0, 1, 2, 3, 4 or 5;
w₁ is nitrogen or carbon;
w₂ is nitrogen or - carbon;
w₃ is nitrogen or carbon;
r is 0, 1 or 2;
v is 0, 1 or 2;
R₁ is selected from the group consisting of hydrogen, substituted or unsubstituted C₁₋₆ alkyl, substituted or unsubstituted C₂₋₆ alkenyl and substituted or unsubstituted C₂₋₆ alkynyl;
R₂ is selected from the group consisting of -R₂₁, -OR₂₁, -NO₂, halogen, -NR₂₁R₂₁', - NR₂₁C(O)R₂₁', -NR₂₁S(O)₂R₂₁', -S(O)₂NR₂₁R₂₁', - NR₂₁C(O)NR₂₁'R₂₁", -SR₂₁, -S(O)R₂₁, -S(O)₂R₂₁, -CN, haloalkyl, haloalkoxy, -C(O)OR₂₁, -C(O)NR₂₁R₂₁', -OCH₂CH₂OR₂₁, - NR₂₁S(O)₂NR₂₁'R₂₁" and -C(CH₃)₂OR₂₁;
   wherein R₂₁, R₂₁' and R₂₁" are independently selected from the group consisting of hydrogen, substituted or unsubstituted C₁₋₆ alkyl, substituted or unsubstituted C₂₋₆ alkenyl, substituted or unsubstituted C₂₋₆ alkynyl and substituted or unsubstituted alkylcycloalkyl;
R₃ is selected from the group consisting of -R₃₁, -OR₃₁, -NO₂, halogen, -NR₃₁R₃₁', - NR₃₁C(O)R₃₁', -NR₃₁S(O)₂R₃₁', -S(O)₂NR₃₁R₃₁', - NR₃₁C(O)NR₃₁'R₃₁", -SR₃₁, -S(O)R₃₁, -S(O)₂R₃₁, -CN, haloalkyl, haloalkoxy, -C(O)OR₃₁, -C(O)NR₃₁R₃₁', -OCH₂CH₂OR₃₁, - NR₃₁S(O)₂NR₃₁'R₃₁" and -C(CH₃)₂OR₃₁;
   wherein R₃₁, R₃₁' and R₃₁" are independently selected from the group consisting of hydrogen, substituted or unsubstituted C₁₋₆ alkyl, substituted or unsubstituted C₂₋₆ alkenyl and substituted or unsubstituted C₂₋₆ alkynyl;
R₄ is 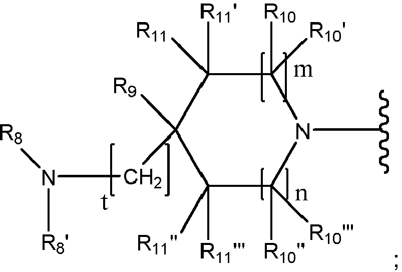
m is 0, 1 or 2;
n is 0, 1 or 2;
t is 0, 1 or 2;
R₅, R₅', R₅" and R₅'" are independently selected from the group consisting of hydrogen, halogen, substituted or unsubstituted C₁₋₆ alkyl, substituted or unsubstituted C₂₋₆ alkenyl and substituted or unsubstituted C₂₋₆ alkynyl;
   alternatively, R₅ and R₅' and/or R₅" and R₅'" taken together with the carbon atom to which they are attached form a carbonyl group;
R₆, R₆', R₆" and R₆'" are independently selected from the group consisting of hydrogen, substituted or unsubstituted C₁₋₆ alkyl, substituted or unsubstituted C₂₋₆ alkenyl and substituted or unsubstituted C₂₋₆ alkynyl;
R₇ is selected from the group consisting of hydrogen, substituted or unsubstituted C₁₋₆ alkyl, substituted or unsubstituted C₂₋₆ alkenyl and substituted or unsubstituted C₂₋₆ alkynyl;
R₈ is selected from the group consisting of substituted or unsubstituted C₁₋₆ alkyl, substituted or unsubstituted C₂₋₆ alkenyl, substituted or unsubstituted C₂₋₆ alkynyl, substituted or unsubstituted cycloalkyl and substituted or unsubstituted alkylcycloalkyl;
R₈' is selected from the group consisting of hydrogen, substituted or unsubstituted C₁₋₆ alkyl, substituted or unsubstituted C₂₋₆ alkenyl, substituted or unsubstituted C₂₋₆ alkynyl, substituted or unsubstituted cycloalkyl and substituted or unsubstituted alkylcycloalkyl;
alternatively, R₈ and R₈' taken together with the nitrogen atom to which they are attached form a substituted or unsubstituted heterocyclyl;
Rg is selected from the group consisting of substituted or unsubstituted C₁₋₆ alkyl, substituted or unsubstituted C₂₋₆ alkenyl, substituted or unsubstituted C₂₋₆ alkynyl, substituted or unsubstituted cycloalkyl, substituted or unsubstituted aryl, substituted or unsubstituted heterocyclyl, substituted or unsubstituted alkylcycloalkyl, substituted or unsubstituted alkylaryl and substituted or unsubstituted alkylheterocyclyl;
R₁₀, R₁₀', R₁₀" and R₁₀'" are independently selected from the group consisting of hydrogen, halogen, substituted or unsubstituted C₁₋₆ alkyl, substituted or unsubstituted C₂₋₆ alkenyl and substituted or unsubstituted C₂₋₆ alkynyl;
alternatively, R₁₀ and R₁₀' and/or R₁₀" and R₁₀'" taken together with the carbon atom to which they are attached form a substituted or unsubstituted cycloalkyl;
alternatively, R₁₀ and R₁₀' and/or R₁₀" and R₁₀'" taken together with the carbon atom to which they are attached form a carbonyl group;
R₁₁, R₁₁', R₁₁" and R₁₁'" are independently selected from the group consisting of hydrogen, halogen, substituted or unsubstituted C₁₋₆ alkyl, substituted or unsubstituted C₂₋₆ alkenyl and substituted or unsubstituted C₂₋₆ alkynyl;
alternatively, R₁₁ and R₁₁' and/or R₁₁" and R₁₁'" taken together with the carbon atom to which they are attached form a substituted or unsubstituted cycloalkyl;
These compounds according to the invention are optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

Note that "or a corresponding salt thereof" does also mean "or a corresponding pharmaceutically acceptable salt thereof". This does apply to all below described embodiments and uses of "salt" being thus equivalent to "pharmaceutically acceptable salt".

In another embodiment, these compounds according to the invention are optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof.

In a particular embodiment, the following proviso applies: when w₃ is carbon substituted with -OR₂₁ with R₂₁ being hydrogen, then w₂ is carbon.

In a particular embodiment, the following proviso applies: when w₃ is carbon substituted with -OR₂₁ with R₂₁ being hydrogen, then w₂ is not nitrogen.

In a particular embodiment, the following proviso applies:
when w₃ is carbon substituted with =O, then w₂ is carbon.

In a particular embodiment, the following proviso applies:
when w₃ is 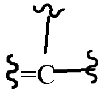 substituted with -OR₂₁ with R₂₁ being hydrogen, then w₂ is carbon.

In a particular embodiment, the following proviso applies:
when w₃ is =CH- substituted with -OR₂₁ with R₂₁ being hydrogen, then w₂ is carbon.

In a particular embodiment, the following proviso applies:
when w₃ is =C(OH)-, then w₂ is carbon.

In a particular embodiment, the following proviso applies:
when w₃ is C(O), then w₂ is carbon.

In a further embodiment the compound according to the invention is a compound of general Formula (I) 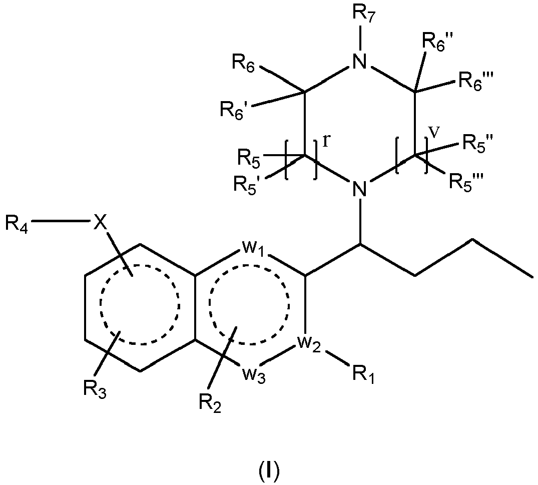 wherein
X is selected from the group consisting of a bond and -[C(RₐR_{b})]ₚ-;
   Rₐ is selected from the group consisting of hydrogen, substituted or unsubstituted C₁₋₆ alkyl, substituted or unsubstituted C₂₋₆ alkenyl and substituted or unsubstituted C₂₋₆ alkynyl;
   R_{b} is selected from the group consisting of hydrogen, substituted or unsubstituted C₁₋₆ alkyl, substituted or unsubstituted C₂₋₆ alkenyl and substituted or unsubstituted C₂₋₆ alkynyl;
      alternatively, Rₐ and R_{b}, taken together with the carbon atom to which they are attached, form a substituted or unsubstituted cycloalkyl;
   p is 0, 1, 2, 3, 4 or 5;
w₁ is nitrogen or carbon;
w₂ is nitrogen or carbon;
w₃ is nitrogen or carbon;
r is 0, 1 or 2;
v is 0, 1 or 2;
R₁ is selected from the group consisting of hydrogen, substituted or unsubstituted C₁₋₆ alkyl, substituted or unsubstituted C₂₋₆ alkenyl and substituted or unsubstituted C₂₋₆ alkynyl;
wherein
   the alkyl, alkenyl or alkynyl defined in R₁, if substituted, is substituted with one or more substituent/s selected from the group consisting of -OR₁₅, halogen, -CN, haloalkyl, haloalkoxy and -NR₁₅R₁₅';
   wherein R₁₅ and R₁₅' are independently selected from the group consisting of hydrogen, substituted or unsubstituted C₁₋₆ alkyl, substituted or unsubstituted C₂₋₆ alkenyl, and substituted or unsubstituted C₂₋₆ alkynyl;
R₂ is selected from the group consisting of -R₂₁, -OR₂₁, -NO₂, halogen, -NR₂₁R₂₁', - NR₂₁C(O)R₂₁', -NR₂₁S(O)₂R₂₁', -S(O)₂NR₂₁R₂₁', - NR₂₁C(O)NR₂₁'R₂₁", -SR₂₁, -S(O)R₂₁, -S(O)₂R₂₁, -CN, haloalkyl, haloalkoxy, -C(O)OR₂₁, -C(O)NR₂₁R₂₁', -OCH₂CH₂OR₂₁, - NR₂₁S(O)₂NR₂₁'R₂₁" and -C(CH₃)₂OR₂₁;
   wherein R₂₁, R₂₁' and R₂₁" are independently selected from the group consisting of hydrogen, substituted or unsubstituted C₁₋₆ alkyl, substituted or unsubstituted C₂₋₆ alkenyl, substituted or unsubstituted C₂₋₆ alkynyl and substituted or unsubstituted alkylcycloalkyl;
R₃ is selected from the group consisting of -R₃₁, -OR₃₁, -NO₂, halogen, -NR₃₁R₃₁', - NR₃₁C(O)R₃₁', -NR₃₁S(O)₂R₃₁', -S(O)₂NR₃₁R₃₁', - NR₃₁C(O)NR₃₁'R₃₁", -SR₃₁, -S(O)R₃₁, -S(O)₂R₃₁, -CN, haloalkyl, haloalkoxy, -C(O)OR₃₁, -C(O)NR₃₁R₃₁', -OCH₂CH₂OR₃₁, - NR₃₁S(O)₂NR₃₁'R₃₁" and -C(CH₃)₂OR₃₁;
   wherein R₃₁, R₃₁' and R₃₁" are independently selected from the group consisting of hydrogen, substituted or unsubstituted C₁₋₆ alkyl, substituted or unsubstituted C₂₋₆ alkenyl and substituted or unsubstituted C₂₋₆ alkynyl;
R₄ is 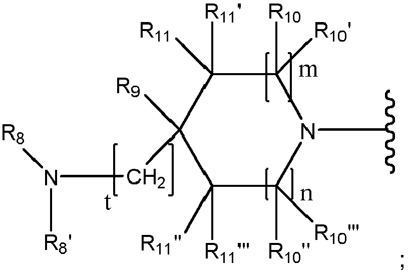
m is 0, 1 or 2;
n is 0, 1 or 2;
t is 0, 1 or 2;
R₅, R₅', R₅" and R₅'" are independently selected from the group consisting of hydrogen, halogen, substituted or unsubstituted C₁₋₆ alkyl, substituted or unsubstituted C₂₋₆ alkenyl and substituted or unsubstituted C₂₋₆ alkynyl;
   alternatively, R₅ and R₅' and/or R₅" and R₅'" taken together with the carbon atom to which they are attached form a carbonyl group;
R₆, R₆', R₆" and R₆'" are independently selected from the group consisting of hydrogen, substituted or unsubstituted C₁₋₆ alkyl, substituted or unsubstituted C₂₋₆ alkenyl and substituted or unsubstituted C₂₋₆ alkynyl;
R₇ is selected from the group consisting of hydrogen, substituted or unsubstituted C₁₋₆ alkyl, substituted or unsubstituted C₂₋₆ alkenyl and substituted or unsubstituted C₂₋₆ alkynyl;
   wherein
   the alkyl, alkenyl or alkynyl defined in R₇, if substituted, is substituted with one or more substituent/s selected from the group consisting of -OR₇₁, halogen, -CN, haloalkyl, haloalkoxy and -NR₇₁R₇₁';
   wherein R₇₁ and R₇₁' are independently selected from the group consisting of hydrogen, substituted or unsubstituted C₁₋₆ alkyl, substituted or unsubstituted C₂₋₆ alkenyl, and substituted or unsubstituted C₂₋₆ alkynyl;
R₈ is selected from the group consisting of substituted or unsubstituted C₁₋₆ alkyl, substituted or unsubstituted C₂₋₆ alkenyl, substituted or unsubstituted C₂₋₆ alkynyl, substituted or unsubstituted cycloalkyl and substituted or unsubstituted alkylcycloalkyl;
   wherein
   the alkyl, alkenyl or alkynyl defined in R₈, if substituted, is substituted with one or more substituent/s selected from the group consisting of - OR₈₁, halogen, -CN, haloalkyl, haloalkoxy and -NR₈₁R₈₁';
   wherein
   the cycloalkyl defined in R₈, also in alkylcycloalkyl, if substituted, is substituted with one or more substituent/s selected from the group consisting of =O, halogen, -R₈₁, -OR₈₁, -NO₂, -NR₈₁R₈₁', -NR₈₁C(O)R₈₁', -NR₈₁S(O)₂R₈₁', -S(O)₂NR₈₁R₈₁', - NR₈₁C(O)NR₈₁'R₈₁", -SR₈₁, -S(O)R₈₁, -S(O)₂R₈₁, -CN, haloalkyl, haloalkoxy, -C(O)OR₈₁, -C(O)NR₈₁R₈₁', - OCH₂CH₂OR₈₁, -NR₈₁S(O)₂NR₈₁'R₈₁" and -C(CH₃)₂OR₈₁;
   wherein R₈₁, R₈₁' and R₈₁" are independently selected from the group consisting of hydrogen, substituted or unsubstituted C₁₋₆ alkyl, substituted or unsubstituted C₂₋₆ alkenyl and substituted or unsubstituted C₂₋₆ alkynyl;
R₈' is selected from the group consisting of hydrogen, substituted or unsubstituted C₁₋₆ alkyl, substituted or unsubstituted C₂₋₆ alkenyl, substituted or unsubstituted C₂₋₆ alkynyl, substituted or unsubstituted cycloalkyl and substituted or unsubstituted alkylcycloalkyl;
   wherein
   the alkyl, alkenyl or alkynyl defined in R₈', if substituted, is substituted with one or more substituent/s selected from the group consisting of -OR₈₂, halogen, -CN, haloalkyl, haloalkoxy and -NR₈₂R₈₂';
   the cyclolakyl defined in R₈', also in alkylcycloalkyl, if substituted, is substituted with one or more substituent/s selected from the group consisting of =O, halogen, -R₈₂, -OR₈₂, -NO₂, -NR₈₂R₈₂', -NR₈₂C(O)R₈₂', - NR₈₂S(O)₂R₈₂', -S(O)₂NR₈₂R₈₂', - NR₈₂C(O)NR₈₂'R₈₂", -SR₈₂, -S(O)R₈₂, - S(O)₂R₈₂, -CN, haloalkyl, haloalkoxy, -C(O)OR₈₂, -C(O)NR₈₂R₈₂', - OCH₂CH₂OR₈₂, -NR₈₂S(O)₂NR₈₂'R₈₂" and -C(CH₃)₂OR₈₂;
   wherein R₈₂, R₈₂' and R₈₂" are independently selected from the group consisting of hydrogen, substituted or unsubstituted C₁₋₆ alkyl, substituted or unsubstituted C₂₋₆ alkenyl, and substituted or unsubstituted C₂₋₆ alkynyl;
alternatively, R₈ and R₈' taken together with the nitrogen atom to which they are attached form a substituted or unsubstituted heterocyclyl;
   wherein the heterocyclyl, as defined in R₈-R₈', if substituted, is substituted with one or more substituent/s selected from the group consisting of =O, halogen, -R₈₃, -OR₈₃, -NO₂, -NR₈₃R₈₃', -NR₈₃C(O)R₈₃', -NR₈₃S(O)₂R₈₃', - S(O)₂NR₈₃R₈₃', - NR₈₃C(O)NR₈₃'R₈₃", -SR₈₃, -S(O)R₈₃, -S(O)₂R₈₃, -CN, haloalkyl, haloalkoxy, -C(O)OR₈₃, -C(O)NR₈₃R₈₃', -OCH₂CH₂OR₈₃, - NR₈₃S(O)₂NR₈₃'R₈₃" and -C(CH₃)₂OR₈₃;
   wherein R₈₃, R₈₃' and R₈₃" are independently selected from the group consisting of hydrogen, substituted or unsubstituted C₁₋₆ alkyl, substituted or unsubstituted C₂₋₆ alkenyl, and substituted or unsubstituted C₂₋₆ alkynyl;
R₉ is selected from the group consisting of substituted or unsubstituted C₁₋₆ alkyl, substituted or unsubstituted C₂₋₆ alkenyl, substituted or unsubstituted C₂₋₆ alkynyl, substituted or unsubstituted cycloalkyl, substituted or unsubstituted aryl, substituted or unsubstituted heterocyclyl, substituted or unsubstituted alkylcycloalkyl, substituted or unsubstituted alkylaryl and substituted or unsubstituted alkylheterocyclyl;
   wherein
   the alkyl, alkenyl or alkynyl defined in R₉, if substituted, is substituted with one or more substituent/s selected from the group consisting of -OR₉₁, halogen, -CN, haloalkyl, haloalkoxy and -NR₉₁R₉₁';
   the cycloalkyl, aryl or heterocyclyl, also in alkylcycloalkyl, alkylaryl or alkylheterocyclyl, as defined in R₉, if substituted, is substituted with one or more substituent/s selected from the group consisting of halogen, -R₉₁, - OR₉₁, -NO₂, -NR₉₁R₉₁', -NR₉₁C(O)R₉₁', -NR₉₁S(O)₂R₉₁', -S(O)₂NR₉₁R₉₁', - NR₉₁C(O)NR₉₁'R₉₁", -SR₉₁, -S(O)R₉₁, -S(O)₂R₉₁, -CN, haloalkyl, haloalkoxy, -C(O)OR₉₁, -OC(O)R₉₁, -C(O)NR₉₁R₉₁', -OCH₂CH₂OR₉₁, - NR₉₁S(O)₂NR₉₁'R₉₁" and -C(CH₃)₂OR₉₁;
   wherein R₉₁, R₉₁' and R₉₁" are independently selected from the group consisting of the group consisting of hydrogen, substituted or unsubstituted C₁₋₆ alkyl, substituted or unsubstituted C₂₋₆ alkenyl, and substituted or unsubstituted C₂₋₆ alkynyl;
R₁₀, R₁₀', R₁₀" and R₁₀'" are independently selected from the group consisting of hydrogen, halogen, substituted or unsubstituted C₁₋₆ alkyl, substituted or unsubstituted C₂₋₆ alkenyl and substituted or unsubstituted C₂₋₆ alkynyl;
alternatively, R₁₀ and R₁₀' and/or R₁₀" and R₁₀'" taken together with the carbon atom to which they are attached form a substituted or unsubstituted cycloalkyl;
alternatively, R₁₀ and R₁₀' and/or R₁₀" and R₁₀'" taken together with the carbon atom to which they are attached form a carbonyl group;
R₁₁, R₁₁', R₁₁" and R₁₁'" are independently selected from the group consisting of hydrogen, halogen, substituted or unsubstituted C₁₋₆ alkyl, substituted or unsubstituted C₂₋₆ alkenyl and substituted or unsubstituted C₂₋₆ alkynyl;
alternatively, R₁₁ and R₁₁' and/or R₁₁" and R₁₁'" taken together with the carbon atom to which they are attached form a substituted or unsubstituted cycloalkyl;
the alkyl, alkenyl or alkynyl, if substituted and the substitution has not been defined otherwise, it is substituted with one or more substituent/s selected from the group consisting of -OR₁₃, halogen, -CN, haloalkyl, haloalkoxy and -NR₁₃R₁₃';
   wherein R₁₃ and R₁₃' are independently selected from the group consisting of hydrogen, unsubstituted C₁₋₆ alkyl, unsubstituted C₂₋₆ alkenyl and unsubstituted C₂₋₆ alkynyl;
the aryl, heterocyclyl or cycloalkyl, also in alkylaryl, alkylheterocyclyl or alkylcycloalkyl, if substituted and the substitution has not been defined otherwise, it is substituted with one or more substituent/s selected from the group consisting of halogen, -R₁₄, -OR₁₄, - NO₂, -NR₁₄R₁₄', -NR₁₄C(O)R₁₄', -NR₁₄S(O)₂R₁₄', -S(O)₂NR₁₄R₁₄', - NR₁₄C(O)NR₁₄'R₁₄", -SR₁₄, -S(O)R₁₄, -S(O)₂R₁₄, -CN, haloalkyl, haloalkoxy, -C(O)OR₁₄, -C(O)NR₁₄R₁₄' - OCH₂CH₂OR₁₄, -NR₁₄S(O)₂NR₁₄'R₁₄" and -C(CH₃)₂OR₁₄;
   wherein R₁₄, R₁₄' and R₁₄" are independently selected from the group consisting of hydrogen, unsubstituted C₁₋₆ alkyl, unsubstituted C₂₋₆ alkenyl, unsubstituted C₂₋₆ alkynyl, unsubstituted aryl, unsubstituted cycloalkyl and unsubstituted heterocyclyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound of general Formula (I') 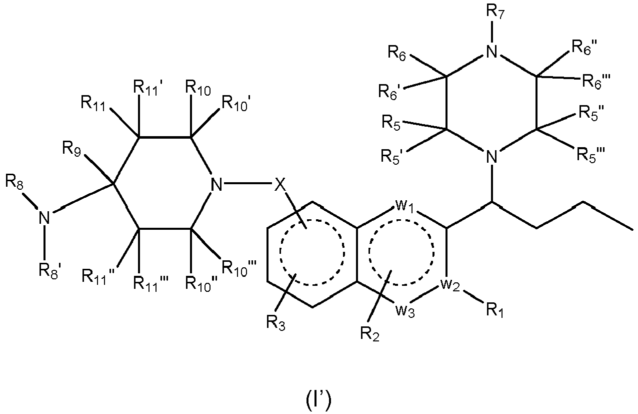 wherein R₁, R₂, R₃, R₅, R₅', R₅", R₅"', R₆, R₆', R₆", R₆'", R₇, R₈, R₈', R₉, R₁₀, R₁₀', R₁₀", R₁₀'", R₁₁, R₁₁', R₁₁", R₁₁"', X, w₁, w₂ and w₃ are as defined in the detailed description;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound of general Formula (I²') 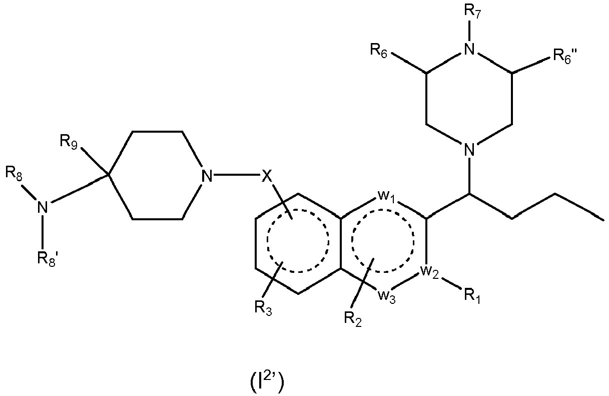 wherein R₁, R₂, R₃, R₆, R₆', R₇, R₈, R₈', R₉, X, w₁, w₂ and w₃ are as defined in the detailed description;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound of general Formula (I³') 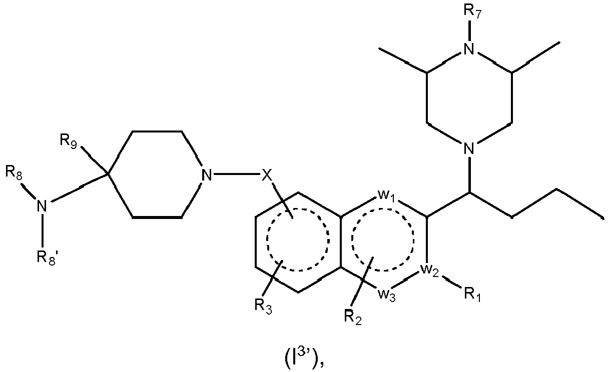 wherein R₁, R₂, R₃, R₇, R₈, R₈', R₉, X, w₁, w₂ and w₃ are as defined in the detailed description;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound of general Formula (I⁴') 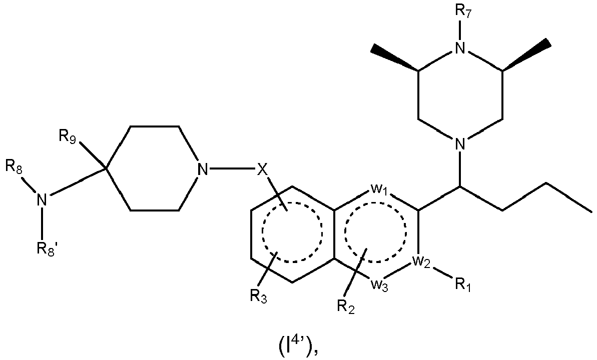 wherein R₁, R₂, R₃, R₇, R₈, R₈', R₉, X, w₁, w₂ and w₃ are as defined in the detailed description;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound of general Formula (I⁵') 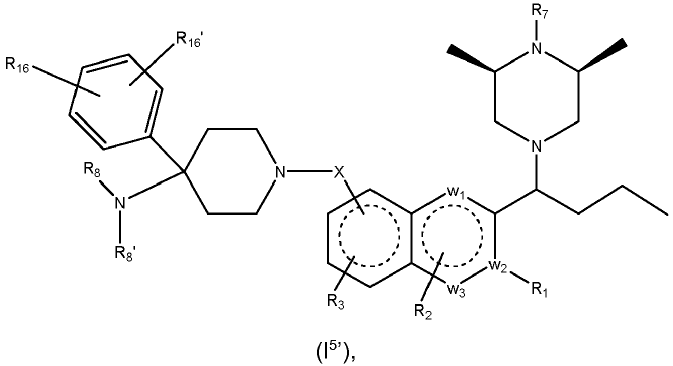 wherein R₁, R₂, R₃, R₇, R₈, R₈', X, w₁, w₂ and w₃ are as defined in the detailed description, and
R₁₆ and R₁₆' are independently selected from halogen, **-R₉₁**, **-OR₉₁**, -NO₂, -NR₉₁R₉₁', -NR₉₁C(O)R₉₁', -NR₉₁S(O)₂R₉₁', -S(O)₂NR₉₁R₉₁', - NR₉₁C(O)NR₉₁'R₉₁", -SR₉₁, -S(O)R₉₁, -S(O)₂R₉₁, -CN, haloalkyl, haloalkoxy, -C(O)OR₉₁, -OC(O)R₉₁, -C(O)NR₉₁R₉₁', -OCH₂CH₂OR₉₁, - NR₉₁S(O)₂NR₉₁'R₉₁" and -C(CH₃)₂OR₉₁,
wherein R₉₁, R₉₁' and R₉₁" are independently selected from the group consisting of the group consisting of **hydrogen,** substituted or unsubstituted C₁₋₆ alkyl, substituted or unsubstituted C₂₋₆ alkenyl, and substituted or unsubstituted C₂₋₆ alkynyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound of general Formula (I⁶') 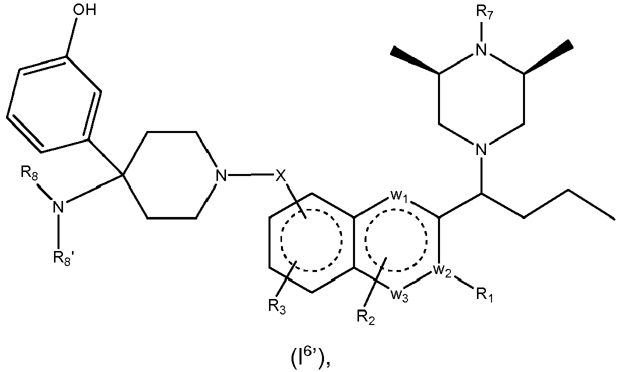 wherein R₁, R₂, R₃, R₇, R₈, R₈', X, w₁, w₂ and w₃ are as defined in the detailed description;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound of general Formula (I⁷') 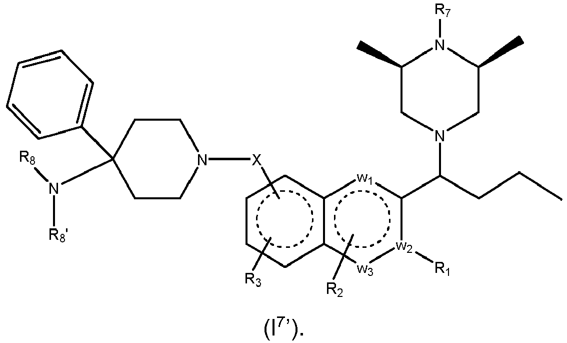 wherein R₁, R₂, R₃, R₇, R₈, R₈', X, w₁, w₂ and w₃ are as defined in the detailed description;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

For clarity purposes, all groups and definitions described in the present description and referring to compounds of general Formula(I), also apply to compounds of general Markush Formulae (I'), (I²'), (I³'), (I⁴'), (I⁵'), (I⁶'), (I⁷'), (I^{a}) or (IZ), (where applicable), and to all intermediate of synthesis, when those groups are present in the mentioned general Markush formulae, since compounds of general Markush Formulae (I'), (I²'), (I³'), (I⁴'), (I⁵'), (I⁶'), (I⁷'), (I^{a}) or (IZ) are included within the scope of the larger definition of general Markush Formula (I).

For clarity purposes, the general Markush Formula (I) 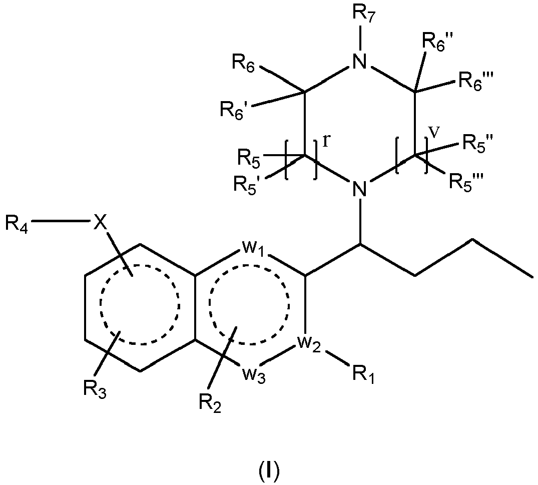 is equivalent to 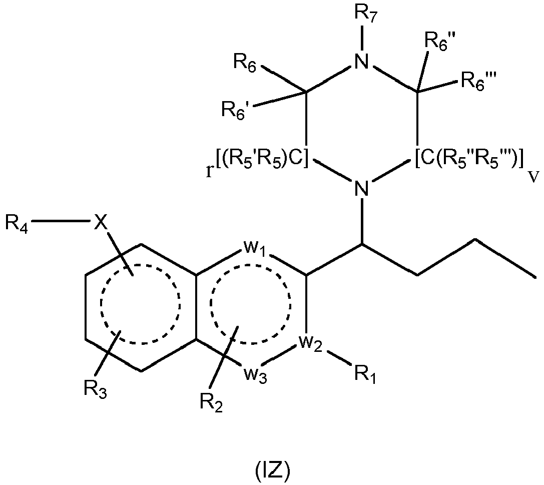 wherein only -C(R₅R₅')- and -C(R₅"R₅"')- are included into the brackets, and r and v mean the number of times that -C(R₅R₅')- and -C(R₅"R₅"')- are repeated, respectively. The same would apply, when applicable, to general Markush Formulae Formulae (I'), (I²'), (I³'), (I⁴'), (I⁵'), (I⁶'), (I⁷'), (I^{a}) and to all intermediates of synthesis.

In addition, and for clarity purposes, it should further be understood that naturally if r is 0, the adjacent groups N and -C(R₆R₆')- are still present. This is also applicable to all intermediates of synthesis.

Further, and for clarity purposes, it should further be understood that naturally if v is 0, the adjacent groups N and -C(R₆"R₆"')- are still present. This is also applicable to all intermediates of synthesis.

For clarity purposes, R₄ present in the Markush Formula (I) 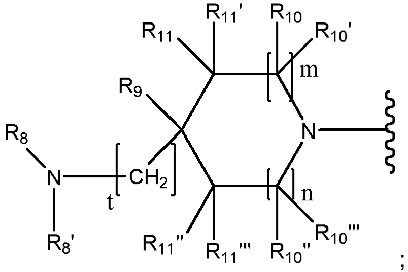 is equivalent to 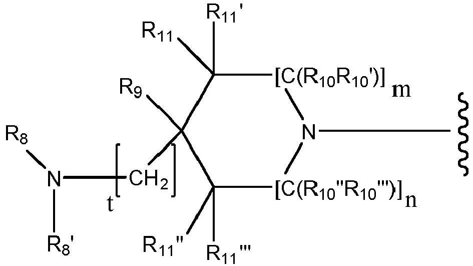 wherein only -C(R₁₀R₁₀')- and -C(R₁₀"R₁₀"')- are included into the brackets, and m and n mean the number of times that -C(R₁₀R₁₀')- and -C(R₁₀"R₁₀"')- are repeated, respectively. The same would apply, when applicable, to R₄ in all intermediates of synthesis.

In addition, and for clarity purposes, it should further be understood that naturally if m is 0, the adjacent groups N and -C(R₁₁R₁₁')- are still present. This is also applicable to all intermediates of synthesis.

Further, and for clarity purposes, it should further be understood that naturally if n is 0, the adjacent groups N and -C(R₁₁"R₁₁"')- are still present. This is also applicable to all intermediates of synthesis.

For clarity purposes, reference is also made to the following statements below in the definitions of substitutions on alkyl etc. or aryl etc. that "wherein when different radicals R₁ to R₉₁" are present simultaneously in Formula (I) they may be identical or different". This statement is reflected in the below general Formula (I^{a}) being derived from and falling into general Formulae (I) or (IZ), 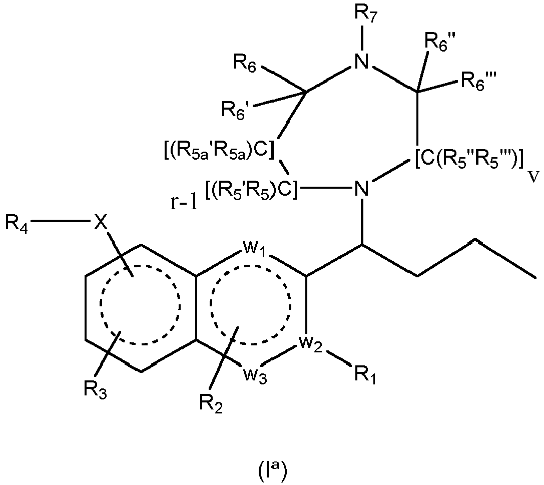 wherein R₁, R₂, R₃, R₄, R₅, R₅', R₅", R₅"', R₆, R₆', R₆", R₆'", R₇, X, w₁, w₂, w₃, v and r are as defined in the description. In addition, R₅ₐ and R₅ₐ' are added. As said above, this statement is thus reflected in that R₅ and R₅' are or could be different from R₅ₐ and R₅ₐ'or not.

The same would be applicable *mutatis mutandis* for general Formulas like general Formula (I) as well as the other general Formulas (I'), (I²'), (I³'), (I⁴'), (I⁵'), (I⁶'), (I⁷') or (IZ), above and to all intermediates of synthesis.

For clarity purposes, the expression e.g. "the cycle in R₈-R₈'", means the cycle resulting when R₈ and R₈' form a cycle together with the atom(s) to which they are attached. This cycle can then be substituted or not. This definition is also generally applicable and can be also applied as a definition of any other cycle (preferably cycloalkyls, heterocyclyls or aryls) formed from two different functional groups like e.g. "the cycle in Rᵢ-R_{i'}" means the cycle resulting when Rᵢ and Rᵢ' form a cycle together with the atom(s) to which they are attached. This cycle can then be substituted or not.

In the context of this invention, alkyl is understood as meaning saturated, linear or branched hydrocarbons, which may be unsubstituted or substituted once or several times. It encompasses e.g. -CH₃ and -CH₂-CH₃. In these radicals, C₁₋₂-alkyl represents C1- or C2-alkyl, C₁₋₃-alkyl represents C1-, C2- or C3-alkyl, C₁₋₄-alkyl represents C1-, C2-, C3- or C4-alkyl, C₁₋₅-alkyl represents C1-, C2-, C3-, C4-, or C5-alkyl, C₁₋₆-alkyl represents C1-, C2-, C3-, C4-, C5- or C6-alkyl, C₁₋₇-alkyl represents C1-, C2-, C3-, C4-, C5-, C6- or C7-alkyl, C₁₋₃-alkyl represents C1-, C2-, C3-, C4-, C5-, C6-, C7- or C8-alkyl, C₁₋₁₀-alkyl represents C1-, C2-, C3-, C4-, C5-, C6-, C7-, C8-, C9- or C10-alkyl and C₁₋₁₈-alkyl represents C1-, C2-, C3-, C4-, C5-, C6-, C7-, C8-, C9-, C10-, C11-, C12-, C13-, C14-, C15-, C16-, C17- or C18-alkyl. The alkyl radicals are preferably methyl, ethyl, propyl, methylethyl, butyl, 1-methylpropyl, 2-methylpropyl, 1,1-dimethylethyl, pentyl, 1,1-dimethylpropyl, 1,2-dimethylpropyl, 2,2-dimethylpropyl, hexyl, 1-methylpentyl, if substituted also CHF₂, CF₃ or CH₂OH etc. Preferably alkyl is understood in the context of this invention as C₁₋₈alkyl like methyl, ethyl, propyl, butyl, pentyl, hexyl, heptyl, or octyl; preferably is C₁₋₆alkyl like methyl, ethyl, propyl, butyl, pentyl, or hexyl; more preferably is C₁₋₄alkyl like methyl, ethyl, propyl or butyl.

Alkenyl is understood as meaning unsaturated, linear or branched hydrocarbons, which may be unsubstituted or substituted once or several times. It encompasses groups like e.g. -CH=CH-CH₃. The alkenyl radicals are preferably vinyl (ethenyl), allyl (2-propenyl). Preferably in the context of this invention alkenyl is C₂₋₁₀-alkenyl or C₂₋₈-alkenyl like ethylene, propylene, butylene, pentylene, hexylene, heptylene or octylene; or is C₂₋₆-alkenyl like ethylene, propylene, butylene, pentylene, or hexylene; or is C₂₋₄-alkenyl, like ethylene, propylene, or butylenes.

Alkynyl is understood as meaning unsaturated, linear or branched hydrocarbons, which may be unsubstituted or substituted once or several times. It encompasses groups like e.g. -C₅≡C-CH₃ (1-propinyl). Preferably alkynyl in the context of this invention is C₂₋₁₀-alkynyl or C₂₋₈-alkynyl like ethyne, propyne, butyene, pentyne, hexyne, heptyne, or octyne; or is C₂₋₆-alkynyl like ethyne, propyne, butyene, pentyne, or hexyne; or is C₂₋₄-alkynyl like ethyne, propyne, butyene, pentyne, or hexyne.

In connection with alkyl (also in alkylaryl, alkylheterocyclyl or alkylcycloalkyl), alkenyl, alkynyl and O-alkyl - unless defined otherwise - the term substituted in the context of this invention is understood as meaning replacement of at least one hydrogen radical on a carbon atom by halogen (F, Cl, Br, I), -NRₖR_{k'}, -SRₖ, -S(O)Rₖ, -S(O)₂Rₖ, -ORₖ, - C(O)Rₖ, -C(O)ORₖ, -CN, -C(0)NRₖR_{k'}, haloalkyl, haloalkoxy, being Rₖ represented by R₁₃, R₁₅, R₇₁, R₈₁, R₈₂ or R₉₁, (being R_{k'} represented by R₁₃', R₁₅', R₇₁', R₈₁', R₈₂' or R₉₁'; wherein R₁ to R₉₁" and Rₐ and R_{b} are as defined in the description, and wherein when different radicals R₁ to R₉₁" and Rₐ and R_{b} are present simultaneously in Formula I they may be identical or different.

Most preferably in connection with alkyl (also in alkylaryl, alkylheterocyclyl or alkylcycloalkyl), alkenyl, alkynyl or O-alkyl, substituted is understood in the context of this invention that any alkyl (also in alkylaryl, alkylheterocyclyl or alkylcycloalkyl), alkenyl, alkynyl or O-alkyl which is substituted with one or more of halogen (F, Cl, Br, I), -NRₖR_{k'}, -ORₖ, -CN, -SRₖ, haloalkyl, haloalkoxy, being Rₖ represented by R₁₃, R₁₅, R₇₁, R₈₁, R₈₂ or R₉₁, (being R_{k'} represented by R₁₃', R₁₅', R₇₁', R₈₁', R₈₂' or R₉₁'; wherein wherein R₁ to R₉₁" and Rₐ and R_{b} are as defined in the description, and wherein when different radicals R₁ to R₉₁" and Rₐ and R_{b} are present simultaneously in Formula I they may be identical or different.

More than one replacement on the same molecule and also on the same carbon atom is possible with the same or different substituents. This includes for example 3 hydrogens being replaced on the same C atom, as in the case of CF₃, or at different places of the same molecule, as in the case of e.g. -CH(OH)-CH=CH-CHCl₂.

In the context of this invention haloalkyl is understood as meaning an alkyl being substituted once or several times by a halogen (selected from F, Cl, Br, I). It encompasses e.g. -CH₂Cl, -CH₂F, -CHCl₂, -CHF₂, -CCl₃, -CF₃ and -CH₂-CHCl₂. Preferably haloalkyl is understood in the context of this invention as halogen-substituted C₁₋₄-alkyl representing halogen substituted C1-, C2-, C3- or C4-alkyl. The halogen-substituted alkyl radicals are thus preferably methyl, ethyl, propyl, and butyl. Preferred examples include -CH₂Cl, -CH₂F, -CHCl₂, -CHF₂, and -CF₃.

In the context of this invention haloalkoxy is understood as meaning an -O-alkyl being substituted once or several times by a halogen (selected from F, Cl, Br, I). It encompasses e.g. -OCH₂Cl, -OCH₂F, -OCHCl₂, -OCHF₂, -OCCl₃, -OCF₃ and - OCH₂-CHCl₂. Preferably haloalkoxy is understood in the context of this invention as halogen-substituted -OC₁₋₄-alkyl representing halogen substituted C1-, C2-, C3- or C4-alkoxy. The halogen-substituted alkyl radicals are thus preferably O-methyl, O-ethyl, O-propyl, and O-butyl. Preferred examples include -OCH₂Cl, -OCH₂F, -OCHCl₂, - OCHF₂, and -OCF₃.

In the context of this invention cycloalkyl is understood as meaning saturated and unsaturated (but not aromatic) cyclic hydrocarbons (without a heteroatom in the ring), which can be unsubstituted or once or several times substituted. Furthermore, C₃₋₄-cycloalkyl represents C3- or C4-cycloalkyl, C₃₋₅-cycloalkyl represents C3-, C4- or C5-cycloalkyl, C₃₋₆-cycloalkyl represents C3-, C4-, C5- or C6-cycloalkyl, C₃₋₇-cycloalkyl represents C3-, C4-, C5-, C6- or C7-cycloalkyl, C₃₋₈-cycloalkyl represents C3-, C4-, C5-, C6-, C7- or C8-cycloalkyl, C₄₋₅-cycloalkyl represents C4- or C5-cycloalkyl, C₄₋₆-cycloalkyl represents C4-, C5- or C6-cycloalkyl, C₄₋₇-cycloalkyl represents C4-, C5-, C6- or C7-cycloalkyl, C₅₋₆-cycloalkyl represents C5- or C6-cycloalkyl and C₅₋₇-cycloalkyl represents C5-, C6- or C7-cycloalkyl. Examples are cyclopropyl, 2-methylcyclopropyl, cyclopropylmethyl, cyclobutyl, cyclopentyl, cyclopentylmethyl, cyclohexyl, cycloheptyl, cyclooctyl, and also adamantyl. Preferably in the context of this invention cycloalkyl is C₃₋₈cycloalkyl like cyclopropyl, cyclobutyl, cyclopentyl, cyclohexyl, cycloheptyl, or cyclooctyl; or is C₃₋₇cycloalkyl like cyclopropyl, cyclobutyl, cyclopentyl, cyclohexyl, or cycloheptyl; or is C₃₋₆cycloalkyl like cyclopropyl, cyclobutyl, cyclopentyl or cyclohexyl, especially cyclopentyl or cyclohexyl.

Aryl is understood as meaning 5 to 18 membered mono or polycyclic ring systems with at least one aromatic ring but without heteroatoms even in only one of the rings. Examples are phenyl, naphthyl, fluoranthenyl, fluorenyl, tetralinyl or indanyl, 9H-fluorenyl or anthracenyl radicals, which can be unsubstituted or once or several times substituted. Most preferably aryl is understood in the context of this invention as phenyl, naphthyl or anthracenyl, preferably is phenyl.

A heterocyclyl radical or group (also called heterocyclyl hereinafter) is understood as meaning 5 to 18 membered mono or polycyclic heterocyclic ring systems, with at least one saturated or unsaturated ring which contains one or more heteroatoms selected from the group consisting of nitrogen, oxygen and/or sulfur in the ring. A heterocyclic group can also be substituted once or several times.

Subgroups inside the heterocyclyls as understood herein include heteroaryls and non-aromatic heterocyclyls.
- the heteroaryl (being equivalent to heteroaromatic radicals or aromatic heterocyclyls) is an aromatic 5 to 18 membered mono or polycyclic heterocyclic ring system of one or more rings of which at least one aromatic ring contains one or more heteroatoms selected from the group consisting of nitrogen, oxygen and/or sulfur in the ring; preferably is a 5 to 18 membered mono or polycyclic aromatic heterocyclic ring system of one or two rings of which at least one aromatic ring contains one or more heteroatoms selected from the group consisting of nitrogen, oxygen and/or sulfur in the ring, more preferably is selected from furan, benzofuran, thiophene, benzothiophene, pyrrole, pyridine, pyrimidine, pyrazine, quinoline, isoquinoline, phthalazine, benzothiazole, indole, benzotriazole, carbazole, quinazoline, thiazole, imidazole, pyrazole, oxazole, thiophene and benzimidazole;
- the non-aromatic heterocyclyl is a 5 to 18 membered mono or polycyclic heterocyclic ring system of one or more rings of which at least one ring - with this (or these) ring(s) then not being aromatic - contains one or more heteroatoms selected from the group consisting of nitrogen, oxygen and/or sulfur in the ring; preferably is a 5 to 18 membered mono or polycyclic heterocyclic ring system of one or two rings of which one or both rings - with this one or two rings then not being aromatic - contain/s one or more heteroatoms selected from the group consisting of nitrogen, oxygen and/or sulfur in the ring, more preferably is selected from oxazepam, pyrrolidine, piperidine, piperazine, tetrahydropyran, morpholine, indoline, oxopyrrolidine, benzodioxane, especially is benzodioxane, morpholine, tetrahydropyran, piperidine, oxopyrrolidine and pyrrolidine.
-

Preferably in the context of this invention heterocyclyl is defined as a 5 to 18 membered mono or polycyclic heterocyclic ring system of one or more saturated or unsaturated rings of which at least one ring contains one or more heteroatoms selected from the group consisting of nitrogen, oxygen and/or sulfur in the ring. Preferably it is a 5 to 18 membered mono or polycyclic heterocyclic ring system of one or two saturated or unsaturated rings of which at least one ring contains one or more heteroatoms selected from the group consisting of nitrogen, oxygen and/or sulfur in the ring.

Preferred examples of heterocyclyls include oxazepan, pyrrolidine, imidazole, oxadiazole, tetrazole, pyridine, pyrimidine, piperidine, piperazine, , benzofuran, benzimidazole, indazole, benzodiazole, thiazole, benzothiazole, tetrahydropyran, morpholine, indoline, furan, triazole, isoxazole, pyrazole, thiophene, benzothiophene, pyrrole, pyrazine, pyrrolo[2,3b]pyridine, quinoline, isoquinoline, tetrahydroisoquinoline, phthalazine, benzo-1,2,5-thiadiazole, indole, benzotriazole, benzoxazole oxopyrrolidine, pyrimidine, benzodioxolane, benzodioxane, carbazole and quinazoline, especially is pyridine, pyrazine, indazole, benzodioxane, thiazole, benzothiazole, morpholine, tetrahydropyran, pyrazole, imidazole, piperidine, thiophene, indole, benzimidazole, pyrrolo[2,3b]pyridine, benzoxazole, oxopyrrolidine, pyrimidine, oxazepane, azetidine and pyrrolidine.

In the context of this invention oxopyrrolidine is understood as meaning pyrrolidin-2-one.

An *N*-containing heterocyclyl is a heterocyclic ring system of one or more saturated or unsaturated rings of which at least one ring contains a nitrogen and optionally one or more further heteroatoms selected from the group consisting of nitrogen, oxygen and/or sulfur in the ring; preferably is a heterocyclic ring system of one or two saturated or unsaturated rings of which at least one ring contains a nitrogen and optionally one or more further heteroatoms selected from the group consisting of nitrogen, oxygen and/or sulfur in the ring, more preferably is selected from oxazepan, pyrrolidine, imidazole, oxadiazole, tetrazole, azetidine, pyridine, pyrimidine, piperidine, piperazine, benzimidazole, indazole, benzothiazole, benzodiazole, morpholine, indoline, triazole, isoxazole, pyrazole, pyrrole, pyrazine, pyrrolo[2,3b]pyridine, quinoline, quinolone, isoquinoline, tetrahydrothienopyridine, phthalazine, benzo-1,2,5-thiadiazole, indole, benzotriazole, benzoxazole oxopyrrolidine, carbazole or thiazole.

In the context of this invention, a cyclic amide is defined as a subgroup of a heterocyclyl (as defined above) formed through the cyclization of a carbon sequence, containing at least the sequence 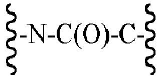 forming part of the cycle. Said cyclic amide may optionally be fused to a ring system. Preferably the cyclic amide is an "indoline-2-one". A cyclic amide may be substituted or unsubstituted as defined for heterocyclyl above.

In the context of this invention, a cyclic urea is defined as a subgroup of a heterocyclyl (as defined above) formed through the cyclization of a carbon sequence containing at least the sequence 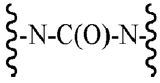 forming part of the cycle. Said cyclic urea may optionally be fused to a ring system. Preferably the cyclic urea is "1H-benzo[d]imidazol-2(3H)-one". A cyclic urea may be substituted or unsubstituted as defined for heterocyclyl above.

In connection with aromatic heterocyclyls (heteroaryls), non-aromatic heterocyclyls, aryls and cycloalkyls, when a ring system falls within two or more of the above cycle definitions simultaneously, then the ring system is defined first as an aromatic heterocyclyl (heteroaryl) if at least one aromatic ring contains a heteroatom. If no aromatic ring contains a heteroatom, then the ring system is defined as a non-aromatic heterocyclyl if at least one non-aromatic ring contains a heteroatom. If no non-aromatic ring contains a heteroatom, then the ring system is defined as an aryl if it contains at least one aryl cycle. If no aryl is present, then the ring system is defined as a cycloalkyl if at least one non-aromatic cyclic hydrocarbon is present.

In the context of this invention alkylaryl is understood as meaning an aryl group (see above) being connected to another atom through a C₁₋₆-alkyl (see above) which may be branched or linear and is unsubstituted or substituted once or several times. Preferably alkylaryl is understood as meaning an aryl group (see above) being connected to another atom through 1 to 4 (-CH₂-) groups. Most preferably alkylaryl is benzyl (i.e. -CH₂-phenyl). More preferably, the "alkyl" in alkylaryl is an unsubstitued alkyl.

In the context of this invention alkylheterocyclyl is understood as meaning an heterocyclyl group being connected to another atom through a C₁₋₆-alkyl (see above) which may be branched or linear and is unsubstituted or substituted once or several times. Preferably alkylheterocyclyl is understood as meaning an heterocyclyl group (see above) being connected to another atom through 1 to 4 (-CH₂-) groups. Most preferably alkylheterocyclyl is -CH₂-pyridine. More preferably, the "alkyl" in alkylheterocyclyl is an unsubstitued alkyl.

In the context of this invention alkylcycloalkyl is understood as meaning an cycloalkyl group being connected to another atom through a C₁₋₆-alkyl (see above) which may be branched or linear and is unsubstituted or substituted once or several times. Preferably alkylcycloalkyl is understood as meaning a cycloalkyl group (see above) being connected to another atom through 1 to 4 (-CH₂-) groups. Most preferably alkylcycloalkyl is -CH₂-cyclopropyl. More preferably, the "alkyl" in alkycycloalkyl is an unsubstitued alkyl.

Preferably, the aryl is a monocyclic aryl. More preferably the aryl is a 5, 6 or 7 membered monocyclic aryl. Even more preferably the aryl is a 5 or 6 membered monocyclic aryl.

Preferably, the heteroaryl is a monocyclic heteroaryl. More preferably the heteroaryl is a 5, 6 or 7 membered monocyclic heteroaryl. Even more preferably the heteroaryl is a 5 or 6 membered monocyclic heteroaryl.

Preferably, the non-aromatic heterocyclyl is a monocyclic non-aromatic heterocyclyl. More preferably the non-aromatic heterocyclyl is a 4, 5, 6 or 7 membered monocyclic non-aromatic heterocyclyl. Even more preferably the non-aromatic heterocyclyl is a 5 or 6 membered monocyclic non-aromatic heterocyclyl.

Preferably, the cycloalkyl is a monocyclic cycloalkyl. More preferably the cycloalkyl is a 3, 4, 5, 6, 7 or 8 membered monocyclic cycloalkyl. Even more preferably the cycloalkyl is a 3, 4, 5 or 6 membered monocyclic cycloalkyl.

An heterocyclyl is a heterocyclic ring system of one or more saturated or unsaturated rings of which at least one ring contains one or more heteroatoms selected from the group consisting of nitrogen, oxygen and/or sulfur in the ring; preferably is a heterocyclic ring system of one or two saturated or unsaturated rings of which at least one ring contains one or more heteroatoms selected from the group consisting of nitrogen, oxygen and/or sulfur in the ring, more preferably is selected from oxazepan, pyrrolidine, imidazole, oxadiazole, tetrazole, azetidine, pyridine, pyrimidine, piperidine, piperazine, benzofuran, benzimidazole, indazole, benzothiazole, benzodiazole, thiazole, benzothiazole, tetrahydropyran, morpholine, indoline, furan, triazole, isoxazole, pyrazole, thiophene, benzothiophene, pyrrole, pyrazine, pyrrolo[2,3b]pyridine, quinoline, quinolone, isoquinoline, tetrahydrothienopyridine, phthalazine, benzo-1,2,5-thiadiazole, indole, benzotriazole, benzoxazole oxopyrrolidine, benzodioxolane, benzodioxane, carbazole, oxaspirodecan or thiazole;

In general, such a heterocyclyl may contain between 3 and 32 atoms in the rings (preferably 4 to 20 atoms in the rings, or most preferably 5 to 18 atoms in the rings). Thus, a heterocyclyl may contain between 3 and 12 atoms in the ring (preferably 4 to 10 atoms in the ring, or 5 to 8 atoms in the ring, or 5 to 6 atoms in the ring) in case of a heterocyclyl of one saturated or unsaturated ring. Such a heterocyclyl may also contain between 5 and 22 atoms in both rings together (preferably 6 to 16 atoms in both rings together, or 7 to 12 atoms in both rings together or 8 to 10 atoms in both rings together) in case of a heterocyclyl of two saturated or unsaturated rings. Such a heterocyclyl may also contain between 7 and 32 atoms in the 3 rings together (preferably 10 to 22 atoms in the three rings together, or 12 to 20 atoms in the three rings together or 10 to 18 atoms in the three rings together) in case of a heterocyclyl of three saturated or unsaturated rings.

In connection with aryl (including alkyl-aryl), cycloalkyl (including alkyl-cycloalkyl), or heterocyclyl (including alkyl-heterocyclyl), substituted is understood - unless defined otherwise - as meaning substitution of the ring-system of the aryl or alkyl-aryl, cycloalkyl or alkyl-cycloalkyl; heterocyclyl or alkyl-heterocyclyl with one or more of halogen (F, Cl, Br, I), -Rₖ ,-ORₖ, -CN, -NO₂, -NRₖR_{k'}, -C(O)ORₖ, NRₖC(O)R_{k"}, -C(O)NRₖR_{k'}, - NRₖS(O)₂R_{k'}, =O, -OCH₂CH₂OH, -NRₖC(O)NR_{k'}R_{k"} -S(O)₂NRₖR_{k'}, -NRₖS(O)₂NR_{k'}R_{k"}, haloalkyl, haloalkoxy, -SRₖ, -S(O)Rₖ, -S(O)₂Rₖ or C(CH₃)ORₖ, or substituted or unsubstituted alkylcycloalkyl, substituted or unsubstituted alkylaryl, substituted or unsubstituted alkylheterocyclyl, with Rₖ, R_{k'} and R_{k"} independently being either H or a saturated or unsaturated, linear or branched, substituted or unsubstituted C₁₋₆-alkyl; a saturated or unsaturated, linear or branched, substituted or unsubstituted C₁₋₆-alkyl; a saturated or unsaturated, linear or branched, substituted or unsubstituted -O-C₁₋₆-alkyl (alkoxy); a saturated or unsaturated, linear or branched, substituted or unsubstituted - S-C₁₋₆-alkyl; a saturated or unsaturated, linear or branched, substituted or unsubstituted -C(O)-C₁₋₆-alkyl-group; a saturated or unsaturated, linear or branched, substituted or unsubstituted -C(O)-O-C₁₋₆-alkyl-group; a substituted or unsubstituted aryl or alkyl-aryl; a substituted or unsubstituted cycloalkyl or alkyl-cycloalkyl; a substituted or unsubstituted heterocyclyl or alkyl-heterocyclyl, being Rₖ one of R₁₄, R₈₁, R₈₂, R₈₃ or R₉₁, (being R_{k'} one of R₁₄', R₈₁', R₈₂', R₈₃' orR₉₁', being Rₖ" one of R₁₄", R₈₁", R₈₂", R₈₃" or R₉₁"; wherein R₁ to R₉₁" and Rₐ and R_{b} are as defined in the description, and wherein when different radicals R₁ to R₉₁" and Rₐ and R_{b} are present simultaneously in Formula I they may be identical or different.

Most preferably in connection with aryl (including alkyl-aryl), cycloalkyl (including alkyl-cycloalkyl), or heterocyclyl (including alkyl-heterocyclyl), substituted is understood in the context of this invention that any aryl, cycloalkyl and heterocyclyl which is substituted is substituted (also in an alyklaryl, alkylcycloalkyl or alkylheterocyclyl) with one or more of halogen (F, Cl, Br, I), -Rₖ ,-ORₖ, -CN, -NO₂, -NRₖR_{k"'}, NRₖC(O)R_{k"}, - NRₖS(O)₂R_{k'}, -S(O)₂NRₖR_{k'}, -NRₖC(O)NR_{k'}R_{k"}, haloalkyl, haloalkoxy, -SRₖ, -S(O)Rₖ or S(O)₂Rₖ, or substituted or unsubstituted alkylcycloalkyl, substituted or unsubstituted alkylaryl, substituted or unsubstituted alkylheterocyclyl, being Rₖ one of R₁₄, R₈₁, R₈₂, R₈₃ or R₉₁, (being R_{k'} one of R₁₄', R₈₁', R₈₂', R₈₃' or R₉₁'; being R_{k"} one of R₁₄", R₈₁", R₈₂", R₈₃" or R₉₁"; wherein R₁ to R₉₁" and Rₐ and R_{b} are as defined in the description, and wherein when different radicals R₁ to R₉₁" and Rₐ and R_{b} are present simultaneously in Formula I they may be identical or different.

In connection with cycloalkyl (including alkyl-cycloalkyl), or heterocyclyl (including alkylheterocyclyl) namely non-aromatic heterocyclyl (including non-aromatic alkyl-heterocyclyl), substituted is also understood - unless defined otherwise - as meaning substitution of the ring-system of the cycloalkyl or alkyl-cycloalkyl, non-aromatic heterocyclyl or non aromatic alkyl-heterocyclyl with 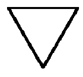 (leading to a spiro structure) and/or with =O.

Moreover, in connection with cycloalkyl (including alkyl-cycloalkyl), or heterocyclyl (including alkylheterocyclyl) namely non-aromatic heterocyclyl (including non-aromatic alkyl-heterocyclyl), substituted is also understood - unless defined otherwise - as meaning substitution of the ring-system of the cycloalkyl or alkyl-cycloalkyl, non-aromatic heterocyclyl or non aromatic alkyl-heterocyclyl is spirosubstituted or substituted with =O.

Moreover, in connection with cycloalkyl (including alkyl-cycloalkyl), or heterocyclyl (including alkylheterocyclyl) namely non-aromatic heterocyclyl (including non-aromatic alkyl-heterocyclyl), substituted is also understood - unless defined otherwise - as meaning substitution of the ring-system of the cycloalkyl or alkyl-cycloalkyl, non-aromatic heterocyclyl or non aromatic alkyl-heterocyclyl with =O.

A ring system is a system consisting of at least one ring of connected atoms but including also systems in which two or more rings of connected atoms (polycyclic ring system) are joined with "joined" meaning that the respective rings are sharing one (like a spiro structure), two or more atoms being a member or members of both joined rings.

The term "polycyclic ring system" means that the ring system is made of two or more rings joined by sharing at least one atom.

The term "leaving group" means a molecular fragment that departs with a pair of electrons in heterolytic bond cleavage. Leaving groups can be anions or neutral molecules. Common anionic leaving groups are halides such as Cl-, Br-, and I-, and sulfonate esters, such as tosylate (TsO-) or mesylate.

The term "salt" is to be understood as meaning any form of the active compound used according to the invention in which it assumes an ionic form or is charged and is coupled with a counter-ion (a cation or anion) or is in solution. By this are also to be understood complexes of the active compound with other molecules and ions, in particular complexes via ionic interactions.

The term "physiologically acceptable salt" means in the context of this invention any salt that is physiologically tolerated (most of the time meaning not being toxic-especially not caused by the counter-ion) if used appropriately for a treatment especially if used on or applied to humans and/or mammals.

These physiologically acceptable salts can be formed with cations or bases and in the context of this invention is understood as meaning salts of at least one of the compounds used according to the invention - usually a (deprotonated) acid - as an anion with at least one, preferably inorganic, cation which is physiologically tolerated - especially if used on humans and/or mammals. The salts of the alkali metals and alkaline earth metals are particularly preferred, and also those with NH₄, but in particular (mono)- or (di)sodium, (mono)- or (di)potassium, magnesium or calcium salts.

Physiologically acceptable salts can also be formed with anions or acids and in the context of this invention is understood as meaning salts of at least one of the compounds used according to the invention as the cation with at least one anion which are physiologically tolerated - especially if used on humans and/or mammals. By this is understood in particular, in the context of this invention, the salt formed with a physiologically tolerated acid, that is to say salts of the particular active compound with inorganic or organic acids which are physiologically tolerated - especially if used on humans and/or mammals. Examples of physiologically tolerated salts of particular acids are salts of: hydrochloric acid, hydrobromic acid, sulfuric acid, methanesulfonic acid, formic acid, acetic acid, oxalic acid, succinic acid, malic acid, tartaric acid, mandelic acid, fumaric acid, lactic acid or citric acid.

The compounds of the invention may be present in crystalline form or in the form of free compounds like a free base or acid.

Any compound that is a solvate of a compound according to the invention like a compound according to general formula I defined above is understood to be also covered by the scope of the invention. Methods of solvation are generally known within the art. Suitable solvates are pharmaceutically acceptable solvates. The term "solvate" according to this invention is to be understood as meaning any form of the active compound according to the invention in which this compound has attached to it via noncovalent binding another molecule (most likely a polar solvent). Especially preferred examples include hydrates and alcoholates, like methanolates or ethanolates.

Any compound that is a prodrug of a compound according to the invention like a compound according to general formula I defined above is understood to be also covered by the scope of the invention. The term "prodrug" is used in its broadest sense and encompasses those derivatives that are converted *in vivo* to the compounds of the invention. Such derivatives would readily occur to those skilled in the art, and include, depending on the functional groups present in the molecule and without limitation, the following derivatives of the present compounds: esters, amino acid esters, phosphate esters, metal salts sulfonate esters, carbamates, and amides. Examples of well known methods of producing a prodrug of a given acting compound are known to those skilled in the art and can be found e.g. in Krogsgaard-Larsen et al. "Textbook of Drug design and Discovery" Taylor & Francis (April 2002).

Any compound that is a N-oxide of a compound according to the invention like a compound according to general formula I defined above is understood to be also covered by the scope of the invention.

Unless otherwise stated, the compounds of the invention are also meant to include compounds which differ only in the presence of one or more isotopically enriched atoms. For example, compounds having the present structures except for the replacement of a hydrogen by a deuterium or tritium, or the replacement of a carbon by ¹³C- or ¹⁴C-enriched carbon or of a nitrogen by ¹⁵N-enriched nitrogen are within the scope of this invention. This would especially also apply to the provisos described above so that any mentioning of hydrogen or any "H" in a formula would also cover deuterium or tritium.

The compounds of formula (I) as well as their salts or solvates of the compounds are preferably in pharmaceutically acceptable or substantially pure form. By pharmaceutically acceptable form is meant, inter alia, having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I), or of its salts. This applies also to its solvates or prodrugs.

In a further embodiment the compound according to the invention of general Formula (I) is a compound wherein

-X-R₄ is - R₄ or -[C(RₐR_{b})]ₚ-R₄;

optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
Rₐ is selected from the group consisting of hydrogen and substituted or unsubstituted C₁₋₆ alkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
R_{b} is selected from the group consisting of hydrogen and substituted or unsubstituted C₁₋₆ alkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
Rₐ and R_{b}, taken together with the carbon atom to which they are attached, form a substituted or unsubstituted cycloalkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
p is 0, 1, 2, 3, 4 or 5;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
w₁ is nitrogen or carbon;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
w₂ is nitrogen or carbon;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
w₃ is nitrogen or carbon;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
r is 0, 1 or 2;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
v is 0, 1 or 2;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
m is 0, 1 or 2;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
n is 0, 1 or 2;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
t is 0, 1 or 2;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
R₁ is selected from the group consisting of hydrogen and substituted or unsubstituted C₁₋₆ alkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
R₂ is selected from the group consisting of -R₂₁ and -OR₂₁;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
R₃ is -R₃₁;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
R₄ is 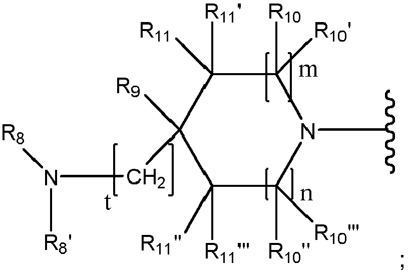 optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
R₅, R₅', R₅" and R₅'" are independently selected from the group consisting of hydrogen, halogen and substituted or unsubstituted C₁₋₆ alkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
R₅ and R₅' and/or R₅" and R₅'" taken together with the carbon atom to which they are attached form a carbonyl group;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
R₆, R₆', R₆" and R₆'" are independently selected from the group consisting of hydrogen and substituted or unsubstituted C₁₋₆ alkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
R₇ is selected from the group consisting of hydrogen and substituted or unsubstituted C₁₋₆ alkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
R₈ is selected from the group consisting of substituted or unsubstituted C₁₋₆ alkyl, substituted or unsubstituted cycloalkyl and substituted or unsubstituted alkylcycloalkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
R₈' is selected from the group consisting of hydrogen, substituted or unsubstituted C₁₋₆ alkyl, substituted or unsubstituted cycloalkyl and substituted or unsubstituted alkylcycloalkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
R₈ and R₈' taken together with the nitrogen atom to which they are attached form a substituted or unsubstituted heterocyclyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
R₉ is selected from the group consisting of substituted or unsubstituted C₁₋₆ alkyl, substituted or unsubstituted cycloalkyl, substituted or unsubstituted aryl, substituted or unsubstituted heterocyclyl, substituted or unsubstituted alkylcycloalkyl, substituted or unsubstituted alkylaryl and substituted or unsubstituted alkylheterocyclyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
R₉ is substituted or unsubstituted aryl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
R₁₀, R₁₀', R₁₀" and R₁₀'" are independently selected from the group consisting of hydrogen, halogen and substituted or unsubstituted C₁₋₆ alkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
R₁₀ and R₁₀' and/or R₁₀" and R₁₀'" taken together with the carbon atom to which they are attached form a substituted or unsubstituted cycloalkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
R₁₁, R₁₁', R₁₁" and R₁₁'" are independently selected from the group consisting of hydrogen, halogen and substituted or unsubstituted C₁₋₆ alkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
R₁₁ and R₁₁' and/or R₁₁" and R₁₁'" taken together with the carbon atom to which they are attached form a substituted or unsubstituted cycloalkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
R₁₃ and R₁₃' are independently selected from the group consisting of hydrogen and unsubstituted C₁₋₆ alkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
R₁₄, R₁₄' and R₁₄" are independently selected from the group consisting of hydrogen, unsubstituted C₁₋₆ alkyl, unsubstituted aryl, unsubstituted cycloalkyl and unsubstituted heterocyclyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
R₁₅ and R₁₅' are independently selected from the group consisting of hydrogen and substituted or unsubstituted C₁₋₆ alkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
R₂₁, R₂₁' and R₂₁" are independently selected from the group consisting of hydrogen, substituted or unsubstituted C₁₋₆ alkyl and substituted or unsubstituted alkylcycloalkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
R₃₁, R₃₁' and R₃₁" are independently selected from the group consisting of hydrogen and substituted or unsubstituted C₁₋₆ alkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
R₇₁ and R₇₁' are independently selected from the group consisting of hydrogen and substituted or unsubstituted C₁₋₆ alkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
R₈₁, R₈₁' and R₈₁" are independently selected from the group consisting of hydrogen and substituted or unsubstituted C₁₋₆ alkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
R₈₂, R₈₂' and R₈₂" are independently selected from the group consisting of hydrogen and substituted or unsubstituted C₁₋₆ alkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
R₈₃, R₈₃' and R₈₃" are independently selected from the group consisting of hydrogen and substituted or unsubstituted C₁₋₆ alkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
R₉₁, R₉₁' and R₉₁" are independently selected from the group consisting of the group consisting of hydrogen and substituted or unsubstituted C₁₋₆ alkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
R₁ is selected from the group consisting of hydrogen, substituted or unsubstituted C₁₋₆ alkyl, substituted or unsubstituted C₂₋₆ alkenyl and substituted or unsubstituted C₂₋₆ alkynyl;
wherein
the alkyl, alkenyl or alkynyl defined in R₁, if substituted, is substituted with one or more substituent/s selected from the group consisting of -OR₁₅, halogen, -CN, haloalkyl, haloalkoxy and -NR₁₅R₁₅';
wherein R₁₅ and R₁₅' are independently selected from the group consisting of hydrogen, substituted or unsubstituted C₁₋₆ alkyl, substituted or unsubstituted C₂₋₆ alkenyl, and substituted or unsubstituted C₂₋₆ alkynyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
R₇ is selected from the group consisting of hydrogen, substituted or unsubstituted C₁₋₆ alkyl, substituted or unsubstituted C₂₋₆ alkenyl and substituted or unsubstituted C₂₋₆ alkynyl;
wherein
the alkyl, alkenyl or alkynyl defined in R₇, if substituted, is substituted with one or more substituent/s selected from the group consisting of -OR₇₁, halogen, -CN, haloalkyl, haloalkoxy and -NR₇₁R₇₁';
wherein R₇₁ and R₇₁' are independently selected from the group consisting of hydrogen, substituted or unsubstituted C₁₋₆ alkyl, substituted or unsubstituted C₂₋₆ alkenyl, and substituted or unsubstituted C₂₋₆ alkynyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
R₈ is selected from the group consisting of substituted or unsubstituted C₁₋₆ alkyl, substituted or unsubstituted C₂₋₆ alkenyl, substituted or unsubstituted C₂₋₆ alkynyl, substituted or unsubstituted cycloalkyl and substituted or unsubstituted alkylcycloalkyl;
wherein
the alkyl, alkenyl or alkynyl defined in R₈, if substituted, is substituted with one or more substituent/s selected from the group consisting of - OR₈₁, halogen, -CN, haloalkyl, haloalkoxy and -NR₈₁R₈₁';
wherein
the cycloalkyl defined in R₈, also in alkylcycloalkyl, if substituted, is substituted with one or more substituent/s selected from the group consisting of =O, halogen, -R₈₁, -OR₈₁, -NO₂, -NR₈₁R₈₁', -NR₈₁C(O)R₈₁', -NR₈₁S(O)₂R₈₁', -S(O)₂NR₈₁R₈₁', - NR₈₁C(O)NR₈₁'R₈₁", -SR₈₁ , -S(O)R₈₁, -S(O)₂R₈₁, -CN, haloalkyl, haloalkoxy, -C(O)OR₈₁, -C(O)NR₈₁R₈₁',-OCH₂CH₂OR₈₁, -NR₈₁S(O)₂NR₈₁'R₈₁" and -C(CH₃)₂OR₈₁;
wherein R₈₁, R₈₁' and R₈₁" are independently selected from the group consisting of hydrogen, substituted or unsubstituted C₁₋₆ alkyl, substituted or unsubstituted C₂₋₆ alkenyl and substituted or unsubstituted C₂₋₆ alkynyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein

R₈' is selected from the group consisting of hydrogen, substituted or unsubstituted C₁₋₆ alkyl, substituted or unsubstituted C₂₋₆ alkenyl, substituted or unsubstituted C₂₋₆ alkynyl, substituted or unsubstituted cycloalkyl and substituted or unsubstituted alkylcycloalkyl;
wherein
the alkyl, alkenyl or alkynyl defined in R₈', if substituted, is substituted with one or more substituent/s selected from the group consisting of -OR₈₂, halogen, -CN, haloalkyl, haloalkoxy and -NR₈₂R₈₂';
the cyclolakyl defined in R₈', also in alkylcycloalkyl, if substituted, is substituted with one or more substituent/s selected from the group consisting of =O, halogen, -R₈₂, -OR₈₂, -NO₂, -NR₈₂R₈₂', -NR₈₂C(O)R₈₂',-NR₈₂S(O)₂R₈₂', -S(O)NR₈₂R₈₂', - NR₈₂C(O)NR₈₂'R₈₂", -SR₈₂, -S(O)R₈₂,-S(O)₂R₈₂, -CN, haloalkyl, haloalkoxy, -C(O)OR₈₂, -C(O)NR₈₂R₈₂',-OCH₂CH₂OR₈₂, -NR₈₂S(O)₂NR₈₂'R₈₂" and -C(CH₃)₂OR₈₂;
wherein R₈₂, R₈₂' and R₈₂" are independently selected from the group consisting of hydrogen, substituted or unsubstituted C₁₋₆ alkyl, substituted or unsubstituted C₂₋₆ alkenyl, and substituted or unsubstituted C₂₋₆ alkynyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
R₈ and R₈' taken together with the nitrogen atom to which they are attached form a substituted or unsubstituted heterocyclyl;
wherein the heterocyclyl, as defined in R₈-R₈', if substituted, is substituted with one or more substituent/s selected from the group consisting of =O, halogen, -R₈₃, -OR₈₃, -NO₂, -NR₈₃R₈₃', -NR₈₃C(O)R₈₃', -NR₈₃S(O)₂R₈₃',-S(O)₂NR₈₃R₈₃', - NR₈₃C(O)NR₈₃'R₈₃", -SR₈₃ , -S(O)R₈₃, -S(O)₂R₈₃, -CN, haloalkyl, haloalkoxy, -C(O)OR₈₃, -C(O)NR₈₃R₈₃', -OCH₂CH₂OR₈₃,-NR₈₃S(O)₂NR₈₃'R₈₃" and -C(CH₃)₂OR₈₃;
wherein R₈₃, R₈₃' and R₈₃" are independently selected from the group consisting of hydrogen, substituted or unsubstituted C₁₋₆ alkyl, substituted or unsubstituted C₂₋₆ alkenyl, and substituted or unsubstituted C₂₋₆ alkynyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
R₉ is selected from the group consisting of substituted or unsubstituted C₁₋₆ alkyl, substituted or unsubstituted C₂₋₆ alkenyl, substituted or unsubstituted C₂₋₆ alkynyl, substituted or unsubstituted cycloalkyl, substituted or unsubstituted aryl, substituted or unsubstituted heterocyclyl, substituted or unsubstituted alkylcycloalkyl, substituted or unsubstituted alkylaryl and substituted or unsubstituted alkylheterocyclyl;
wherein
the alkyl, alkenyl or alkynyl defined in R₉, if substituted, is substituted with one or more substituent/s selected from the group consisting of -OR₉₁, halogen, -CN, haloalkyl, haloalkoxy and -NR₉₁R₉₁';
the cycloalkyl, aryl or heterocyclyl, also in alkylcycloalkyl, alkylaryl or alkylheterocyclyl, as defined in R₉, if substituted, is substituted with one or more substituent/s selected from the group consisting of halogen, -R₉₁,-OR₉₁, -NO₂, -NR₉₁R₉₁', -NR₉₁C(O)R₉₁', -NR₉₁S(O)₂R₉₁', -S(O)₂NR₉₁R₉₁',-NR₉₁C(O)NR₉₁'R₉₁", -SR₉₁, -S(O)R₉₁, -S(O)₂R₉₁, -CN, haloalkyl, haloalkoxy, -C(O)OR₉₁, -OC(O)R₉₁, -C(O)NR₉₁R₉₁', -OCH₂CH₂OR₉₁,-NR₉₁S(O)₂NR₉₁'R₉₁" and -C(CH₃)₂OR₉₁;
wherein R₉₁, R₉₁' and R₉₁" are independently selected from the group consisting of the group consisting of hydrogen, substituted or unsubstituted C₁₋₆ alkyl, substituted or unsubstituted C₂₋₆ alkenyl, and substituted or unsubstituted C₂₋₆ alkynyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
the alkyl, alkenyl or alkynyl, if substituted and the substitution has not been defined otherwise, it is substituted with one or more substituent/s selected from the group consisting of -OR₁₃, halogen, -CN, haloalkyl, haloalkoxy and -NR₁₃R₁₃';
wherein R₁₃ and R₁₃' are independently selected from the group consisting of hydrogen, unsubstituted C₁₋₆ alkyl, unsubstituted C₂₋₆ alkenyl and unsubstituted C₂₋₆ alkynyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
the aryl, heterocyclyl or cycloalkyl, also in alkylaryl, alkylheterocyclyl or alkylcycloalkyl, if substituted and the substitution has not been defined otherwise, it is substituted with one or more substituent/s selected from the group consisting of halogen, -R₁₄, -OR₁₄,-NO₂, -NR₁₄R₁₄', -NR₁₄C(O)R₁₄', -NR₁₄S(O)₂R₁₄', -S(O)₂NR₁₄R₁₄', - NR₁₄C(O)NR₁₄'R₁₄", -SR₁₄ , -S(O)R₁₄, -S(O)₂R₁₄, -CN, haloalkyl, haloalkoxy, -C(O)OR₁₄, -C(O)NR₁₄R₁₄',-OCH₂CH₂OR₁₄, -NR₁₄S(O)₂NR₁₄'R₁₄" and -C(CH₃)₂OR₁₄;
wherein R₁₄, R₁₄' and R₁₄" are independently selected from the group consisting of hydrogen, unsubstituted C₁₋₆ alkyl, unsubstituted C₂₋₆ alkenyl, unsubstituted C₂₋₆ alkynyl, unsubstituted aryl, unsubstituted cycloalkyl and unsubstituted heterocyclyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
the alkyl, alkenyl or alkynyl defined in R₁, if substituted, is substituted with one or more substituent/s selected from the group consisting of -OR₁₅, halogen, -CN, haloalkyl, haloalkoxy and -NR₁₅R₁₅';
wherein R₁₅ and R₁₅' are independently selected from the group consisting of hydrogen, substituted or unsubstituted C₁₋₆ alkyl, substituted or unsubstituted C₂₋₆ alkenyl, and substituted or unsubstituted C₂₋₆ alkynyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
the alkyl, alkenyl or alkynyl defined in R₇, if substituted, is substituted with one or more substituent/s selected from the group consisting of -OR₇₁, halogen, -CN, haloalkyl, haloalkoxy and -NR₇₁R₇₁';
wherein R₇₁ and R₇₁' are independently selected from the group consisting of hydrogen, substituted or unsubstituted C₁₋₆ alkyl, substituted or unsubstituted C₂₋₆ alkenyl, and substituted or unsubstituted C₂₋₆ alkynyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
the alkyl, alkenyl or alkynyl defined in R₈, if substituted, is substituted with one or more substituent/s selected from the group consisting of-OR₈₁, halogen, -CN, haloalkyl, haloalkoxy and -NR₈₁R₈₁';
wherein
the cycloalkyl defined in R₈, also in alkylcycloalkyl, if substituted, is substituted with one or more substituent/s selected from the group consisting of =O, halogen, -R₈₁, -OR₈₁, -NO₂, -NR₈₁R₈₁', -NR₈₁C(O)R₈₁', -NR₈₁S(O)₂R₈₁', -S(O)₂NR₈₁R₈₁', -NR₈₁C(O)NR₈₁'R₈₁", -SR₈₁, -S(O)R₈₁, -S(O)₂R₈₁, -CN, haloalkyl, haloalkoxy, -C(O)OR₈₁, -C(O)NR₈₁R₈₁',-OCH₂CH₂OR₈₁, -NR₈₁S(O)₂NR₈₁'R₈₁" and -C(CH₃)₂OR₈₁;
wherein R₈₁, R₈₁' and R₈₁" are independently selected from the group consisting of hydrogen, substituted or unsubstituted C₁₋₆ alkyl, substituted or unsubstituted C₂₋₆ alkenyl and substituted or unsubstituted C₂₋₆ alkynyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
the alkyl, alkenyl or alkynyl defined in R₈', if substituted, is substituted with one or more substituent/s selected from the group consisting of -OR₈₂, halogen, -CN, haloalkyl, haloalkoxy and -NR₈₂R₈₂';
the cyclolakyl defined in R₈', also in alkylcycloalkyl, if substituted, is substituted with one or more substituent/s selected from the group consisting of =O, halogen, -R₈₂, -OR₈₂, -NO₂, -NR₈₂R₈₂', -NR₈₂C(O)R₈₂',-NR₈₂S(O)₂R₈₂', -S(O)₂NR₈₂R₈₂', -NR₈₂C(O)NR₈₂'R₈₂", -SR₈₂ , -S(O)R₈₂,-S(O)₂R₈₂, -CN, haloalkyl, haloalkoxy, -C(O)OR₈₂, -C(O)NR₈₂R₈₂',-OCH₂CH₂OR₈₂, -NR₈₂S(O)₂NR₈₂'R₈₂" and -C(CH₃)₂OR₃₂;
wherein R₈₂, R₈₂' and R₈₂" are independently selected from the group consisting of hydrogen, substituted or unsubstituted C₁₋₆ alkyl, substituted or unsubstituted C₂₋₆ alkenyl, and substituted or unsubstituted C₂₋₆ alkynyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
wherein the heterocyclyl, as defined in R₈-R₈', if substituted, is substituted with one or more substituent/s selected from the group consisting of =O, halogen, -R₈₃, -OR₈₃, -NO₂, -NR₈₃R₈₃', -NR₈₃C(O)R₈₃', -NR₈₃S(O)₂R₈₃',-S(O)₂NR₈₃R₈₃', - NR₈₃C(O)NR₈₃'R₈₃", -SR₈₃, -S(O)R₈₃, -S(O)₂R₈₃, -CN, haloalkyl, haloalkoxy, -C(O)OR₃₃, -C(O)NR₈₃R₈₃', -OCH₂CH₂OR₈₃,-NR₈₃S(O)₂NR₈₃'R₈₃" and -C(CH₃)₂OR₈₃;
wherein R₈₃, R₈₃' and R₈₃" are independently selected from the group consisting of hydrogen, substituted or unsubstituted C₁₋₆ alkyl, substituted or unsubstituted C₂₋₆ alkenyl, and substituted or unsubstituted C₂₋₆ alkynyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
the alkyl, alkenyl or alkynyl defined in R₉, if substituted, is substituted with one or more substituent/s selected from the group consisting of -OR₉₁, halogen, -CN, haloalkyl, haloalkoxy and -NR₉₁R₉₁';
the cycloalkyl, aryl or heterocyclyl, also in alkylcycloalkyl, alkylaryl or alkylheterocyclyl, as defined in R₉, if substituted, is substituted with one or more substituent/s selected from the group consisting of halogen, -R₉₁,-OR₉₁, -NO₂, -NR₉₁R₉₁', -NR₉₁C(O)R₉₁', -NR₉₁S(O)₂R₉₁', -S(O)₂NR₉₁R₉₁',-NR₉₁C(O)NR₉₁'R₉₁", -SR₉₁, -S(O)R₉₁, -S(O)₂R₉₁, -CN, haloalkyl, haloalkoxy, -C(O)OR₉₁, -OC(O)R₉₁, -C(O)NR₉₁R₉₁', -OCH₂CH₂OR₉₁,-NR₉₁S(O)₂NR₉₁'R₉₁" and -C(CH₃)₂OR₉₁;
wherein R₉₁, R_{91'} and R₉₁" are independently selected from the group consisting of the group consisting of hydrogen, substituted or unsubstituted C₁₋₆ alkyl, substituted or unsubstituted C₂₋₆ alkenyl, and substituted or unsubstituted C₂₋₆ alkynyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein
X is selected from the group consisting of a bond and -[C(RₐR_{b})]ₚ-;
and/or
Rₐ is selected from the group consisting of hydrogen, substituted or unsubstituted C₁₋₆ alkyl, substituted or unsubstituted C₂₋₆ alkenyl and substituted or unsubstituted C₂₋₆ alkynyl;
and/or
R_{b} is selected from the group consisting of hydrogen, substituted or unsubstituted C₁₋₆ alkyl, substituted or unsubstituted C₂₋₆ alkenyl and substituted or unsubstituted C₂₋₆ alkynyl;
and/or
Rₐ and R_{b}, taken together with the carbon atom to which they are attached, form a substituted or unsubstituted cycloalkyl;
and/or
p is 0, 1, 2, 3, 4 or 5;
and/or
w₁ is nitrogen or carbon;
and/or
w₂ is nitrogen or - carbon;
and/or
w₃ is nitrogen or carbon;
and/or
r is 0, 1 or 2;
and/or
v is 0, 1 or 2;
and/or
R₁ is selected from the group consisting of hydrogen, substituted or unsubstituted C₁₋₆ alkyl, substituted or unsubstituted C₂₋₆ alkenyl and substituted or unsubstituted C₂₋₆ alkynyl;
and/or
R₂ is selected from the group consisting of -R₂₁, -OR₂₁, -NO₂, halogen, -NR₂₁R₂₁',-NR₂₁C(O)R₂₁', -NR₂₁S(O)₂R₂₁', -S(O)₂NR₂₁R₂₁', -NR₂₁C(O)NR₂₁'R₂₁", -SR₂₁, -S(O)R₂₁, -S(O)₂R₂₁, -CN, haloalkyl, haloalkoxy, -C(O)OR₂₁, -C(O)NR₂₁R₂₁', -OCH₂CH₂OR₂₁,-NR₂₁S(O)₂NR₂₁'R₂₁" and -C(CH₃)₂OR₂₁;
and/or
R₃ is selected from the group consisting of -R₃₁, -OR₃₁, -NO₂, halogen, -NR₃₁R₃₁',-NR₃₁C(O)R₃₁', -NR₃₁S(O)₂R₃₁', -S(O)₂NR₃₁R₃₁', - NR₃₁C(O)NR₃₁'R₃₁", -SR₃₁ , -S(O)R₃₁, -S(O)₂R₃₁, -CN, haloalkyl, haloalkoxy, -C(O)OR₃₁, -C(O)NR₃₁R₃₁', -OCH₂CH₂OR₃₁,-NR₃₁S(O)₂NR₃₁'R₃₁" and -C(CH₃)₂OR₃₁;
and/or
R₄ is 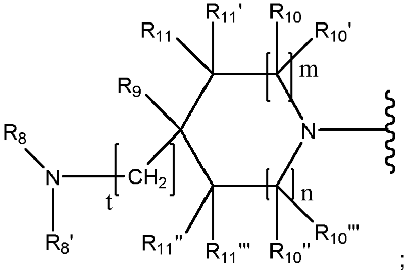 and/or
m is 0, 1 or 2;
and/or
n is 0, 1 or 2;
and/or
t is 0, 1 or 2;
and/or
R₅, R₅', R₅" and R₅'" are independently selected from the group consisting of hydrogen, halogen, substituted or unsubstituted C₁₋₆ alkyl, substituted or unsubstituted C₂₋₆ alkenyl and substituted or unsubstituted C₂₋₆ alkynyl;
and/or
R₅ and R₅' and/or R₅" and R₅'" taken together with the carbon atom to which they are attached form a carbonyl group;
and/or
R₆, R₆', R₆" and R₆'" are independently selected from the group consisting of hydrogen, substituted or unsubstituted C₁₋₆ alkyl, substituted or unsubstituted C₂₋₆ alkenyl and substituted or unsubstituted C₂₋₆ alkynyl;
and/or
R₇ is selected from the group consisting of hydrogen, substituted or unsubstituted C₁₋₆ alkyl, substituted or unsubstituted C₂₋₆ alkenyl and substituted or unsubstituted C₂₋₆ alkynyl;
and/or
R₈ is selected from the group consisting of substituted or unsubstituted C₁₋₆ alkyl, substituted or unsubstituted C₂₋₆ alkenyl, substituted or unsubstituted C₂₋₆ alkynyl, substituted or unsubstituted cycloalkyl and substituted or unsubstituted alkylcycloalkyl;
and/or

R₈' is selected from the group consisting of hydrogen, substituted or unsubstituted C₁₋₆ alkyl, substituted or unsubstituted C₂₋₆ alkenyl, substituted or unsubstituted C₂₋₆ alkynyl, substituted or unsubstituted cycloalkyl and substituted or unsubstituted alkylcycloalkyl;
and/or
R₈ and R₈' taken together with the nitrogen atom to which they are attached form a substituted or unsubstituted heterocyclyl;
and/or
R₉ is selected from the group consisting of substituted or unsubstituted C₁₋₆ alkyl, substituted or unsubstituted C₂₋₆ alkenyl, substituted or unsubstituted C₂₋₆ alkynyl, substituted or unsubstituted cycloalkyl, substituted or unsubstituted aryl, substituted or unsubstituted heterocyclyl, substituted or unsubstituted alkylcycloalkyl, substituted or unsubstituted alkylaryl and substituted or unsubstituted alkylheterocyclyl;
and/or
R₁₀, R₁₀', R₁₀" and R₁₀'" are independently selected from the group consisting of hydrogen, halogen, substituted or unsubstituted C₁₋₆ alkyl, substituted or unsubstituted C₂₋₆ alkenyl and substituted or unsubstituted C₂₋₆ alkynyl;
and/or
R₁₀ and R₁₀' and/or R₁₀" and R₁₀'" taken together with the carbon atom to which they are attached form a substituted or unsubstituted cycloalkyl;
and/or
R₁₀ and R₁₀' and/or R₁₀" and R₁₀'" taken together with the carbon atom to which they are attached form a carbonyl group;
and/or
R₁₁, R₁₁', R₁₁" and R₁₁'" are independently selected from the group consisting of hydrogen, halogen, substituted or unsubstituted C₁₋₆ alkyl, substituted or unsubstituted C₂₋₆ alkenyl and substituted or unsubstituted C₂₋₆ alkynyl;
and/or
R₁₁ and R₁₁' and/or R₁₁" and R₁₁'" taken together with the carbon atom to which they are attached form a substituted or unsubstituted cycloalkyl;
and/or
R₁₃ and R₁₃' are independently selected from the group consisting of hydrogen, unsubstituted C₁₋₆ alkyl, unsubstituted C₂₋₆ alkenyl and unsubstituted C₂₋₆ alkynyl;
and/or
R₁₄, R₁₄' and R₁₄" are independently selected from the group consisting of hydrogen, unsubstituted C₁₋₆ alkyl, unsubstituted C₂₋₆ alkenyl, unsubstituted C₂₋₆ alkynyl, unsubstituted aryl, unsubstituted cycloalkyl and unsubstituted heterocyclyl;
and/or
R₁₅ and R₁₅' are independently selected from the group consisting of hydrogen, substituted or unsubstituted C₁₋₆ alkyl, substituted or unsubstituted C₂₋₆ alkenyl, and substituted or unsubstituted C₂₋₆ alkynyl;
and/or
R₁₆ and R₁₆' are independently selected from halogen, -R₉₁, -OR₉₁, -N0₂, -NR₉₁R₉₁',-NR₉₁C(O)R₉₁', -NR₉₁S(O)₂R₉₁', -S(O)₂NR₉₁R₉₁', -NR₉₁C(O)NR₉₁'R₉₁", -SR₉₁, -S(O)R₉₁, -S(O)₂R₉₁, -CN, haloalkyl, haloalkoxy, -C(O)OR₉₁, -OC(O)R₉₁, -C(O)NR₉₁R₉₁',-OCH₂CH₂OR₉₁, -NR₉₁S(O)₂NR₉₁'R₉₁" and -C(CH₃)₂OR₉₁;
and/or
R₂₁, R₂₁' and R₂₁" are independently selected from the group consisting of hydrogen, substituted or unsubstituted C₁₋₆ alkyl, substituted or unsubstituted C₂₋₆ alkenyl, substituted or unsubstituted C₂₋₆ alkynyl and substituted or unsubstituted alkylcycloalkyl;
and/or
R₃₁, R₃₁' and R₃₁" are independently selected from the group consisting of hydrogen, substituted or unsubstituted C₁₋₆ alkyl, substituted or unsubstituted C₂₋₆ alkenyl and substituted or unsubstituted C₂₋₆ alkynyl;
and/or
R₇₁ and R₇₁' are independently selected from the group consisting of hydrogen, substituted or unsubstituted C₁₋₆ alkyl, substituted or unsubstituted C₂₋₆ alkenyl, and substituted or unsubstituted C₂₋₆ alkynyl;
and/or
R₈₁, R₈₁' and R₈₁" are independently selected from the group consisting of hydrogen, substituted or unsubstituted C₁₋₆ alkyl, substituted or unsubstituted C₂₋₆ alkenyl and substituted or unsubstituted C₂₋₆ alkynyl;
and/or
R₈₂, R₈₂' and R₈₂" are independently selected from the group consisting of hydrogen, substituted or unsubstituted C₁₋₆ alkyl, substituted or unsubstituted C₂₋₆ alkenyl, and substituted or unsubstituted C₂₋₆ alkynyl;
and/or
R₈₃, R₈₃' and R₈₃" are independently selected from the group consisting of hydrogen, substituted or unsubstituted C₁₋₆ alkyl, substituted or unsubstituted C₂₋₆ alkenyl, and substituted or unsubstituted C₂₋₆ alkynyl;
and/or
R₉₁, R₉₁' and R₉₁" are independently selected from the group consisting of the group consisting of hydrogen, substituted or unsubstituted C₁₋₆ alkyl, substituted or unsubstituted C₂₋₆ alkenyl, and substituted or unsubstituted C₂₋₆ alkynyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein in Rₐ as defined in any of the embodiments of the present invention,
the C₁₋₆ alkyl is preferably selected from methyl, ethyl, propyl, butyl, pentyl, hexyl, isopropyl, or 2-methylpropyl;
and/or
the C₂₋₆ -alkenyl is preferably selected from ethylene, propylene, butylene, pentylene, hexylene, isopropylene and isobutylene;
and/or
the C₂₋₆ -alkynyl is preferably selected from ethyne, propyne, butyne, pentyne, hexyne, isopropyne and isobutyne;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein in R_{b} as defined in any of the embodiments of the present invention,
the C₁₋₆ alkyl is preferably selected from methyl, ethyl, propyl, butyl, pentyl, hexyl, isopropyl, or 2-methylpropyl;
and/or
the C₂₋₆ -alkenyl is preferably selected from ethylene, propylene, butylene, pentylene, hexylene, isopropylene and isobutylene;
and/or
the C₂₋₆ -alkynyl is preferably selected from ethyne, propyne, butyne, pentyne, hexyne, isopropyne and isobutyne;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein in Rₐ and R_{b} as defined in any of the embodiments of the present invention,
the cycloalkyl is C₃₋₈ cycloalkyl like cyclopropyl, cyclobutyl, cyclopentyl, cyclohexyl, cycloheptyl, or cyclooctyl; preferably is C₃₋₇ cycloalkyl like cyclopropyl, cyclobutyl, cyclopentyl, cyclohexyl, or cycloheptyl; more preferably from C₃₋₆ cycloalkyl like cyclopropyl, cyclobutyl, cyclopentyl or cyclohexyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein in R₁ as defined in any of the embodiments of the present invention,
the C₁₋₆ alkyl is preferably selected from methyl, ethyl, propyl, butyl, pentyl, hexyl, isopropyl, or 2-methylpropyl; more preferably the C₁₋₆ alkyl is ethyl;
and/or
the C₂₋₆ -alkenyl is preferably selected from ethylene, propylene, butylene, pentylene, hexylene, isopropylene and isobutylene;
and/or
the C₂₋₆ -alkynyl is preferably selected from ethyne, propyne, butyne, pentyne, hexyne, isopropyne and isobutyne;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein in R₂ as defined in any of the embodiments of the present invention,
the alkyl in haloalkyl or haloalkoxy is C₁₋₆ alkyl like methyl, ethyl, propyl, butyl,pentyl, hexyl, isopropyl, or 2-methylpropyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein in R₃ as defined in any of the embodiments of the present invention,
the alkyl in haloalkyl or haloalkoxy is C₁₋₆ alkyl like methyl, ethyl, propyl, butyl,pentyl, hexyl, isopropyl, or 2-methylpropyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein in R₅, R₅', R₅" and R₅'" as defined in any of the embodiments of the present invention,
the C₁₋₆ alkyl is preferably selected from methyl, ethyl, propyl, butyl, pentyl, hexyl, isopropyl, or 2-methylpropyl;
and/or
the C₂₋₆ -alkenyl is preferably selected from ethylene, propylene, butylene, pentylene, hexylene, isopropylene and isobutylene;
and/or
the C₂₋₆ -alkynyl is preferably selected from ethyne, propyne, butyne, pentyne, hexyne, isopropyne and isobutyne;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein in R₆, R₆', R₆" and R₆'" as defined in any of the embodiments of the present invention,
the C₁₋₆ alkyl is preferably selected from methyl, ethyl, propyl, butyl, pentyl, hexyl, isopropyl, or 2-methylpropyl; preferably the C₁₋₆ alkyl is methyl;
and/or
the C₂₋₆ -alkenyl is preferably selected from ethylene, propylene, butylene, pentylene, hexylene, isopropylene and isobutylene;
and/or
the C₂-₆ -alkynyl is preferably selected from ethyne, propyne, butyne, pentyne, hexyne, isopropyne and isobutyne;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein in R₇ as defined in any of the embodiments of the present invention,
the C₁₋₆ alkyl is preferably selected from methyl, ethyl, propyl, butyl, pentyl, hexyl, isopropyl, or 2-methylpropyl;
and/or
the C₂₋₆ -alkenyl is preferably selected from ethylene, propylene, butylene, pentylene, hexylene, isopropylene and isobutylene;
and/or
the C₂₋₆ -alkynyl is preferably selected from ethyne, propyne, butyne, pentyne, hexyne, isopropyne and isobutyne;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein in R₈ as defined in any of the embodiments of the present invention,
the alkyl in alkylcycloalkyl is C₁₋₆ alkyl like methyl, ethyl, propyl, butyl,pentyl, hexyl, isopropyl, or 2-methylpropyl;
and/or
the C₁₋₆ alkyl is preferably selected from methyl, ethyl, propyl, butyl, pentyl, hexyl, isopropyl, or 2-methylpropyl; more preferably the C₁₋₆ alkyl is methyl;
and/or
the C₂₋₆ -alkenyl is preferably selected from ethylene, propylene, butylene, pentylene, hexylene, isopropylene and isobutylene;
and/or
the C₂₋₆ -alkynyl is preferably selected from ethyne, propyne, butyne, pentyne, hexyne, isopropyne and isobutyne;
and/or
the cycloalkyl is C₃₋₈ cycloalkyl like cyclopropyl, cyclobutyl, cyclopentyl, cyclohexyl, cycloheptyl, or cyclooctyl; preferably is C₃₋₇ cycloalkyl like cyclopropyl, cyclobutyl, cyclopentyl, cyclohexyl, or cycloheptyl; more preferably from C₃₋₆ cycloalkyl like cyclopropyl, cyclobutyl, cyclopentyl or cyclohexyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein in R₈' as defined in any of the embodiments of the present invention,
the alkyl in alkylcycloalkyl is C₁₋₆ alkyl like methyl, ethyl, propyl, butyl,pentyl, hexyl, isopropyl, or 2-methylpropyl;
and/or
the C₁₋₆ alkyl is preferably selected from methyl, ethyl, propyl, butyl, pentyl, hexyl, isopropyl, or 2-methylpropyl; more preferably the C₁₋₆ alkyl is methyl;
and/or
the C₂₋₆ -alkenyl is preferably selected from ethylene, propylene, butylene, pentylene, hexylene, isopropylene and isobutylene;
and/or
the C₂₋₆ -alkynyl is preferably selected from ethyne, propyne, butyne, pentyne, hexyne, isopropyne and isobutyne;
and/or
the cycloalkyl is C₃₋₈ cycloalkyl like cyclopropyl, cyclobutyl, cyclopentyl, cyclohexyl, cycloheptyl, or cyclooctyl; preferably is C₃₋₇ cycloalkyl like cyclopropyl, cyclobutyl, cyclopentyl, cyclohexyl, or cycloheptyl; more preferably from C₃₋₆ cycloalkyl like cyclopropyl, cyclobutyl, cyclopentyl or cyclohexyl
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein in R₈-R₈' as defined in any of the embodiments of the present invention,
the heterocyclyl is a heterocyclic ring system of one or more saturated or unsaturated rings of which at least one ring contains one or more heteroatoms selected from the group consisting of nitrogen, oxygen and/or sulfur in the ring; preferably is a heterocyclic ring system of one or two saturated or unsaturated rings of which at least one ring contains one or more heteroatoms selected from the group consisting of nitrogen, oxygen and/or sulfur in the ring, more preferably is selected from oxazepan, pyrrolidine, imidazole, oxadiazole, tetrazole, azetidine, pyridine, pyrimidine, piperidine, piperazine, benzofuran, benzimidazole, indazole, benzothiazole, benzodiazole, thiazole, benzothiazole, Tetrahydropyran, morpholine, indoline, furan, triazole, isoxazole, pyrazole, thiophene, benzothiophene, pyrrole, pyrazine, pyrrolo[2,3b]pyridine, quinoline, isoquinoline, phthalazine, benzo-1,2,5-thiadiazole, indole, benzotriazole, benzoxazole oxopyrrolidine, pyrimidine, benzodioxolane, benzodioxane, carbazole, octahydro-ethanopyrrolo-pyridine, oxaspirodecane, oxadiazaspiroundecane, indoline-2-one and quinazoline;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein in R₉ as defined in any of the embodiments of the present invention,
the alkyl in alkylaryl, alkylheterocyclyl or alkylcycloalkyl is C₁₋₆ alkyl like methyl, ethyl, propyl, butyl,pentyl, hexyl, isopropyl, or 2-methylpropyl;
and/or
the C₁₋₆ alkyl is preferably selected from methyl, ethyl, propyl, butyl, pentyl, hexyl, isopropyl, or 2-methylpropyl;
and/or
the C₂₋₆ -alkenyl is preferably selected from ethylene, propylene, butylene, pentylene, hexylene, isopropylene and isobutylene;
and/or
the C₂₋₆ -alkynyl is preferably selected from ethyne, propyne, butyne, pentyne, hexyne, isopropyne and isobutyne;
and/or
the cycloalkyl is C₃₋₈ cycloalkyl like cyclopropyl, cyclobutyl, cyclopentyl, cyclohexyl, cycloheptyl, or cyclooctyl; preferably is C₃₋₇ cycloalkyl like cyclopropyl, cyclobutyl, cyclopentyl, cyclohexyl, or cycloheptyl; more preferably from C₃₋₆ cycloalkyl like cyclopropyl, cyclobutyl, cyclopentyl or cyclohexyl
and/or
the aryl is selected from phenyl, naphthyl, or anthracene; preferably is naphthyland phenyl; more preferably the aryl is phenyl;
and/or
the heterocyclyl is a heterocyclic ring system of one or more saturated or unsaturated rings of which at least one ring contains one or more heteroatoms selected from the group consisting of nitrogen, oxygen and/or sulfur in the ring; preferably is a heterocyclic ring system of one or two saturated or unsaturated rings of which at least one ring contains one or more heteroatoms selected from the group consisting of nitrogen, oxygen and/or sulfur in the ring, more preferably is selected from oxazepan, pyrrolidine, imidazole, oxadiazole, tetrazole, azetidine, pyridine, pyrimidine, piperidine, piperazine, benzofuran, benzimidazole, indazole, benzothiazole, benzodiazole, thiazole, benzothiazole, Tetrahydropyran, morpholine, indoline, furan, triazole, isoxazole, pyrazole, thiophene, benzothiophene, pyrrole, pyrazine, pyrrolo[2,3b]pyridine, quinoline, isoquinoline, phthalazine, benzo-1,2,5-thiadiazole, indole, benzotriazole, benzoxazole oxopyrrolidine, pyrimidine, benzodioxolane, benzodioxane, carbazole, octahydro-ethanopyrrolo-pyridine, oxaspirodecane, oxadiazaspiroundecane, indoline-2-one and quinazoline;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein in R₁₀, R₁₀', R₁₀" and R₁₀'" as defined in any of the embodiments of the present invention,
the C₁₋₆ alkyl is preferably selected from methyl, ethyl, propyl, butyl, pentyl, hexyl, isopropyl, or 2-methylpropyl;
and/or
the C₂₋₆ -alkenyl is preferably selected from ethylene, propylene, butylene, pentylene, hexylene, isopropylene and isobutylene;
and/or
the C₂₋₆ -alkynyl is preferably selected from ethyne, propyne, butyne, pentyne, hexyne, isopropyne and isobutyne;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein in R₁₀- R₁₀' and/or R₁₀"-R₁₀"' as defined in any of the embodiments of the present invention,
the cycloalkyl is C₃₋₈ cycloalkyl like cyclopropyl, cyclobutyl, cyclopentyl, cyclohexyl, cycloheptyl, or cyclooctyl; preferably is C₃₋₇ cycloalkyl like cyclopropyl, cyclobutyl, cyclopentyl, cyclohexyl, or cycloheptyl; more preferably from C₃₋₆ cycloalkyl like cyclopropyl, cyclobutyl, cyclopentyl or cyclohexyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein in R₁₁, R₁₁', R₁₁" and R₁₁'" as defined in any of the embodiments of the present invention,
the C₁₋₆ alkyl is preferably selected from methyl, ethyl, propyl, butyl, pentyl, hexyl, isopropyl, or 2-methylpropyl;
and/or
the C₂₋₆ -alkenyl is preferably selected from ethylene, propylene, butylene, pentylene, hexylene, isopropylene and isobutylene;
and/or
the C₂₋₆ -alkynyl is preferably selected from ethyne, propyne, butyne, pentyne, hexyne, isopropyne and isobutyne;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein in R₁₁ and R₁₁' and/or R₁₁" and R₁₁'" as defined in any of the embodiments of the present invention,
the cycloalkyl is C₃₋₈ cycloalkyl like cyclopropyl, cyclobutyl, cyclopentyl, cyclohexyl, cycloheptyl, or cyclooctyl; preferably is C₃₋₇ cycloalkyl like cyclopropyl, cyclobutyl, cyclopentyl, cyclohexyl, or cycloheptyl; more preferably from C₃₋₆ cycloalkyl like cyclopropyl, cyclobutyl, cyclopentyl or cyclohexyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein in R₁₃ and R₁₃' as defined in any of the embodiments of the present invention,
the C₁₋₆ alkyl is preferably selected from methyl, ethyl, propyl, butyl, pentyl, hexyl, isopropyl, or 2-methylpropyl;
and/or
the C₂₋₆ -alkenyl is preferably selected from ethylene, propylene, butylene, pentylene, hexylene, isopropylene and isobutylene;
and/or
the C₂₋₆ -alkynyl is preferably selected from ethyne, propyne, butyne, pentyne, hexyne, isopropyne and isobutyne;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein in R₁₄, R₁₄' and R₁₄" as defined in any of the embodiments of the present invention,
the C₁₋₆ alkyl is preferably selected from methyl, ethyl, propyl, butyl, pentyl, hexyl, isopropyl, or 2-methylpropyl;
and/or
the C₂₋₆ -alkenyl is preferably selected from ethylene, propylene, butylene, pentylene, hexylene, isopropylene and isobutylene;
and/or
the C₂₋₆ -alkynyl is preferably selected from ethyne, propyne, butyne, pentyne, hexyne, isopropyne and isobutyne;
and/or
the cycloalkyl is C₃₋₈ cycloalkyl like cyclopropyl, cyclobutyl, cyclopentyl, cyclohexyl, cycloheptyl, or cyclooctyl; preferably is C₃₋₇ cycloalkyl like cyclopropyl, cyclobutyl, cyclopentyl, cyclohexyl, or cycloheptyl; more preferably from C₃₋₆ cycloalkyl like cyclopropyl, cyclobutyl, cyclopentyl or cyclohexyl;
and/or
the aryl is selected from phenyl, naphthyl, or anthracene; preferably is naphthyland phenyl;
and/or
the heterocyclyl is a heterocyclic ring system of one or more saturated or unsaturated rings of which at least one ring contains one or more heteroatoms selected from the group consisting of nitrogen, oxygen and/or sulfur in the ring; preferably is a heterocyclic ring system of one or two saturated or unsaturated rings of which at least one ring contains one or more heteroatoms selected from the group consisting of nitrogen, oxygen and/or sulfur in the ring, more preferably is selected from oxazepan, pyrrolidine, imidazole, oxadiazole, tetrazole, azetidine, pyridine, pyrimidine, piperidine, piperazine, benzofuran, benzimidazole, indazole, benzothiazole, benzodiazole, thiazole, benzothiazole, Tetrahydropyran, morpholine, indoline, furan, triazole, isoxazole, pyrazole, thiophene, benzothiophene, pyrrole, pyrazine, pyrrolo[2,3b]pyridine, quinoline, isoquinoline, phthalazine, benzo-1,2,5-thiadiazole, indole, benzotriazole, benzoxazole oxopyrrolidine, pyrimidine, benzodioxolane, benzodioxane, carbazole, octahydro-ethanopyrrolo-pyridine, oxaspirodecane, oxadiazaspiroundecane, indoline-2-one and quinazoline;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein in R₁₅ and R₁₅' as defined in any of the embodiments of the present invention,
the C₁-₆ alkyl is preferably selected from methyl, ethyl, propyl, butyl, pentyl, hexyl, isopropyl, or 2-methylpropyl;
and/or
the C₂₋₆ -alkenyl is preferably selected from ethylene, propylene, butylene, pentylene, hexylene, isopropylene and isobutylene;
and/or
the C₂₋₆ -alkynyl is preferably selected from ethyne, propyne, butyne, pentyne, hexyne, isopropyne and isobutyne;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein in R₁₆ and R₁₆' as defined in any of the embodiments of the present invention,
the alkyl in haloalkyl or haloalkoxy is C₁₋₆ alkyl like methyl, ethyl, propyl, butyl,pentyl, hexyl, isopropyl, or 2-methylpropyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein in R₂₁, R₂₁' and R₂₁" as defined in any of the embodiments of the present invention,
the alkyl in alkylcycloalkyl is C₁₋₆ alkyl like methyl, ethyl, propyl, butyl,pentyl, hexyl, isopropyl, or 2-methylpropyl;
and/or
the C₁₋₆ alkyl is preferably selected from methyl, ethyl, propyl, butyl, pentyl, hexyl, isopropyl, or 2-methylpropyl; more preferably the C₁₋₆ alkyl is methyl;
and/or
the C₂₋₆ -alkenyl is preferably selected from ethylene, propylene, butylene, pentylene, hexylene, isopropylene and isobutylene;
and/or
the C₂₋₆ -alkynyl is preferably selected from ethyne, propyne, butyne, pentyne, hexyne, isopropyne and isobutyne;
and/or
the cycloalkyl is C₃₋₈ cycloalkyl like cyclopropyl, cyclobutyl, cyclopentyl, cyclohexyl, cycloheptyl, or cyclooctyl; preferably is C₃₋₇ cycloalkyl like cyclopropyl, cyclobutyl, cyclopentyl, cyclohexyl, or cycloheptyl; more preferably from C₃₋₆ cycloalkyl like cyclopropyl, cyclobutyl, cyclopentyl or cyclohexyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein in R₃₁, R₃₁' and R₃₁" as defined in any of the embodiments of the present invention,
the C₁₋₆ alkyl is preferably selected from methyl, ethyl, propyl, butyl, pentyl, hexyl, isopropyl, or 2-methylpropyl;
and/or
the C₂₋₆ -alkenyl is preferably selected from ethylene, propylene, butylene, pentylene, hexylene, isopropylene and isobutylene;
and/or
the C₂₋₆ -alkynyl is preferably selected from ethyne, propyne, butyne, pentyne, hexyne, isopropyne and isobutyne;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein in R₇₁ and R₇₁' as defined in any of the embodiments of the present invention,
the C₁₋₆ alkyl is preferably selected from methyl, ethyl, propyl, butyl, pentyl, hexyl, isopropyl, or 2-methylpropyl;
and/or
the C₂₋₆ -alkenyl is preferably selected from ethylene, propylene, butylene, pentylene, hexylene, isopropylene and isobutylene;
and/or
the C₂₋₆ -alkynyl is preferably selected from ethyne, propyne, butyne, pentyne, hexyne, isopropyne and isobutyne;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein in R₈₁, R₈₁' and R₈₁" as defined in any of the embodiments of the present invention,
the C₁₋₆ alkyl is preferably selected from methyl, ethyl, propyl, butyl, pentyl, hexyl, isopropyl, or 2-methylpropyl;
and/or
the C₂₋₆ -alkenyl is preferably selected from ethylene, propylene, butylene, pentylene, hexylene, isopropylene and isobutylene;
and/or
the C₂₋₆ -alkynyl is preferably selected from ethyne, propyne, butyne, pentyne, hexyne, isopropyne and isobutyne;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein in R₈₂, R₈₂' and R₈₂" as defined in any of the embodiments of the present invention,
the C₁₋₆ alkyl is preferably selected from methyl, ethyl, propyl, butyl, pentyl, hexyl, isopropyl, or 2-methylpropyl;
and/or
the C₂₋₆ -alkenyl is preferably selected from ethylene, propylene, butylene, pentylene, hexylene, isopropylene and isobutylene;
and/or
the C₂₋₆ -alkynyl is preferably selected from ethyne, propyne, butyne, pentyne, hexyne, isopropyne and isobutyne;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein in R₈₃, R₈₃' and R₈₃" as defined in any of the embodiments of the present invention,
the C₁₋₆ alkyl is preferably selected from methyl, ethyl, propyl, butyl, pentyl, hexyl, isopropyl, or 2-methylpropyl;
and/or
the C₂₋₆ -alkenyl is preferably selected from ethylene, propylene, butylene, pentylene, hexylene, isopropylene and isobutylene;
and/or
the C₂₋₆ -alkynyl is preferably selected from ethyne, propyne, butyne, pentyne, hexyne, isopropyne and isobutyne;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein in R₉₁, R₉₁' and R₉₁" as defined in any of the embodiments of the present invention,
the C₁₋₆ alkyl is preferably selected from methyl, ethyl, propyl, butyl, pentyl, hexyl, isopropyl, or 2-methylpropyl;
and/or
the C₂₋₆ -alkenyl is preferably selected from ethylene, propylene, butylene, pentylene, hexylene, isopropylene and isobutylene;
and/or
the C₂₋₆ -alkynyl is preferably selected from ethyne, propyne, butyne, pentyne, hexyne, isopropyne and isobutyne;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein
X is selected from the group consisting of a bond or -[C(RₐR_{b})]ₚ-; preferably X is a bond or -CH₂-;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein
Rₐ is selected from the group consisting of hydrogen, substituted or unsubstituted C₁₋₆ alkyl, substituted or unsubstituted C₂₋₆ alkenyl and substituted or unsubstituted C₂₋₆ alkynyl; preferably Rₐ is hydrogen;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein
R_{b} is selected from the group consisting of hydrogen, substituted or unsubstituted C₁₋₆ alkyl, substituted or unsubstituted C₂₋₆ alkenyl and substituted or unsubstituted C₂₋₆ alkynyl; preferably R_{b} is hydrogen;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein
p is 0, 1, 2, 3, 4 or 5; preferably p is 0 or 1;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein
w₁ is nitrogen or carbon;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein
w₂ is nitrogen or - carbon;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein
w₃ is nitrogen or carbon;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein
r is 0, 1 or 2; preferably r is 1;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein
v is 0, 1 or 2; preferably v is 1;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein
R₁ is selected from the group consisting of hydrogen, substituted or unsubstituted C₁₋₆ alkyl, substituted or unsubstituted C₂₋₆ alkenyl and substituted or unsubstituted C₂₋₆ alkynyl; preferably R₁ is hydrogen or substituted or unsubstituted ethyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein
R₂ is selected from the group consisting of -R₂₁, -OR₂₁, -NO₂, halogen, -NR₂₁R₂₁',-NR₂₁C(O)R₂₁', -NR₂₁S(O)₂R₂₁', -S(O)₂NR₂₁R₂₁', - NR₂₁C(O)NR₂₁'R₂₁", -SR₂₁ , -S(O)R₂₁, -S(O)₂R₂₁, -CN, haloalkyl, haloalkoxy, -C(O)OR₂₁, -C(O)NR₂₁R₂₁', -OCH₂CH₂OR₂₁,-NR₂₁S(O)₂NR₂₁'R₂₁" and -C(CH₃)₂OR₂₁; preferably R₂ is -R₂₁ or -OR₂₁; more preferably R₂ is hydrogen or -OCH₃;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein
R₃ is selected from the group consisting of -R₃₁, -OR₃₁, -NO₂, halogen, -NR₃₁R₃₁',-NR₃₁C(O)R₃₁', -NR₃₁S(O)₂R₃₁', -S(O)₂NR₃₁R₃₁', - NR₃₁C(O)NR₃₁'R₃₁", -SR₃₁ , -S(O)R₃₁, -S(O)₂R₃₁, -CN, haloalkyl, haloalkoxy, -C(O)OR₃₁, -C(O)NR₃₁R₃₁', -OCH₂CH₂OR₃₁,-NR₃₁S(O)2NR₃₁'R₃₁" and -C(CH₃)₂OR₃₁, preferably R₃ is -R₃₁; more preferably R₃ is hydrogen;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein
R₄ is 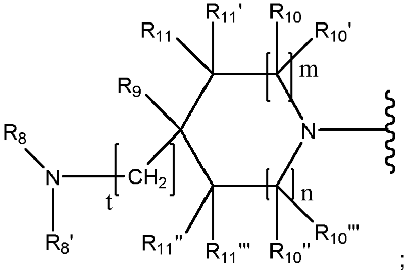 preferably R₄ is 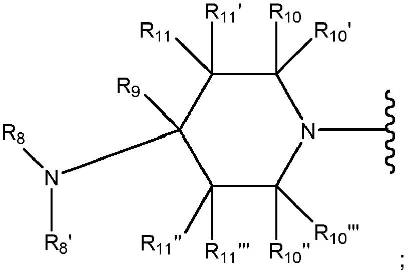 more preferably R₄ is 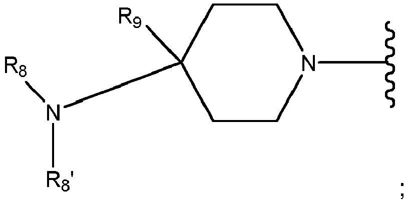 even more preferably R₄ is 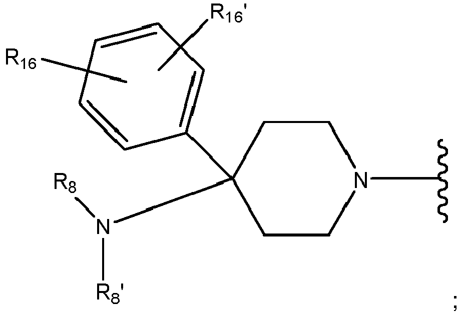 even more preferably R₄ is 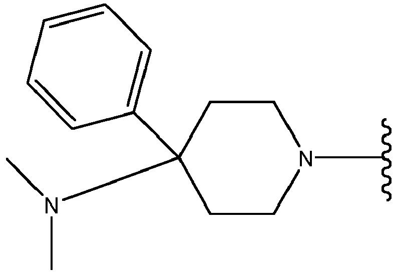 or 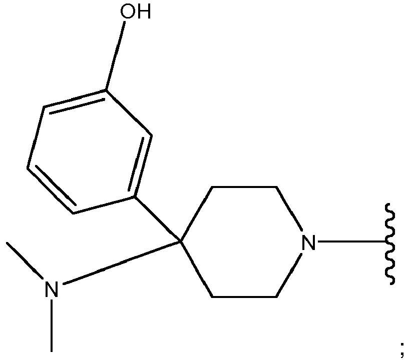 optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein
R₅, R₅', R₅" and R₅'" are independently selected from the group consisting of hydrogen, halogen, substituted or unsubstituted C₁₋₆ alkyl, substituted or unsubstituted C₂₋₆ alkenyl and substituted or unsubstituted C₂₋₆ alkynyl; R₅, R₅', R₅" and R₅'" are independently selected from the group consisting of hydrogen, halogen and substituted or unsubstituted C₁₋₆ alkyl; more preferably, R₅, R₅', R₅" and R₅'" are all hydrogen;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein
R₆, R₆', R₆" and R₆'" are independently selected from the group consisting of hydrogen, substituted or unsubstituted C₁₋₆ alkyl, substituted or unsubstituted C₂₋₆ alkenyl and substituted or unsubstituted C₂₋₆ alkynyl; preferably R₆, R₆', R₆" and R₆'" are independently selected from the group consisting of hydrogen and substituted or unsubstituted C₁₋₆ alkyl; more preferably R₆, R₆', R₆" and R₆'" are hydrogen or substituted or unsubstituted methyl; even more preferably R₆ and R₆" are substituted or unsubstituted methyl and R₆' and R₆'" are hydrogen;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein
R₇ is selected from the group consisting of hydrogen, substituted or unsubstituted C₁₋₆ alkyl, substituted or unsubstituted C₂₋₆ alkenyl and substituted or unsubstituted C₂₋₆ alkynyl; preferably R₇ is selected from the group consisting of hydrogen and substituted or unsubstituted C₁₋₆ alkyl; more preferably R₇ is hydrogen;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein
R₈ is selected from the group consisting of substituted or unsubstituted C₁₋₆ alkyl, substituted or unsubstituted C₂₋₆ alkenyl, substituted or unsubstituted C₂₋₆ alkynyl, substituted or unsubstituted cycloalkyl and substituted or unsubstituted alkylcycloalkyl; preferably R₈ is selected from the group consisting of substituted or unsubstituted C₁₋₆ alkyl, substituted or unsubstituted cycloalkyl and substituted or unsubstituted alkylcycloalkyl; more preferably R₈ is substituted or unsubstituted methyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein
R₈' is selected from the group consisting of hydrogen, substituted or unsubstituted C₁₋₆ alkyl, substituted or unsubstituted C₂₋₆ alkenyl, substituted or unsubstituted C₂₋₆ alkynyl, substituted or unsubstituted cycloalkyl and substituted or unsubstituted alkylcycloalkyl; preferably, R₈' is selected from the group consisting of hydrogen, substituted or unsubstituted C₁₋₆ alkyl, substituted or unsubstituted cycloalkyl and substituted or unsubstituted alkylcycloalkyl; more preferably R₈' is substituted or unsubstituted methyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein
R₉ is selected from the group consisting of substituted or unsubstituted C₁₋₆ alkyl, substituted or unsubstituted C₂₋₆ alkenyl, substituted or unsubstituted C₂₋₆ alkynyl, substituted or unsubstituted cycloalkyl, substituted or unsubstituted aryl, substituted or unsubstituted heterocyclyl, substituted or unsubstituted alkylcycloalkyl, substituted or unsubstituted alkylaryl and substituted or unsubstituted alkylheterocyclyl; preferably R₉ is selected from the group consisting of substituted or unsubstituted C₁₋₆ alkyl, substituted or unsubstituted cycloalkyl, substituted or unsubstituted aryl, substituted or unsubstituted heterocyclyl, substituted or unsubstituted alkylcycloalkyl, substituted or unsubstituted alkylaryl and substituted or unsubstituted alkylheterocyclyl; more preferably R₉ is substituted or unsubstituted phenyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein
R₁₀, R₁₀', R₁₀" and R₁₀'" are independently selected from the group consisting of hydrogen, halogen, substituted or unsubstituted C₁₋₆ alkyl, substituted or unsubstituted C₂₋₆ alkenyl and substituted or unsubstituted C₂₋₆ alkynyl; preferably R₁₀, R₁₀', R₁₀" and R₁₀'" are independently selected from the group consisting of hydrogen, halogen and substituted or unsubstituted C₁₋₆ alkyl; more preferably R₁₀, R₁₀', R₁₀" and R₁₀'" are all hydrogen;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein
R₁₁, R₁₁', R₁₁" and R₁₁'" are independently selected from the group consisting of hydrogen, halogen, substituted or unsubstituted C₁₋₆ alkyl, substituted or unsubstituted C₂₋₆ alkenyl and substituted or unsubstituted C₂₋₆ alkynyl; preferably R₁₁, R₁₁', R₁₁" and R₁₁'" are independently selected from the group consisting of hydrogen, halogen and substituted or unsubstituted C₁₋₆ alkyl; R₁₁, R₁₁', R₁₁" and R₁₁'" are all hydrogen;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein
R₁₆ and R₁₆' are independently selected from halogen, -R₉₁, -OR₉₁, -NO₂, -NR₉₁R₉₁',-NR₉₁C(O)R₉₁', -NR₉₁S(O)₂R₉₁', -S(O)₂NR₉₁R₉₁', -NR₉₁C(O)NR₉₁'R₉₁", -SR₉₁, -S(O)R₉₁, -S(O)₂R₉₁, -CN, haloalkyl, haloalkoxy, -C(O)OR₉₁, -OC(O)R₉₁, -C(O)NR₉₁R₉₁',-OCH₂CH₂OR₉₁, -NR₉₁S(O)₂NR₉₁'R₉₁" and -C(CH₃)₂OR₉₁; preferably R₁₆ and R₁₆' are independently selected from hydrogen and -OR₉₁; more preferably R₁₆ and R₁₆' are independently selected from hydrogen and -OH;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a further embodiment the compound according to the invention of general Formula (I) is a compound wherein
R₂₁, R₂₁' and R₂₁" are independently selected from the group consisting of hydrogen, substituted or unsubstituted C₁₋₆ alkyl, substituted or unsubstituted C₂₋₆ alkenyl, substituted or unsubstituted C₂₋₆ alkynyl and substituted or unsubstituted alkylcycloalkyl; preferably R₂₁, R₂₁' and R₂₁" are independently selected from the group consisting of hydrogen, substituted or unsubstituted C₁₋₆ alkyl and substituted or unsubstituted alkylcycloalkyl; more preferably R₂₁ is selected from the group consisting of hydrogen and substituted or unsubstituted methyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein
R₃₁, R₃₁' and R₃₁" are independently selected from the group consisting of hydrogen, substituted or unsubstituted C₁₋₆ alkyl, substituted or unsubstituted C₂₋₆ alkenyl and substituted or unsubstituted C₂₋₆ alkynyl; preferably R₃₁, R₃₁' and R₃₁" are independently selected from the group consisting of hydrogen and substituted or unsubstituted C₁₋₆ alkyl; more preferably R₃₁ is hydrogen;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein
R₉₁, R₉₁' and R₉₁" are independently selected from the group consisting of the group consisting of hydrogen, substituted or unsubstituted C₁₋₆ alkyl, substituted or unsubstituted C₂₋₆ alkenyl, and substituted or unsubstituted C₂₋₆ alkynyl; preferably R₉₁, R₉₁' and R₉₁" are independently selected from the group consisting of the group consisting of hydrogen and substituted or unsubstituted C₁₋₆ alkyl; more preferably R₉₁ is hydrogen;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to general Formula (I) the compound is a compound, wherein
X is a bond or -CH₂-;
and/or
Rₐ is hydrogen;
and/or
R_{b} is hydrogen;
and/or
p is 0 or 1;
and/or
w₁ is nitrogen or carbon;
and/or
w₂ is nitrogen or - carbon;
and/or
w₃ is nitrogen or carbon;
and/or
r is 1;
and/or
v is 1;
and/or
R₁ is hydrogen or substituted or unsubstituted ethyl;
and/or
R₂ is hydrogen or -OCH₃;
and/or
R₃ is hydrogen;
and/or
R₄ is 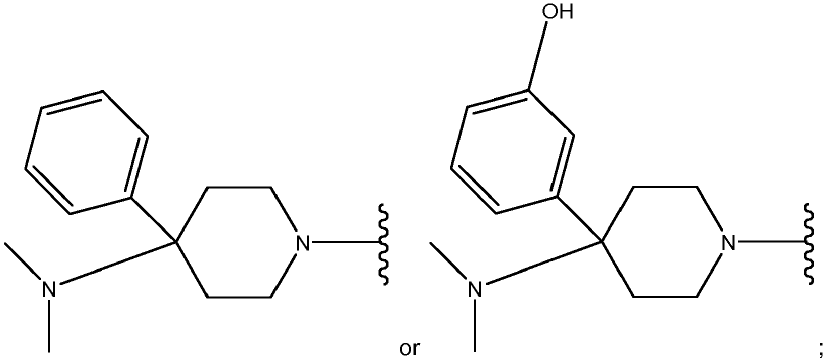 and/or
R₅, R₅', R₅" and R₅"' are all hydrogen;
and/or
R₆, R₆', R₆" and R₆"' are hydrogen or substituted or unsubstituted methyl;
and/or
R₇ is hydrogen;
and/or
R₈ is substituted or unsubstituted methyl;
and/or
R₈' is substituted or unsubstituted methyl;
and/or
R₉ is substituted or unsubstituted phenyl;
and/or
R₁₀, R₁₀', R₁₀" and R₁₀'" are all hydrogen;
and/or
R₁₁, R₁₁', R₁₁" and R₁₁'" are all hydrogen;
and/or
R₁₆ and R₁₆' are independently selected from hydrogen and -OH;
and/or
R₂₁ is hydrogen or substituted or unsubstituted methyl;
and/or
R₃₁ is hydrogen;
and/or
R₉₁ is hydrogen;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.
In a preferred embodiment
X is a bond or -CH₂-.
In a preferred embodiment
Rₐ is hydrogen.
In a preferred embodiment
R_{b} is hydrogen.
In a preferred embodiment
p is 0 or 1.
In a preferred embodiment
w₁ is nitrogen or carbon.
In a preferred embodiment
w₂ is nitrogen or - carbon.

In a preferred embodiment
w₃ is nitrogen or carbon.

In a preferred embodiment
r is 1.

In a preferred embodiment
v is 1.

In a preferred embodiment
R₁ is hydrogen or substituted or unsubstituted ethyl.

In a preferred embodiment
R₂ is hydrogen or -OCH₃.

In a preferred embodiment
R₃ is hydrogen.

In a preferred embodiment
R₄ is

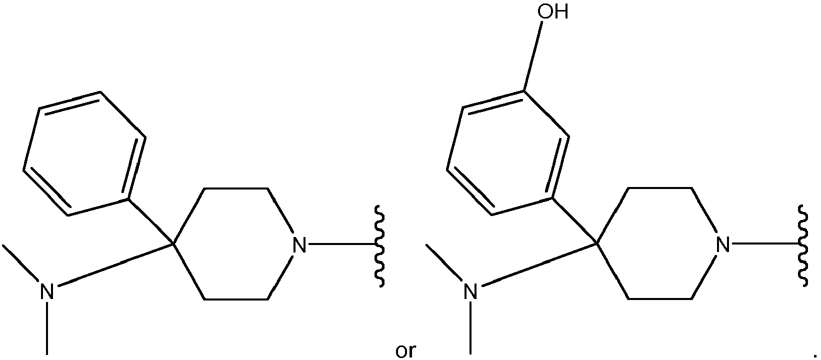

In a preferred embodiment
R₅, R₅', R₅" and R₅'" are all hydrogen.

In a preferred embodiment
R₆, R₆', R₆" and R₆"' are hydrogen or substituted or unsubstituted methyl.

In a preferred embodiment
R₆ and R₆" are substituted or unsubstituted methyl.

In a preferred embodiment
R₆' and R₆"' are hydrogen.

In a preferred embodiment
R₆ and R₆" are substituted or unsubstituted methyl, while R₆' and R₆"' are hydrogen.

In a preferred embodiment
R₇ is hydrogen.

In a preferred embodiment
R₈ is substituted or unsubstituted methyl.

In a preferred embodiment
R₈' is substituted or unsubstituted methyl.

In a preferred embodiment
R₉ is substituted or unsubstituted phenyl.

In a preferred embodiment
R₁₀, R₁₀', R₁₀" and R₁₀'" are all hydrogen.

In a preferred embodiment
R₁₁, R₁₁', R₁₁" and R₁₁'" are all hydrogen.

In a preferred embodiment
R₁₆ and R₁₆' are independently selected from hydrogen and -OH.

In a preferred embodiment
R₁₆ is selected from hydrogen and -OH.

In a preferred embodiment
R₁₆' is hydrogen.

In a preferred embodiment
R₁₆ is selected from hydrogen and -OH, while R₁₆' is hydrogen.

In a preferred embodiment
R₂₁ is hydrogen or substituted or unsubstituted methyl.

In a preferred embodiment
R₃₁ is hydrogen.

In a preferred embodiment
R₉₁ is hydrogen.

In an embodiment of the compound according to the invention of general Formula (I),
the haloalkyl is -CF₃ ;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another embodiment of the compound according to the invention of general Formula (I),
the haloalkoxy is -OCF₃;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a preferred further embodiment, the compounds of the general Formula (I) are selected from

| **EX** | **CHEMICALNAME** |
|---|---|
| **1** | 3-(4-(Dimethylamino)-1-(6-(1-((3*S*,5*R*)-3,5-dimethylpiperazin-1-yl)butyl)naphthalen-2-yl)piperidin-4-yl)phenol |
| **2** | 3-(4-(Dimethylamino)-1-(3-(1-((3*S*,5*R*)-3,5-dimethylpiperazin-1-yl)butyl)quinolin-7-yl)piperidin-4-yl)phenol |
| **3** | 3-(4-(Dimethylamino)-1-(2-(1-((3*S*,5*R*)-3,5-dimethylpiperazin-1-yl)butyl)quinolin-6-yl)piperidin-4-yl)phenol |
| **4** | 3-(4-(Dimethylamino)-1-(3-((*R*)-1-((3*S*,5*R*)-3,5-dimethylpiperazin-1-yl)butyl)quinolin-7-yl)piperidin-4-yl)phenol |
| **5** | 3-(4-(Dimethylamino)-1-(3-((*S*)-1-((3*S*,5*R*)-3,5-dimethylpiperazin-1-yl)butyl)quinolin-7-yl)piperidin-4-yl)phenol |
| **6** | 3-(4-(Dimethylamino)-1-((3-(1-((3*S*,5*R*)-3,5-dimethylpiperazin-1-yl)butyl)quinolin-7-yl)methyl)piperidin-4-yl)phenol |
| **7** | 3-(4-(Dimethylamino)-1-((2-(1-((3*S*,5*R*)-3,5-dimethylpiperazin-1-yl)butyl)quinolin-6-yl)methyl)piperidin-4-yl)phenol |
| **8** | 3-(4-(Dimethylamino)-1-((3-((*R*)-1-((3*S*,5*R*)-3,5-dimethylpiperazin-1-yl)butyl)quinolin-7-yl)methyl)piperidin-4-yl)phenol |
| **9** | 3-(4-(Dimethylamino)-1-((3-((*S*)-1-((3*S*,5*R*)-3,5-dimethylpiperazin-1-yl)butyl)quinolin-7-yl)methyl)piperidin-4-yl)phenol |
| **10** | 3-(4-(Dimethylamino)-1-((2-((*R*)-1-((3*S*,5*R*)-3,5-dimethylpiperazin-1-yl)butyl)quinolin-6-yl)methyl)piperidin-4-yl)phenol |
| **11** | 3-(4-(Dimethylamino)-1-((2-((*S*)-1-((3*S*,5*R*)-3,5-dimethylpiperazin-1-yl)butyl)quinolin-6-yl)methyl)piperidin-4-yl)phenol |
| **12** | 1-((3-((*R*)-1-((3*S*,5*R*)-3,5-Dimethylpiperazin-1-yl)butyl)quinolin-7-yl)methyl)-*N*,*N*-dimethyl-4-phenylpiperidin-4-amine |
| **13** | 1-((3-((*S*)-1-((3*S*,5*R*)-3,5-Dimethylpiperazin-1-yl)butyl)quinolin-7-yl)methyl)-*N*,*N*-dimethyl-4-phenylpiperidin-4-amine |
| **14** | 3-(4-(Dimethylamino)-1-(3-(1-((3*S*,5*R*)-3,5-dimethylpiperazin-1-yl)butyl)quinolin-6-yl)piperidin-4-yl)phenol |
| **15** | 3-(4-(Dimethylamino)-1-((3-(1-((3*S*,5*R*)-3,5-dimethylpiperazin-1-yl)butyl)quinolin-6-yl)methyl)piperidin-4-yl)phenol |
| **16** | 3-(4-(Dimethylamino)-1-((3-((*R*)-1-((3*S*,5*R*)-3,5-dimethylpiperazin-1-yl)butyl)quinolin-6-yl)methyl)piperidin-4-yl)phenol |
| **17** | 3-(4-(Dimethylamino)-1-((3-((*S*)-1-((3*S*,5*R*)-3,5-dimethylpiperazin-1-yl)butyl)quinolin-6-yl)methyl)piperidin-4-yl)phenol |
| **18** | 1-((3-((*R*)-1-((3*S*,5*R*)-3,5-Dimethylpiperazin-1-yl)butyl)quinolin-6-yl)methyl)-*N*,*N*-dimethyl-4-phenylpiperidin-4-amine |
| **19** | 1-((3-((*S*)-1-((3*S*,5*R*)-3,5-Dimethylpiperazin-1-yl)butyl)quinolin-6-yl)methyl)-*N*,*N*-dimethyl-4-phenylpiperidin-4-amine |
| **20** | 3-(4-(Dimethylamino)-1-(3-(1-((3*S*,5*R*)-3,5-dimethylpiperazin-1-yl)butyl)-2-ethylquinolin-7-yl)piperidin-4-yl)phenol |
| **21** | 3-(4-(Dimethylamino)-1-((3-(1-((3*S*,5*R*)-3,5-dimethylpiperazin-1-yl)butyl)-2-ethylquinolin-7-yl)methyl)piperidin-4-yl)phenol |
| **22** | 3-(4-(Dimethylamino)-1-((3-(1-((3*S*,5*R*)-3,5-dimethylpiperazin-1-yl)butyl)-2-ethylquinolin-6-yl)methyl)piperidin-4-yl)phenol |
| **23** | 3-(4-(Dimethylamino)-1-((3-((*R*)-1-((3*S*,5*R*)-3,5-dimethylpiperazin-1-yl)butyl)-2-ethylquinolin-7-yl)methyl)piperidin-4-yl)phenol |
| **24** | 3-(4-(Dimethylamino)-1-((3-((*S*)-1-((3*S*,5*R*)-3,5-dimethylpiperazin-1-yl)butyl)-2-ethylquinolin-7-yl)methyl)piperidin-4-yl)phenol |
| **25** | 3-(4-(Dimethylamino)-1-(2-(1-((3*S*,5*R*)-3,5-dimethylpiperazin-1-yl)butyl)quinazolin-6-yl)piperidin-4-yl)phenol |
| **26** | 3-(4-(Dimethylamino)-1-(2-((*R*)-1-((3*S*,5*R*)-3,5-dimethylpiperazin-1-yl)butyl)quinazolin-6-yl)piperidin-4-yl)phenol |
| **27** | 3-(4-(Dimethylamino)-1-(2-((*S*)-1-((3*S*,5*R*)-3,5-dimethylpiperazin-1-yl)butyl)quinazolin-6-yl)piperidin-4-yl)phenol |
| **28** | 3-(4-(Dimethylamino)-1-((2-(1-((3*S*,5*R*)-3,5-dimethylpiperazin-1-yl)butyl)quinazolin-6-yl)methyl)piperidin-4-yl)phenol |
| **29** | 3-(4-(Dimethylamino)-1-((2-((*R*)-1-((3*S*,5*R*)-3,5-dimethylpiperazin-1-yl)butyl)quinazolin-6-yl)methyl)piperidin-4-yl)phenol |
| **30** | 3-(4-(Dimethylamino)-1-((2-((*S*)-1-((3*S*,5*R*)-3,5-dimethylpiperazin-1-yl)butyl)quinazolin-6-yl)methyl)piperidin-4-yl)phenol |
| **31** | 1-(2-(1-((3*S*,5*R*)-3,5-Dimethylpiperazin-1-yl)butyl)-4-methoxyquinolin-6-yl)-*N*,*N*-dimethyl-4-phenylpiperidin-4-amine |
| **32** | 3-(4-(Dimethylamino)-1-(2-(1-((3*S*,5*R*)-3,5-dimethylpiperazin-1-yl)butyl)quinoxalin-6-yl)piperidin-4-yl)phenol |
| **33** | 3-(4-(Dimethylamino)-1-(3-(1-((3*S*,5*R*)-3,5-dimethylpiperazin-1-yl)butyl)quinoxalin-6-yl)piperidin-4-yl)phenol |
| **34** | 3-(4-(Dimethylamino)-1-(3-((*R*)-1-((3*S*,5*R*)-3,5-dimethylpiperazin-1-yl)butyl)quinoxalin-6-yl)piperidin-4-yl)phenol |
| **35** | 3-(4-(Dimethylamino)-1-(3-((*S*)-1-((3*S*,5*R*)-3,5-dimethylpiperazin-1-yl)butyl)quinoxalin-6-yl)piperidin-4-yl)phenol |
| **36** | 3-(4-(Dimethylamino)-1-((2-(1-((3*S*,5*R*)-3,5-dimethylpiperazin-1-yl)butyl)quinoxalin-6-yl)methyl)piperidin-4-yl)phenol |
| **37** | 3-(4-(Dimethylamino)-1-((3-(1-((3*S*,5*R*)-3,5-dimethylpiperazin-1-yl)butyl)quinoxalin-6-yl)methyl)piperidin-4-yl)phenol |
| **38** | 3-(4-(Dimethylamino)-1-((3-((*R*)-1-((3*S*,5*R*)-3,5-dimethylpiperazin-1-yl)butyl)quinoxalin-6-yl)methyl)piperidin-4-yl)phenol |
| **39** | 3-(4-(Dimethylamino)-1-((3-((*S*)-1-((3*S*,5*R*)-3,5-dimethylpiperazin-1-yl)butyl)quinoxalin-6-yl)methyl)piperidin-4-yl)phenol |
| **40** | 3-(4-(Dimethylamino)-1-((2-(1-((3*S*,5*R*)-3,5-dimethylpiperazin-1-yl)butyl)-3-ethylquinoxalin-6-yl)methyl)piperidin-4-yl)phenol |
| **41** | 3-(4-(Dimethylamino)-1-((2-((*R*)-1-((3*S*,5*R*)-3,5-dimethylpiperazin-1-yl)butyl)-3-ethylquinoxalin-6-yl)methyl)piperidin-4-yl)phenol |
| **42** | 3-(4-(Dimethylamino)-1-((2-((*S*)-1-((3*S*,5*R*)-3,5-dimethylpiperazin-1-yl)butyl)-3-ethylquinoxalin-6-yl)methyl)piperidin-4-yl)phenol |

optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a very preferred embodiment, the compounds are selected which act as dual ligands of the α2δ subunit, particularly the α2δ-1 subunit, of the voltage-gated calcium channel and the µ-opioid receptor and especially compounds which have a binding expressed as Kᵢ responding to the following scales:
Kᵢ(µ) is preferably < 1000 nM, more preferably < 500 nM, even more preferably < 100 nM.

Ki(α2δ1) is preferably < 10000 nM, more preferably < 5000 nM, even more preferably < 500 nM.

In the following the phrase "compound of the invention" is used. This is to be understood as any compound according to the invention as described above according to general Formula (I), (I'), (I²'), (I³'), (I⁴'), (I⁵'), (I⁶'), (I⁷'), (I^{a}) or (IZ).

The compounds of the invention represented by the above described Formula (I) may include enantiomers depending on the presence of chiral centres or isomers depending on the presence of multiple bonds (e.g. Z, E). The single isomers, enantiomers or diastereoisomers and mixtures thereof fall within the scope of the present invention. For the sake of clarity the expression "a compound according to Formula (I), wherein e.g. R₁, R₂, R₃, R₄, R₅, R₅', R₅", R₅"', R₆, R₆', R₆", R₆"', R₇, X, w₁, w₂, w₃, r and v are as defined below in the detailed description" would (just like the expression "a compound of Formula (I) as defined in any one of claims e.g. 1 to 8" found in the claims) refer to "a compound according to Formula (I)", wherein the definitions of the respective substituents R₁ etc. (also from the cited claims) are applied. In addition, this would also mean, though (especially in regards to the claims) that also one or more disclaimers or provisos defined in the description (or used in any of the cited claims like e.g. claim 1) would be applicable to define the respective compound. Thus, a disclaimer or a proviso found in e.g. claim 1 would be also used to define the compound "of Formula (I) as defined in any one of the corresponding related claims e.g. 1 to 8".

In general the processes are described below in the experimental part. The starting materials are commercially available or can be prepared by conventional methods.

A preferred embodiment of the invention is a process for the production of a compound according to Formula (I), wherein, if not defined otherwise, R₁, R₂, R₃, R₄, R₅, R₅', R₅", R₅"', R₆, R₆', R₆", R₆"', R₇, X, w₁, w₂, w₃, r and v have the meanings defined in the description. LG represents a leaving group (such as chloro, bromo, iodo, mesylate, tosylate, nosylate or triflate).

In a particular embodiment there is a process for the production of a compound according to Formula (I), said process comprises reacting a compound of formula VII wherein T is -X-R₄ 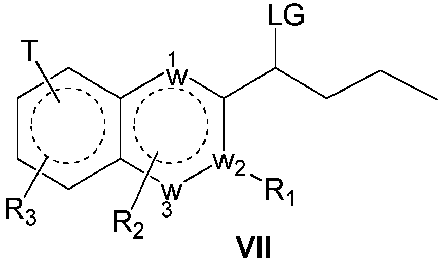 with a suitable amine of formula XI,

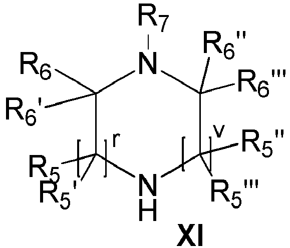

in a suitable solvent, such as acetonitrile, dichloromethane or dimethylformamide, in the presence of a base such as triethylamine, K₂CO₃ or *N*,*N*-diisopropylethylamine, at a suitable temperature comprised between room temperature and the reflux temperature, preferably heating, wherein R₁, R₂, R₃, R₄, R₅, R₅', R₅", R₅"', R₆, R₆', R₆", R₆"', R₇, X, w₁, w₂, w₃, r and v have the meanings as defined in the description.

In a particular embodiment there is a process for the production of a compound according to Formula (I), wherein w₁ is nitrogen and w₂ and w₃ are carbon, said process comprises reacting a compound of formula XIV 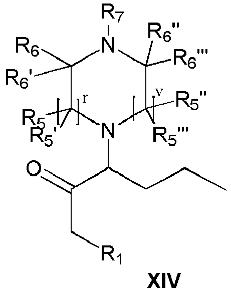 with an aminoaldehyde of formula XIII,

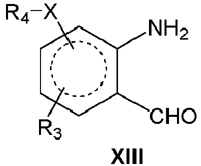

in the presence of a base, such as potassium hydroxide, in a suitable solvent, such as ethanol, at a suitable temperature, preferably heating, wherein R₁, R₂, R₃, R₄, R₅, R₅', R₅", R₅"', R₆, R₆', R₆", R₆"', R₇, X, w₁, w₂, w₃, r and v have the meanings as defined in the description.

In a particular embodiment there is a process for the production of a compound according to Formula (I) wherein X is a bond, said process comprises reacting a compound of formula XII wherein T is halogen, 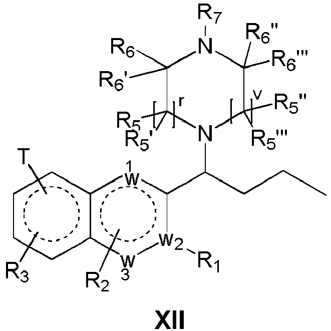 with a compound of formula XVI 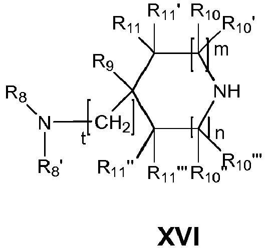 under Buchwald-Hartwig arylation conditions, using a Pd catalyst such as tris(dibenzylideneacetone)dipalladium(0) or palladium acetate, and a suitable ligand, preferably a phosphine ligand such as DavePhos, XPhos or BINAP, using a suitable base such as sodium *tert*-butoxide or cesium carbonate, in a suitable solvent such as toluene or 1,4-dioxane, at a suitable temperature, preferably heating, wherein R₁, R₂, R₃, R₄, R₅, R₅', R₅", R₅"', R₆, R₆', R₆", R₆"', R₇, R₈, R₈', R₉, R₁₀, R₁₀', R₁₀", R₁₀"', R₁₁, R₁₁', R₁₁", R₁₁"', X, w₁, w₂, w₃, n, m, t, r and v have the meanings as defined in the description.

In a particular embodiment there is a process for the production of a compound according to Formula (I) wherein X is -CH₂-, said process comprises reacting a compound of formula XII wherein T is halogen, 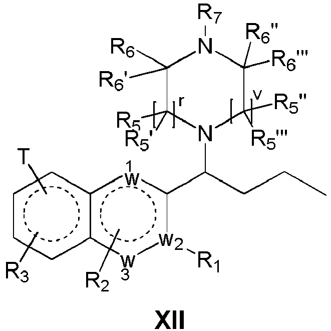 with a compound of formula XVII 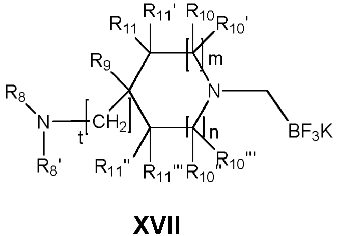 by coupling using a Pd catalyst such as palladium acetate, using a suitable base such as cesium carbonate, and a suitable ligand, preferably a phosphine ligand such as XPhos, in a suitable solvent such as mixtures of dioxane and water, at a suitable temperature, preferably heating and optionally under microwave irradiation, wherein R₁, R₂, R₃, R₄, R₅, R₅', R₅", R₅"', R₆, R₆', R₆", R₆"', R₇, R₈, R₈', R₉, R₁₀, R₁₀', R₁₀", R₁₀"', R₁₁, R₁₁', R₁₁", R₁₁"', X, w₁, w₂, w₃, n, m, t, r and v have the meanings as defined in the description.

In a particular embodiment there is a process for the production of a compound according to Formula (I), said process comprises
a) reacting a compound of formula VII wherein T is -X-R₄ 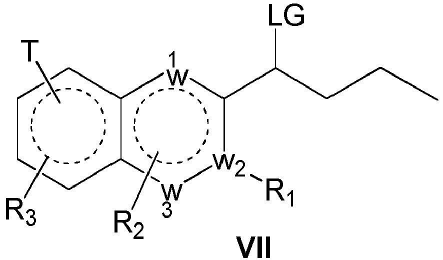 with a suitable amine of formula XI, 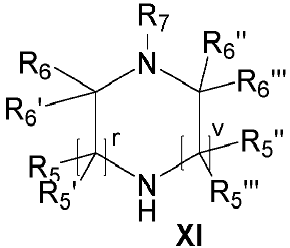 in a suitable solvent, such as acetonitrile, dichloromethane or dimethylformamide, in the presence of a base such as triethylamine, K₂CO₃ or *N*,*N*-diisopropylethylamine, at a suitable temperature comprised between room temperature and the reflux temperature, preferably heating, wherein R₁, R₂, R₃, R₄, R₅, R₅', R₅", R₅"', R₆, R₆', R₆", R₆"', R₇, X, w₁, w₂, w₃, r and v have the meanings as defined in the description; or
b) wherein w₁ is nitrogen and w₂ and w₃ are carbon, said process comprises reacting a compound of formula XIV 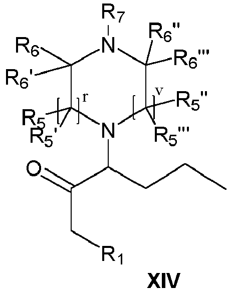 with an aminoaldehyde of formula XIII, 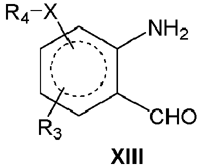 in the presence of a base, such as potassium hydroxide, in a suitable solvent, such as ethanol, at a suitable temperature, preferably heating, wherein R₁, R₂, R₃, R₄, R₅, R₅', R₅", R₅"', R₆, R₆', R₆", R₆"', R₇, X, w₁, w₂, w₃, r and v have the meanings as defined in the description;
   or
c) when X is a bond, said process comprises reacting a compound of formula XII wherein T is halogen, 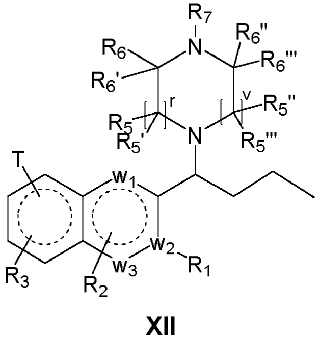 with a compound of formula XVI 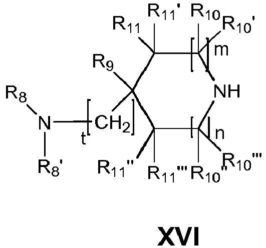 under Buchwald-Hartwig arylation conditions, using a Pd catalyst such as tris(dibenzylideneacetone)dipalladium(0) or palladium acetate, and a suitable ligand, preferably a phosphine ligand such as DavePhos, XPhos or BINAP, using a suitable base such as sodium *tert*-butoxide or cesium carbonate, in a suitable solvent such as toluene or 1,4-dioxane, at a suitable temperature, preferably heating, wherein R₁, R₂, R₃, R₄, R₅, R₅', R₅", R₅"', R₆, R₆', R₆", R₆"', R₇, R₈, R₈', R₉, R₁₀, R₁₀', R₁₀", R₁₀"', R₁₁, R₁₁', R₁₁", R₁₁"', X, w₁, w₂, w₃, n, m, t, r and v have the meanings as defined in the description;
   or
d) wherein X is -CH₂-, said process comprises reacting a compound of formula XII wherein T is halogen, 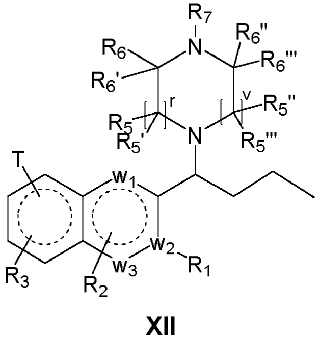 with a compound of formula XVII 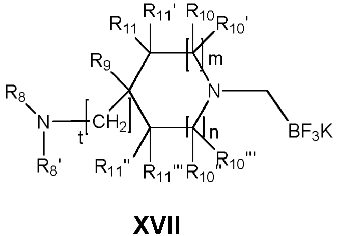 by coupling using a Pd catalyst such as palladium acetate, using a suitable base such as cesium carbonate, and a suitable ligand, preferably a phosphine ligand such as XPhos, in a suitable solvent such as mixtures of dioxane and water, at a suitable temperature, preferably heating and optionally under microwave irradiation, wherein R₁, R₂, R₃, R₄, R₅, R₅', R₅", R₅"', R₆, R₆', R₆", R₆"', R₇, R₈, R₈', R₉, R₁₀, R₁₀', R₁₀", R₁₀"', R₁₁, R₁₁', R₁₁", R₁₁"', X, w₁, w₂, w₃, n, m, t, r and v have the meanings as defined in the description.

In a particular embodiment there is a process for the production of a compound according to Formula (I), by the reduction reaction of a carbonyl derivative with a suitable reductive reagent, preferably sodium borohydride, in an organic solvent, preferably MeOH, to afford a hydroxyl compound.

In a particular embodiment there is a process for the production of a compound according to Formula (I), by deprotection reaction of a compound of formula I that contains an amine protecting group such as a carbamate, preferably tert-butoxy carbonyl, by any suitable method, such as treatment with an acid, preferably HCl or trifluoroacetic acid in an appropriate solvent such as 1,4-dioxane, DCM, ethyl acetate or a mixture of an organic solvent and water.

In a particular embodiment there is a process for the production of a compound according to Formula (I), by reductive amination reaction of a compound of formula I that contains an amino group with an aldehyde, preferably carried out with a reductive reagent, preferably sodium triacetoxyborohydride, in an organic solvent, preferably DCE, in the presence of an organic base, preferably DIPEA or TEA. Alternatively, the reaction can be carried out in the presence of an acid, preferably acetic acid.

In a particular embodiment there is a process for the production of a compound according to Formula (I), by reaction of a compound of formula I that contains an amino group with an alkylating reagent, in the presence of a base, preferably DIPEA or K₂CO₃, in an organic solvent, preferably acetonitrile, at suitable temperature, such as in the range of 0-120 °C.

In a particular embodiment there is a process for the production of a compound according to Formula (I), by reaction of a compound of formula I that contains an amino group with a vinyl derivative, in an organic solvent, preferably 2-methoxyethanol, at suitable temperature, such as in the range of 20-140 °C.

A particular embodiment of the invention refers to the use of a compound of Formula (II), 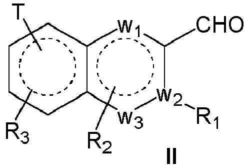 wherein T represents a halogen atom or a group XR₄, and R₁, R₂, R₃, R₄, R₅, X, w₁, w₂, and w₃ have the meanings as defined in the description, for the preparation of compounds of Formula (I).

A particular embodiment of the invention refers to the use of a compound of Formula (III), 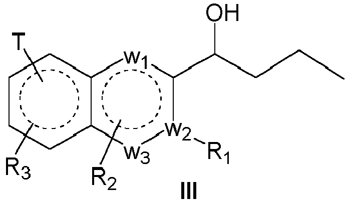 wherein T represents a halogen atom or a group XR₄, and R₁, R₂, R₃, R₄, X, w₁, w₂ and w₃ have the meanings as defined in the description, for the preparation of compounds of Formula (I).

A particular embodiment of the invention refers to the use of a propylmagnesium derivative of Formula (IV),

PrMgY (**IV**)

wherein Y represents a halogen atom, for the preparation of compounds of Formula (I).

A particular embodiment of the invention refers to the use of a a suitable allyl derivative of Formula (V),

YAllyl **V**

wherein Y represents a halogen atom, for the preparation of compounds of Formula (I).

A particular embodiment of the invention refers to the use of a compound of Formula (VI), 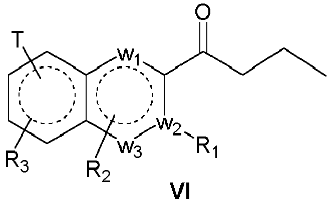 wherein T represents a halogen atom or a group XR₄, and R₁, R₂, R₃, R₄, X, w₁, w₂ and w₃ have the meanings as defined in the description, for the preparation of compounds of Formula (I).

A particular embodiment of the invention refers to the use of a compound of Formula (VII), 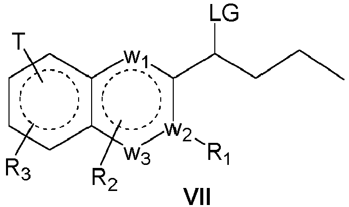 wherein LG represents a leaving group (such as chloro, bromo, iodo, mesylate, tosylate, nosylate or triflate), T represents a halogen atom or a group XR₄, and R₁, R₂, R₃, R₄, X, w₁, w₂ and w₃ have the meanings as defined in the description, for the preparation of compounds of Formula (I).

A particular embodiment of the invention refers to the use of a compound of Formula (VIII), 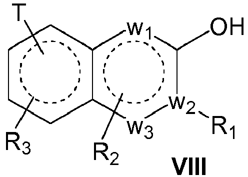 wherein T represents a halogen atom or a group XR₄, and R₁, R₂, R₃, R₄, X, w₁, w₂ and w₃ have the meanings as defined in the description, for the preparation of compounds of Formula (I).

A particular embodiment of the invention refers to the use of a suitable butylzinc derivative of Formula (IX),

YZnBu (**IX**)

wherein Y represents a halogen atom, for the preparation of compounds of Formula (I).

A particular embodiment of the invention refers to the use of a compound of Formula (X), 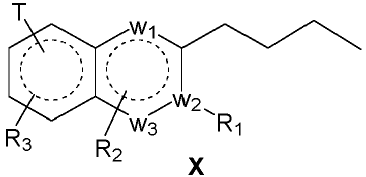 wherein T represents a halogen atom or a group XR₄, and R₁, R₂, R₃, R₄, X, w₁, w₂ and w₃ have the meanings as defined in the description, for the preparation of compounds of Formula (I).

A particular embodiment of the invention refers to the use of a compound of Formula (XI), 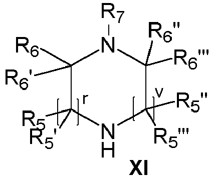 wherein R₅, R₅', R₅", R₅"', R₆, R₆', R₆", R₆"' and R₇ have the meanings as defined in the description, for the preparation of compounds of Formula (I).

A particular embodiment of the invention refers to the use of a compound of Formula (XII), 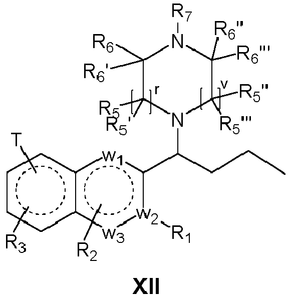 wherein T represents a halogen atom or a group XR₄ and R₁, R₂, R₃, R₄, R₅, R₅', R₅", R₅"', R₆, R₆', R₆", R₆"', R₇, X, w₁, w₂, w₃, r and v have the meanings as defined in the description, for the preparation of compounds of Formula (I).

A particular embodiment of the invention refers to the use of a compound of Formula (XIII), 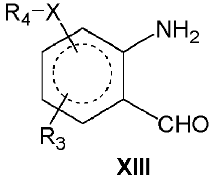 wherein R₃, R₄ and X have the meanings as defined in the description, for the preparation of compounds of Formula (I).

A particular embodiment of the invention refers to the use of a compound of Formula (XIV), 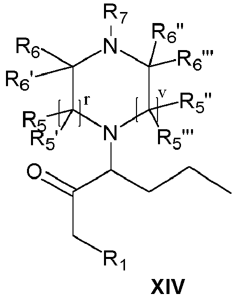 wherein R₁, R₅, R₅', R₅", R₅"', R₆, R₆', R₆", R₆"', R₇, r and v have the meanings as defined in the description, for the preparation of compounds of Formula (I).

A particular embodiment of the invention refers to the use of a compound of Formula (XV), 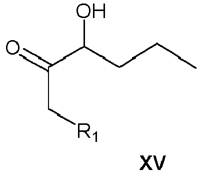 wherein R₁ has the meaning as defined in the description, for the preparation of compounds of Formula (I).

A particular embodiment of the invention refers to the use of a compound of Formula (XVI), 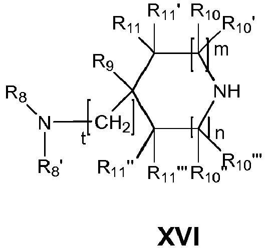 wherein R₈, R₈', R₉, R₁₀, R₁₀', R₁₀", R₁₀"', R₁₁, R₁₁', R₁₁", R₁₁"', n, m and t have the meanings as defined in the description, for the preparation of compounds of Formula (I).

A particular embodiment of the invention refers to the use of a compound of Formula (XVII), 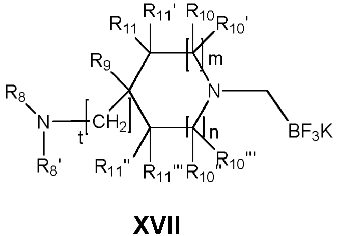 wherein R₈, R₈', R₉, R₁₀, R₁₀', R₁₀", R₁₀"', R₁₁, R₁₁', R₁₁", R₁₁"', n, m and t have the meanings as defined in the description, for the preparation of compounds of Formula (I).

A particular embodiment of the invention refers to the use of a compound of Formula II, III, IV, V, VI, VII, VIII, IX, X, XI, XII, XIII, XIV, XV, XVI or XVII 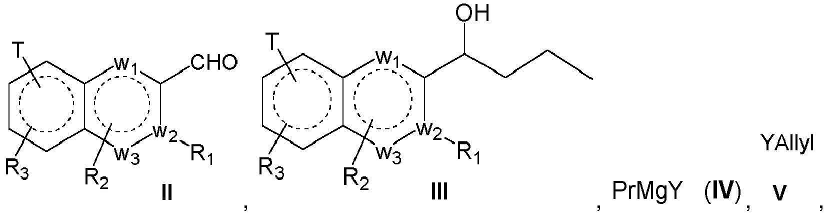 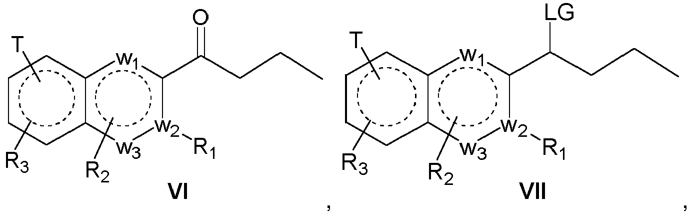 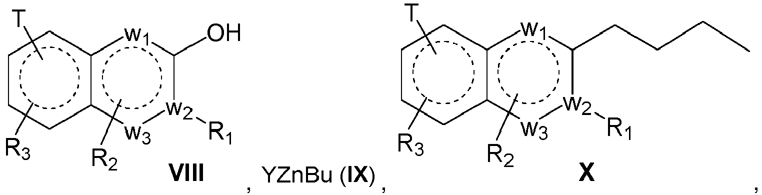 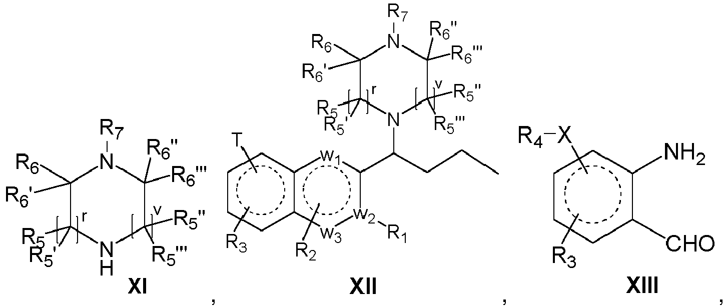 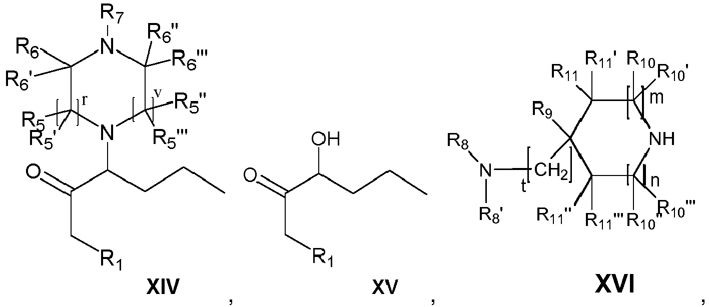 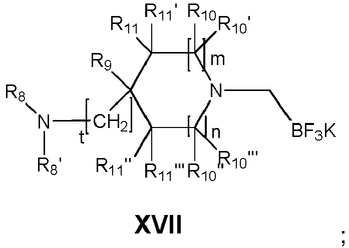 wherein LG represents a leaving group (such as chloro, bromo, iodo, mesylate, tosylate, nosylate or triflate), Y represents a halogen atom, T represents a halogen atom or a group XR₄, and R₁, R₂, R₃, R₄, R₅, R₅', R₅", R₅"', R₆, R₆', R₆", R₆"', R₇, R₈, R₈', R₉, R₁₀, R₁₀', R₁₀", R₁₀"', R₁₁, R₁₁', R₁₁", R₁₁"', X, w₁, w₂, w₃, n, m, t, r and v have the meanings as defined in the description, for the preparation of compounds of Formula (I).

In a preferred embodiment,
T represents halogen.

In a preferred embodiment,
T represents -XR₄.

The obtained reaction products may, if desired, be purified by conventional methods, such as crystallisation and chromatography. Where the above described processes for the preparation of compounds of the invention give rise to mixtures of stereoisomers, these isomers may be separated by conventional techniques such as preparative chromatography. If there are chiral centers the compounds may be prepared in racemic form, or individual enantiomers may be prepared either by enantiospecific synthesis or by resolution.

One preferred pharmaceutically acceptable form of a compound of the invention is the crystalline form, including such form in pharmaceutical composition. In the case of salts and also solvates of the compounds of the invention the additional ionic and solvent moieties must also be non-toxic. The compounds of the invention may present different polymorphic forms, it is intended that the invention encompasses all such forms.

Another aspect of the invention refers to a pharmaceutical composition which comprises a compound according to the invention as described above according to general formula I or a pharmaceutically acceptable salt or steroisomer thereof, and a pharmaceutically acceptable carrier, adjuvant or vehicle. The present invention thus provides pharmaceutical compositions comprising a compound of this invention, or a pharmaceutically acceptable salt or stereoisomers thereof together with a pharmaceutically acceptable carrier, adjuvant, or vehicle, for administration to a patient.

Examples of pharmaceutical compositions include any solid (tablets, pills, capsules, granules etc.) or liquid (solutions, suspensions or emulsions) composition for oral, topical or parenteral administration.

In a preferred embodiment the pharmaceutical compositions are in oral form, either solid or liquid. Suitable dose forms for oral administration may be tablets, capsules, syrops or solutions and may contain conventional excipients known in the art such as binding agents, for example syrup, acacia, gelatin, sorbitol, tragacanth, or polyvinylpyrrolidone; fillers, for example lactose, sugar, maize starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulfate.

The solid oral compositions may be prepared by conventional methods of blending, filling or tabletting. Repeated blending operations may be used to distribute the active agent throughout those compositions employing large quantities of fillers. Such operations are conventional in the art. The tablets may for example be prepared by wet or dry granulation and optionally coated according to methods well known in normal pharmaceutical practice, in particular with an enteric coating.

The pharmaceutical compositions may also be adapted for parenteral administration, such as sterile solutions, suspensions or lyophilized products in the apropriate unit dosage form. Adequate excipients can be used, such as bulking agents, buffering agents or surfactants.

The mentioned formulations will be prepared using standard methods such as those described or referred to in the Spanish and US Pharmacopoeias and similar reference texts.

Administration of the compounds or compositions of the present invention may be by any suitable method, such as intravenous infusion, oral preparations, and intraperitoneal and intravenous administration. Oral administration is preferred because of the convenience for the patient and the chronic character of the diseases to be treated.

Generally an effective administered amount of a compound of the invention will depend on the relative efficacy of the compound chosen, the severity of the disorder being treated and the weight of the sufferer. However, active compounds will typically be administered once or more times a day for example 1, 2, 3 or 4 times daily, with typical total daily doses in the range of from 0.1 to 1000 mg/kg/day.

The compounds and compositions of this invention may be used with other drugs to provide a combination therapy. The other drugs may form part of the same composition, or be provided as a separate composition for administration at the same time or at different time.

Another aspect of the invention refers to the use of a compound of the invention or a pharmaceutically acceptable salt or isomer thereof in the manufacture of a medicament.

Another aspect of the invention refers to a compound of the invention according as described above according to general formula I, or a pharmaceutically acceptable salt or isomer thereof, for use as a medicament for the treatment of pain. Preferably the pain is medium to severe pain, visceral pain, chronic pain, cancer pain, migraine, inflammatory pain, acute pain or neuropathic pain, allodynia or hyperalgesia. This may include mechanical allodynia or thermal hyperalgesia.

Another aspect of the invention refers to the use of a compound of the invention in the manufacture of a medicament for the treatment or prophylaxis of pain.

In a preferred embodiment the pain is selected from medium to severe pain, visceral pain, chronic pain, cancer pain, migraine, inflammatory pain, acute pain or neuropathic pain, allodynia or hyperalgesia, also preferably including mechanical allodynia or thermal hyperalgesia.

Another aspect of this invention relates to a method of treating or preventing pain which method comprises administering to a patient in need of such a treatment a therapeutically effective amount of a compound as above defined or a pharmaceutical composition thereof. Among the pain syndromes that can be treated are medium to severe pain, visceral pain, chronic pain, cancer pain, migraine, inflammatory pain, acute pain or neuropathic pain, allodynia or hyperalgesia, whereas this could also include mechanical allodynia or thermal hyperalgesia.

The present invention is illustrated below with the aid of examples. These illustrations are given solely by way of example and do not limit the general spirit of the present invention.

### General Experimental Part

### SYNTHESIS DESCRIPTION

The compounds of formula I may be prepared by a multistep process as described in Scheme 1, 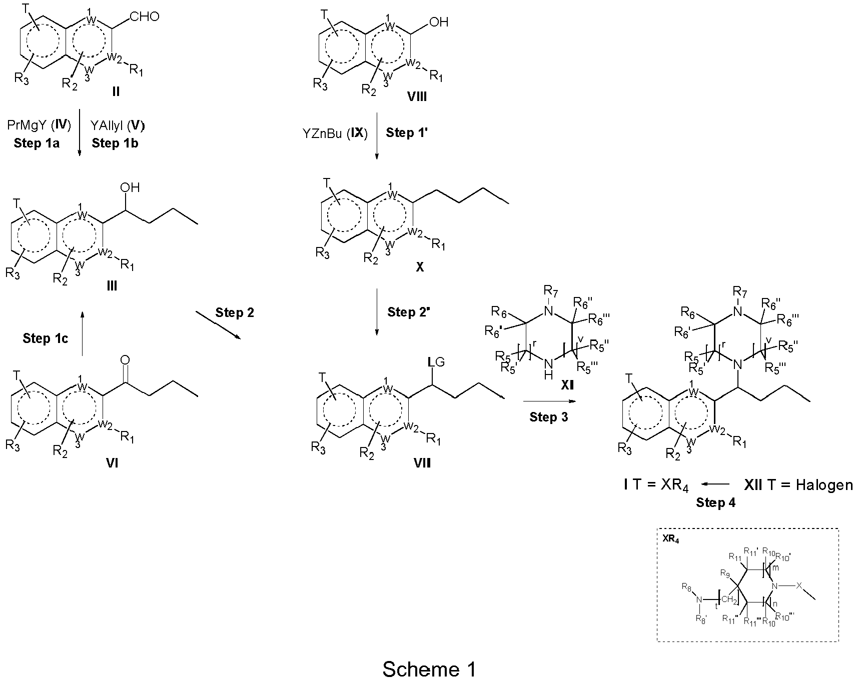 wherein R₁, R₂, R₃, R₄, R_{5-5"'}, R_{6-6"'}, w₁, w₂ and w₃ have the meanings as defined in claim 1, LG represents a leaving group (such as chloro, bromo, iodo, mesylate, tosylate, nosylate or triflate), Y represents a halogen atom and T represents a halogen atom or a group XR₄.

The process can be carried out as described below:
Step 1: A compound of formula III can be prepared (step 1a) by treating an aldehyde of formula II with a suitable propylmagnesium derivative of formula IV, in a suitable solvent, such as tetrahydrofuran or diethylether, at a suitable temperature, preferably at room temperature.
   Alternatively (step 1b), a compound of formula III can be prepared by treating an aldehyde of formula II with a suitable allyl derivative of formula V in the presence of a metal such as indium, in a suitable solvent, such as tetrahydrofuran, at a suitable temperature, preferably at room temperature; followed by reduction with a suitable reagent such as *p*-tosylhydrazide, in the presence of a base such as sodium acetate, in a suitable solvent, such as 1,4-dioxane, at a suitable temperature, preferably at reflux temperature.
   Alternatively (step 1c), a compound of formula III can be prepared by reduction of a ketone of formula VI with a suitable reducing agent, such as sodium borohydride , in a suitable solvent, such as methanol, at a suitable temperature, such as between 0 °C and room temperature.
Step 2: A compound of formula VII can be prepared by converting the hydroxyl group of a compound of formula III into a leaving group (LG). For instance, it can be converted to a bromine atom by using bromine in the presence of triphenylphosphine, in a suitable solvent, such as dichloromethane, at a suitable temperature such as between 0 °C and room temperature. Alternatively, it can be converted to a chlorine atom by using thionyl chloride in a suitable solvent, such as dichloromethane, at a suitable temperature such as room temperature. Alternatively, it can be converted to a triflate group by using triflic anhydride in the presence of a suitable base, such as 2,6-lutidine, in a suitable solvent, such as dichloromethane, at a suitable temperature, such as between -78 °C and room temperature.
   Alternatively, a compound of formula VII can be obtained by a two-step procedure from a compound of formula VIII:
   A compound of formula X can be prepared (step 1') by treating an alcohol of formula VIII with triflic anhydride in the presence of a suitable base, such as pyridine, in a suitable solvent, such as dichloromethane, at a suitable temperature such as between -78 °C and room temperature, followed by reaction with a suitable butylzinc derivative of formula IX, in the presence of an organometallic catalyst, such as Ni(dppp)Cl₂, in a suitable solvent, such as tetrahydrofuran, at a suitable temperature, preferably at room temperature.
   A compound of formula VII, where LG represents a leaving group, can be prepared (step 2') by reacting a compound of formula X with a suitable halogenating agent, such as bromine in the presence of a suitable base such as sodium acetate, in a suitable solvent, such as acetic acid, at a suitable temperature, preferably heating. Alternatively, the reaction can be carried out with N-bromosuccinimide in the presence of azobisisobutyronitrile, in a suitable solvent, such as tetrachloromethane, at a suitable temperature, preferably heating.
Step 3: A compound of formula I can be prepared by reacting a compound of formula VII with a suitable amine of formula XI, in a suitable solvent, such as acetonitrile, dichloromethane or dimethylformamide, in the presence of a base such as triethylamine, K₂CO₃ or *N*,*N*-diisopropylethylamine, at a suitable temperature comprised between room temperature and the reflux temperature, preferably heating. Alternatively, the reactions can be carried out under microwave heating and optionally using an activating agent such as sodium iodide or potassium iodide.

Alternatively, the compounds of formula I can be prepared by condensation reactions of conveniently functionalized reagents that may vary depending on the nature of the atoms w₁ to w₃. As a matter of example, a compound of formula I where w₁ is nitrogen and w₂ and w₃ are carbon (Ia), may be prepared by the method described in Scheme 2. This method comprises the reaction of a hydroxyketone of formula XV with an amine of formula XI in a suitable solvent, such as benzene, at a suitable temperature such as reflux temperature and extracting water by means of a Dean-Stark apparatus, to give a compound of formula XIV which is then treated with an aminoaldehyde of formula XIII, in the presence of a base, such as potassium hydroxide, in a suitable solvent, such as ethanol, at a suitable temperature, preferably heating.

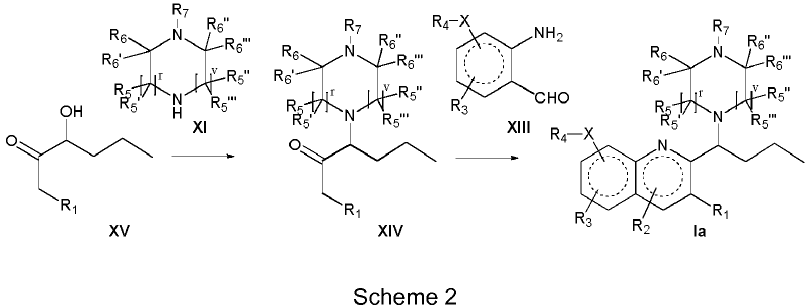

In addition, certain compounds of the present invention can also be obtained by functional group interconversion over compounds of formula I or any of the suitable intermediates shown in Scheme 1. In particular, for introducing the group XR₄ (Step 4, for transformation of a compound of formula XII to a compound of formula I, when performed as the last step of the synthesis), the following transformations may be carried out:
- a compound in which T is halogen may be converted to a compound in which T is XR₄, where X is a bond, by reaction with a compound of formula XVI 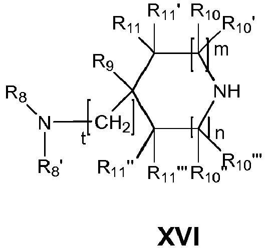 under Buchwald-Hartwig arylation conditions, using a Pd catalyst such as tris(dibenzylideneacetone)dipalladium(0) or palladium acetate, and a suitable ligand, preferably a phosphine ligand such as DavePhos, XPhos or BINAP, using a suitable base such as sodium *tert*-butoxide or cesium carbonate, in a suitable solvent such as toluene or 1,4-dioxane, at a suitable temperature, preferably heating.
- a compound in which T is halogen may be converted to a compound in which T is XR₄, where X is CH₂, by reaction with a compound of formula XVII 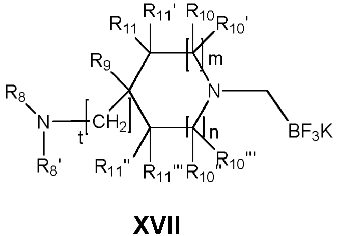 by coupling using a Pd catalyst such as palladium acetate, using a suitable base such as cesium carbonate, and a suitable ligand, preferably a phosphine ligand such as XPhos, in a suitable solvent such as mixtures of dioxane and water, at a suitable temperature, preferably heating and optionally under microwave irradiation. A compound of formula XVII may be obtained by reaction of a compound of formula XVI with potassium (bromomethyl)trifluoroborate, in a suitable solvent, such as mixtures of tetrahydrofuran/*tert*-butanol, at a suitable temperature, preferably heating.

In addition, certain compounds of the present invention can also be obtained by functional group interconversion over compounds of formula I or any of the intermediates shown in Scheme 1.
- The hydrogen atom of a position alfa to an aromatic nitrogen atom may be converted to an alkyl group by reaction with an alkyllithium or an alkylmagnesium derivative in a suitable solvent, such as tetrahydrofuran, at a suitable temperature, preferably at room temperature; followed by oxidation with ammonium cerium nitrate in a suitable solvent, such as acetone, at a suitable temperature, preferably at room temperature.-A hydroxyl or an amino group may be alkylated using a suitable alkylating agent, such as an alkyl halide, in the presence of a base, such as potassium hydroxide, in a suitable solvent, such as methanol, at a suitable temperature, preferably at room temperature.

In some of the processes described above it may be necessary to protect the reactive or labile groups present with suitable protecting groups, such as for example Boc (tert-butoxycarbonyl), Teoc (2-(trimethylsilyl)ethoxycarbonyl) or benzyl for the protection of amino groups, and common silyl protecting groups for the protection of the hydroxyl group. The procedures for the introduction and removal of these protecting groups are well known in the art and can be found thoroughly described in the literature.

In addition, a compound of formula I can be obtained in enantiopure form by resolution of a racemic compound of formula I either by chiral preparative HPLC or by crystallization of a diastereomeric salt or co-crystal. Alternatively, the resolution step can be carried out at a previous stage, using any suitable intermediate.

The compounds of formula II, IV, V, VI, VIII, IX, XI, XIII, XV and XVI used in the methods disclosed above are commercially available or can be synthesized following common procedures described in the literature.

### Examples

The following abbreviations are used in the examples:
ACN: acetonitrile
Anh: anhydrous
Aq: aqueous
AIBN: azobisisobutyronitrile
Boc: *tert*-butoxycarbonyl
CAN: ammonium cerium nitrate
Chx: cyclohexane
Conc: concentrated
DavePhos: 2-dicyclohexylphosphino-2'-(*N*,*N*-dimethylamino)biphenyl
DCE: dichloroethane
DCM: dichloromethane
EDC.HCI: *N*-(3-Dimethylaminopropyl)-*N'*-ethylcarbodiimide hydrochloride
Eq: equivalent/s
Et₂O: diethyl ether
EtOAc: ethyl acetate
EtOH: ethanol
EX: example
h: hours
HOBt: 1-hydroxybenzotriazole
HPLC: high performance liquid chromatography
IPA: isopropanol
MeOH: methanol
MS: mass spectrometry
Min: minutes
NBS: *N*-bromosuccinimide
Quant: quantitative
Rt.: retention time
r.t.: room temperature
Sat: saturated
SFC: Supercritical Fluid Chromatography
Sol: solution
tBuOH: *tert*-butanol
TEA: triethylamine
TFA: trifluoroacetic acid
THF: tetrahydrofuran
TMSOTf: trimethylsilyl trifluoromethanesulfonate
XPhos: 2-dicyclohexylphosphino-2',4',6'-triisopropylbiphenyl
Wt: weight

The following methods were used to determine the HPLC-MS spectra:
A: Column Luna C18 (2) 5 µm, 2.0x50 mm; flow rate: 0.30 mL/min; A: ACN:MeOH (1:1); B: water; C: 100 mM ammonium acetate pH 7; gradient A:B:C: 3 min in 10:85:5 + from 10:85:5 to 95:0:5 in 6 min + 6 min in 95:0:5.
B: Column: SunFire C18, 3.5 µm, 2.1x100 mm; flow rate: 0.30 mL/min; A: ACN:MeOH (1:1); B: water; C: 100 mM ammonium acetate pH 7; gradient: 5 min in 10:85:5 + from 10:85:5 to 95:0:5 in 15 min + 10 min in 95:0:5.
C: Column Acquity UPLC BEH C18 2.1 x 50 mm, 1.7 µ; flow rate: 0.61 mL/min; A: NH₄HCO₃ 10 mM, B: ACN, C: MeOH + 0.1% formic acid; gradient 0.3 min 98% A, 98%A to 0:95:5 A:B:C in 2.7 min; 0:95:5 A:B:C to 100% B in 0.1 min; isocratic 2 min 100% B.
D: Column Aquity UPLC BEH C18 2.1 x 50 mm, 1.7 µm; flow rate 0.61 mL/min; A: NH₄HCO₃ 10 mM, B: ACN; gradient 0.3 min 98% A, 98%A to 100% B in 2.65 min; isocratic 2.05 min 100% B.
E: Column Acquity UPLC BEH C18 2.1x50 mm, 1.7 µm; flow rate 0.60 mL/min; A: NH₄HCO₃ 10 mM pH 10; B: ACN; Gradient: 0.2 min in 90% A, 90% A to 5% A in 3.3 min, isocratic 0.5 min 5% A.

### Synthesis of examples

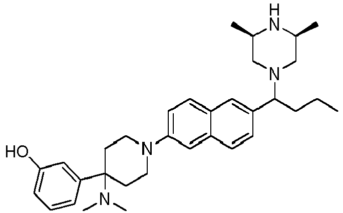

### Example 1. 3-(4-(Dimethylamino)-1-(6-(1-((3S,5R)-3,5-dimethylpiperazin-1-yl)butyl)naphthalen-2-yl)piperidin-4-yl)phenol

### Step a. 1-(6-Bromonaphthalen-2-yl)butan-1-ol.

A 2 M solution of propylmagnesium chloride in Et₂O (0.87 mL, 1.7 mmol) was added to a solution of 6-bromo-2-naphthaldehyde (340 mg, 1.4 mmol) in THF (10 mL) at 0 °C, the solution was allowed to reach r.t. and stirred for 1 h. The reaction was quenched with aq sat NH₄Cl, diluted with EtOAc and washed with brine. The organic layer was dried over anh Na₂SO₄, filtered and solvent was removed under vacuum. The crude product was purified by flash chromatography, silica gel, Chx:EtOAc to give the title compound (308 mg, Yield: 76%).

HPLC-MS (Method C): Rt. 2.38 min; ESI⁻-MS m/z: 277.

### Step b. 2-Bromo-6-(1-chlorobutyl)naphthalene.

Thionyl chloride (0.1 mL, 1.34 mmol) was added to a solution of the compound obtained in step a (75 mg, 0.27 mmol) in DCM (3 mL) and the solution was stirred for 1 h. The reaction was diluted with EtOAc and washed with aq sat NaHCO₃ and brine. The organic layer was dried over anh Na₂SO₄, filtered and concentrated to dryness to give the title compound (75 mg, Yield: 93%).

HPLC-MS (Method C): Rt. 2.89 min; ESI⁺-MS m/z: 297.

### Step c. (3S,5R)-1-(1-(6-Bromonaphthalen-2-yl)butyl)-3,5-dimethylpiperazine.

TEA (0.14 mL, 1 mmol), KI (4 mg, 0.025 mmol) and (2*R*,6*S*)-2,6-dimethylpiperazine (71 mg, 0.6 mmol) were added to a solution of the compound obtained in step b (75 mg, 0.25 mmol) in ACN (5 mL) and the reaction was stirred at 90 °C for 16 h. The mixture was concentrated under vacuum and the crude product was dissolved in EtOAc and washed with aq sat NaHCO₃. The organic layer was dried over anh Na₂SO₄, filtered and concentrated to dryness. The crude product was purified by flash chromatography, silica gel, DCM:MeOH to give the title compound (80 mg, Yield: 84%).

HPLC-MS (Method C): Rt. 2.45 min; ESI⁺-MS m/z: 375.

### Step d. Title compound.

A schlenk flask was charged with the compound obtained in step c (75 mg, 0.2 mmol), 3-(4-(dimethylamino)piperidin-4-yl)phenol dihydrochloride (70 mg, 0.24 mmol), DavePhos (15 mg, 0.04 mmol), Pd₂dba₃ (18 mg, 0.02 mmol) and NaOtBu (67 mg, 0.7 mmol) and was evacuated and backfilled with argon. Dioxane (4 mL), degassed by means of bubbling argon for 5 min, was added and the reaction mixture was heated at 100 °C for 16 h. The suspension was filtered through Celite, washed with EtOAc and the solvent was removed under vacuum. The residue was purified by flash chromatography, silica gel, DCM:MeOH, to give the title compound (42 mg, Yield: 41%).

HPLC-MS (Method C): Rt. 2.01 min; ESI⁺-MS m/z: 401.

This method was used for the preparation of EX 2-3 using suitable starting materials:

| **EX** | **STRUCTURE** | **CHEMICAL NAME** | **Rt (min)** | **MS (M+*H)*** | **HPLC Method** |
|---|---|---|---|---|---|
| **2** | 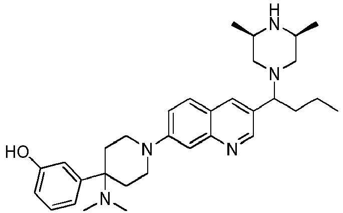 | 3-(4-(Dimethylamino)-1-(3-(1-((3*S*,5*R*)-3,5-dimethylpiperazin-1-yl)butyl)quinolin-7-yl)piperidin-4-yl)phenol | 1.74 | 516 | C |
| **3** | 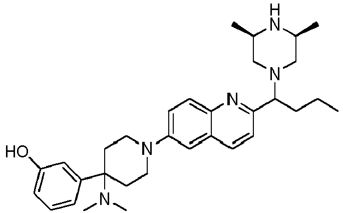 | 3-(4-(Dimethylamino)-1-(2-(1-((3*S*,5*R*)-3,5-dimethylpiperazin-1-yl)butyl)quinolin-6-yl)piperidin-4-yl)phenol | 1.69 | 516 | C |

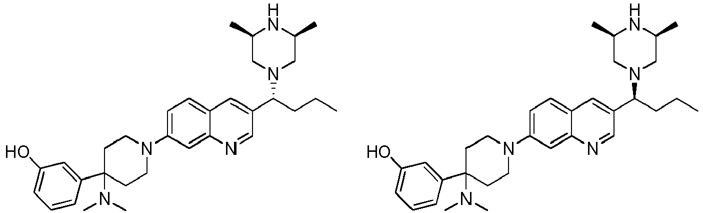

### Examples 4 and 5. 3-(4-(Dimethylamino)-1-(3-((R)-1-((3S,5R)-3,5-dimethylpiperazin-1-yl)butyl)quinolin-7-yl)piperidin-4-yl)phenol and 3-(4-(dimethylamino)-1-(3-((S)-1-((3S,5R)-3,5-dimethylpiperazin-1-yl)butyl)quinolin-7-yl)piperidin-4-yl)phenol

Starting from the compound obtained in EX 2, a chiral preparative HPLC (Column Chiralcel OD-H 20x250 mm, 5 µm; temperature: r.t.; eluent: n-Heptane/EtOH/Et₂NH 90/10/0.03 v/v/v; flow rate 13 mL/min; Rt1: 19.8 min, Rt2: 23.3 min) was carried out to give the title compounds.

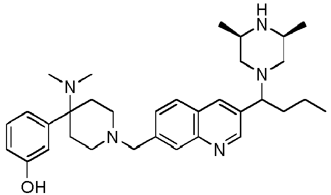

### Example 6. 3-(4-(Dimethylamino)-1-((3-(1-((3S,5R)-3,5-dimethylpiperazin-1-yl)butyl)quinolin-7-yl)methyl)piperidin-4-yl)phenol

### Step a. 7-Bromo-3-(1-((3S,5R)-3,5-dimethylpiperazin-1-yl)butyl)quinoline.

Starting from 7-bromoquinoline-3-carbaldehyde (2.1 g, 9 mmol) and following the procedure described in steps a-c of example 1, the title compound was obtained (858 mg, Yield: 25%).

HPLC-MS (Method C): Rt. 1.63 min; ESI⁺-MS m/z: 530.

### Step b. ((4-(Dimethylamino)-4-(3-hydroxyphenyl)piperidin-1-ium-1-yl)methyl)trifluoroborate.

A schlenk flask charged with 3-(4-(dimethylamino)piperidin-4-yl)phenol (1.9 g, 8.7 mmol) and potassium (bromomethyl)trifluoroborate (2.08 g, 10.38 mmol), was evacuated and backfilled with argon. THF:^{t}BuOH (2:1, 45 mL), degassed by means of bubbling argon to the solution for 5 min, was added and the reaction mixture was heated at 80 °C overnight. The mixture was dried under vacuum and the crude product was purified by flash chromatography, silica gel, DCM:MeOH to give the title compound (2.44 g, Yield: 93%).

HPLC-MS (Method C): Rt. 1.12 min; ESI⁺-MS m/z: 303.

### Step c. Title compound.

A sealed tube charged with the compound obtained in the step a (100 mg, 0.27 mmol), the compound obtained in step b (144 mg, 0.5 mmol), Pd(OAc)₂ (10 mg, 0.05 mmol), XPhos (45 mg, 0.1 mmol) and Cs₂CO₃ (260 mg, 0.8 mmol), was evacuated and backfilled with argon. Dioxane:H₂O (9:1, 10 mL), degassed by means of bubbling argon to the solution for 5 min, was added and the reaction mixture was stirred at 110 °C overnight. The solvent was removed under vacuum, the residue was dissolved in EtOAc and washed with aq NaHCO₃ sat sol. The combined organic layers were dried over anh Na₂SO₄, filtered and concentrated under vacuum. The crude product was purified by flash chromatography, silica gel, DCM:MeOH to give the title compound (68 mg, Yield: 48%).

HPLC-MS (Method C): Rt. 1.64 min; ESI⁺-MS m/z: 530.

This method was used for the preparation of EX 7 using suitable starting materials:

| **EX** | **STRUCTURE** | **CHEMICAL NAME** | **Rt (min)** | **MS (M+*H)*** | **HPLC Method** |
|---|---|---|---|---|---|
| **7** | 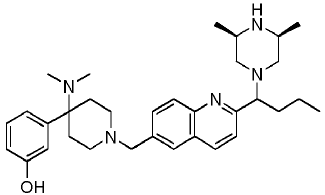 | 3-(4-(Dimethylamino)-1-((2-(1-((3*S*,5*R*)-3,5-dimethylpiperazin-1-yl)butyl)quinolin-6-yl)methyl)piperidin-4-yl)phenol | 1.75 | 530 | C |

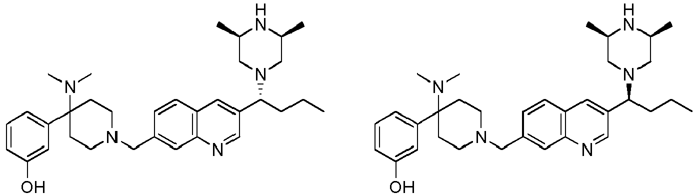

### Examples 8 and 9. 3-(4-(Dimethylamino)-1-((3-((R)-1-((3S,5R)-3,5-dimethylpiperazin-1-yl)butyl)quinolin-7-yl)methyl)piperidin-4-yl)phenol and 3-(4-(dimethylamino)-1-((3-((S)-1-((3S,5R)-3,5-dimethylpiperazin-1-yl)butyl)quinolin-7-yl)methyl)piperidin-4-yl)phenol

Starting from the compound obtained in EX 6, a chiral preparative HPLC (Column Chiralcel OD-H 20x250 mm, 5 µm; temperature: r.t.; eluent: n-Heptane/EtOH/Et2NH 93/7/0.02 v/v/v; flow rate 12 mL/min; Rt1: 25.8 min, Rt2: 31.7 min) was carried out to give the title compounds.

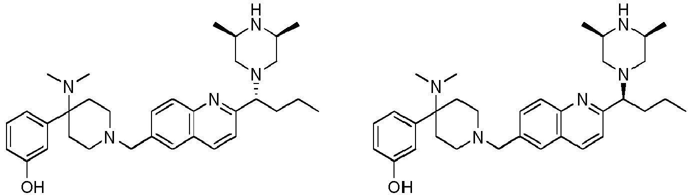

### Examples 10 and 11. 3-(4-(Dimethylamino)-1-((2-((R)-1-((3S,5R)-3,5-dimethylpiperazin-1-yl)butyl)quinolin-6-yl)methyl)piperidin-4-yl)phenol and 3-(4-(dimethylamino)-1-((2-((S)-1-((3S,5R)-3,5-dimethylpiperazin-1-yl)butyl)quinolin-6-yl)methyl)piperidin-4-yl)phenol

Starting from the compound obtained in EX 7, a chiral preparative HPLC (Column Chiralpak AD-H 20x250 mm, 5 µm; temperature: r.t.; eluent: n-Heptane/EtOH/Et₂NH 70/30/0.1 v/v/v; flow rate 11 mL/min; Rt1: 6.4 min, Rt2: 8.7 min) was carried out to give the title compounds.

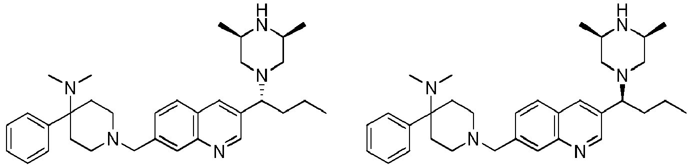

### Examples 12 and 13. 1-((3-((R)-1-((3S,5R)-3,5-Dimethylpiperazin-1-yl)butyl)quinolin-7-yl)methyl)-N,N-dimethyl-4-phenylpiperidin-4-amine and 1-((3-((S)-1-((3S,5R)-3,5-dimethylpiperazin-1-yl)butyl)quinolin-7-yl)methyl)-N,N-dimethyl-4-phenylpiperidin-4-amine

Starting from 1-((3-(1-((3*S*,5*R*)-3,5-dimethylpiperazin-1-yl)butyl)quinolin-7-yl)methyl)-*N*,*N*-dimethyl-4-phenylpiperidin-4-amine, obtained following a similar procedure to that described in EX 6, a chiral preparative HPLC separation (Column Chiralpak IA 20x250 mm, 5 µm; temperature: r.t.; eluent: n-Heptane/IPA/Et₂NH 95/5/0.02 v/v/v; flow rate 14 mL/min; Rt1: 23.3 min, Rt2: 30.7 min) was carried out to give the title compounds.

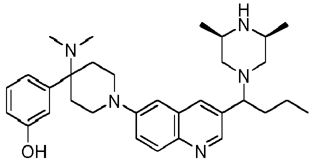

### Example 14. 3-(4-(Dimethylamino)-1-(3-(1-((3S,5R)-3,5-dimethylpiperazin-1-yl)butyl)quinolin-6-yl)piperidin-4-yl)phenol

### Step a. 6-Bromo-3-butylquinoline.

KOH (197 mg, 3mmol) was added to a solution of 2-amino-5-bromobenzaldehyde (2 g, 10 mmol) and hexanal (1.4 mL, 11.5 mmol) in EtOH (12 mL) and the solution was stirred at 75 °C for 20 h. The mixture was concentrated under vacuum and the crude product was dissolved in EtOAc and washed with water. The organic layer was dried over anh Na₂SO₄, filtered and concentrated to dryness. The crude product was purified by flash chromatography, silica gel, Chx:EtOAc to give the title compound (1.7 g, Yield: 63%).

HPLC-MS (Method C): Rt. 2.53 min; ESI⁺-MS m/z: 264.

### Step b. 6-Bromo-3-(1-bromobutyl)quinoline.

AIBN (48 mg, 0.3 mmol) and NBS (633 mg, 3.6 mmol) were added to a solution the compound obtained in step a (784 mg, 3 mmol) in CCl₄ (29 mL) and the solution was stirred at 75 °C for 20 h. The mixture was dissolved in EtOAc and washed with 10% aq K₂CO₃. The organic layer was dried over anh Na₂SO₄, filtered and concentrated to dryness to give the title compound (1.1 g, Yield: Quant.).

HPLC-MS (Method C): Rt. 2.55 min; ESI⁺-MS m/z: 342.

### Step c. 6-Bromo-3-(1-((3S,5R)-3,5-dimethylpiperazin-1-yl)butyl)quinoline.

TEA (1.5 mL, 10.7 mmol) and (2*R*,6*S*)-2,6-dimethylpiperazine (769 mg, 6.7 mmol) were added to a solution of the compound obtained in step b (1g, 2.7 mmol) in ACN (44 mL) and the reaction mixture was stirred at 90 °C for 16 h. The mixture was concentrated under vacuum and the crude product was dissolved in EtOAc and washed with aq sat NaHCO₃. The organic layer was dried over anh Na₂SO₄, filtered and concentrated to dryness. The crude product was purified by flash chromatography, silica gel, DCM:MeOH to give the title compound (367 mg, Yield: 36%).

HPLC-MS (Method C): Rt. 1.90 min; ESI⁺-MS m/z: 376.

### Step d. Title compound.

Starting from the compound obtained in step c (168 mg, 0.44 mmol) and following the procedure described in step d of EX 1, the title compound was obtained (8 mg, Yield: 3%).

HPLC-MS (Method C): Rt 1.64 min; ESI⁺-MS m/z 516.

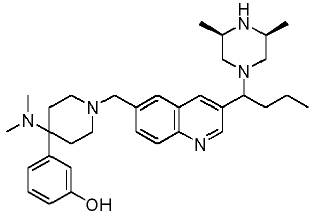

### Example 15. 3-(4-(Dimethylamino)-1-((3-(1-((3S,5R)-3,5-dimethylpiperazin-1-yl)butyl)quinolin-6-yl)methyl)piperidin-4-yl)phenol

Starting from the compound obtained in step c of EX 14 (168 mg, 0.44 mmol) and following the procedure described in step c of EX 6, the title compound was obtained (111 mg, Yield: 47%).

HPLC-MS (Method C): Rt 1.57 min; ESI⁺-MS m/z 530.

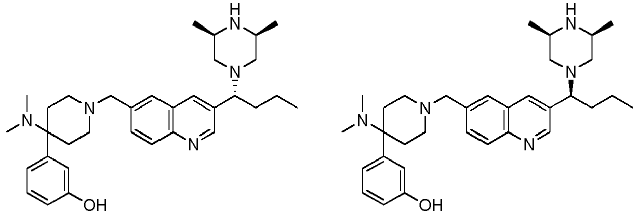

### Examples 16 and 17. 3-(4-(Dimethylamino)-1-((3-((R)-1-((3S,5R)-3,5-dimethylpiperazin-1-yl)butyl)quinolin-6-yl)methyl)piperidin-4-yl)phenol and 3-(4-(dimethylamino)-1-((3-((S)-1-((3S,5R)-3,5-dimethylpiperazin-1-yl)butyl)quinolin-6-yl)methyl)piperidin-4-yl)phenol

Starting from the compound obtained in EX 15, a chiral preparative SFC (Column Chiralpak IG 20x250 mm, 5 µm; temperature: 40 °C; eluent: EtOH/CO₂/NH₃ 25/75/0.5 v/v/v; flow rate 50 mL/min; Rt1: 17.3 min, Rt2: 20.2 min) was carried out to give the title compounds.

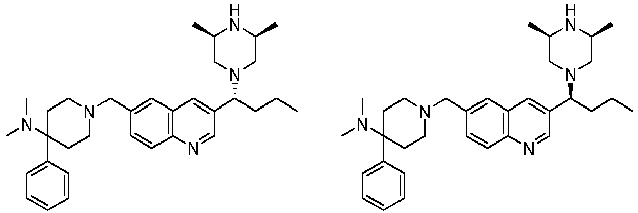

### Examples 18 and 19. 1-((3-((R)-1-((3S,5R)-3,5-Dimethylpiperazin-1-yl)butyl)quinolin-6-yl)methyl)-N,N-dimethyl-4-phenylpiperidin-4-amine and 1-((3-((S)-1-((3S,5R)-3,5-dimethylpiperazin-1-yl)butyl)quinolin-6-yl)methyl)-N,N-dimethyl-4-phenylpiperidin-4-amine

Starting from 1-((3-(1-((3*S*,5*R*)-3,5-dimethylpiperazin-1-yl)butyl)quinolin-6-yl)methyl)-*N*,*N*-dimethyl-4-phenylpiperidin-4-amine, obtained following a similar procedure to that described in EX 15, a chiral preparative HPLC separation (Column LUX C2 21.2x250 mm, 5 µm; temperature: r.t.; eluent: MeOH/NH₃ 100/0.5 v/v; flow rate 21 mL/min; Rt1: 4.4 min, Rt2: 5.1 min) was carried out to give the title compounds.

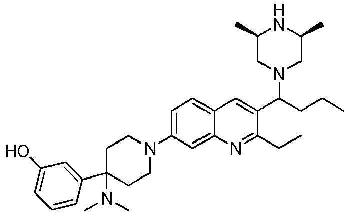

### Example 20. 3-(4-(Dimethylamino)-1-(3-(1-((3S,5R)-3,5-dimethylpiperazin-1-yl)butyl)-2-ethylquinolin-7-yl)piperidin-4-yl)phenol

### Step a. 7-Bromo-N-methoxy-N-methylquinoline-3-carboxamide.

*N*,*O*-Dimethylhydroxylamine hydrochloride (230 mg, 2.4 mmol), DIPEA (0.76 mL, 4.3 mmol), EDC·HCl (456 mg, 2.4 mmol) and HOBt (80 mg, 0.6 mmol) were added to a solution of 7-bromoquinoline-3-carboxylic acid (500 mg, 2 mmol) in DCM (15 mL) and the mixture was stirred at r.t. for 16 h. The reaction was diluted with DCM and washed with 10% aq NaOH and H₂O. The organic layer was dried over anh Na₂SO₄, filtered and concentrated to dryness. The crude product was purified by flash chromatography, silica gel, Chx:EtOAc to give the title compound (502 mg, Yield: 86%).

HPLC-MS (Method C): Rt. 1.71 min; ESI⁺-MS m/z: 295.

### Step b. 7-Bromo-2-ethyl-N-methoxy-N-methylquinoline-3-carboxamide.

A 1 M solution of ethylmagnesium bromide in THF (4.5 mL, 4.5 mmol) was added to a solution of the compound obtained in step a (1 g, 3.5 mmol) in THF (30 mL) at 0 °C, the solution was allowed to reach r.t. and stirred for 30 min. The reaction was quenched with aq sat NH₄Cl, diluted with EtOAc and washed with brine. The organic layer was dried over anh Na₂SO₄, filtered and solvent was removed under vacuum. The crude material was dissolved in acetone, CAN (2.2 g, 4 mmol) was added in portions and the reaction was stirred for 15 min. The mixture was concentrated under vacuum and the crude product was dissolved in Et₂O and washed with aq sat NaHCO₃. The organic layer was dried over anh Na₂SO₄, filtered and concentrated to dryness. The crude product was purified by flash chromatography, silica gel, Chx:EtOAc to give the title compound (1 g, Yield: 97%).

HPLC-MS (Method C): Rt. 1.86 min; ESI⁺-MS m/z: 323.

### Step c. 7-Bromo-2-ethylquinoline-3-carbaldehyde.

A 1 M solution of LiAlH₄ in THF (3.9 mL, 3.9 mmol) was added to a solution of the compound obtained in step b (1 g, 3.3 mmol) in THF (30 mL) at 0 °C, the solution was allowed to reach r.t. and stirred for 30 min. The reaction was quenched with Rochelle salt, extracted with Et₂O and washed with brine. The organic layer was dried over anh Na₂SO₄, filtered and concentrated to dryness. The crude material was dissolved in acetone, CAN (1.8 g, 3.3 mmol) was added in portions and the reaction was stirred for 15 min. The mixture was concentrated under vacuum and the crude product was dissolved in Et₂O and washed with aq sat NaHCO₃. The organic layer was dried over anh Na₂SO₄, filtered and concentrated to dryness to give the title compound (870 mg, Yield: 99%).

HPLC-MS (Method C): Rt. 2.02 min; ESI⁺-MS m/z: 264.

### Step d. 1-(7-Bromo-2-ethylquinolin-3-yl)but-3-en-1-ol.

An oven-dried round-bottomed flask was placed under argon and charged with indium powder (35 mg, 0.3 mmol), pyridine (0.02 mL, 0.3 mmol) and allyl bromide (0.03 mL, 0.3 mmol) in anh THF (2 mL). The mixture was stirred vigorously for 30 min. Then, the compound obtained in step c (40 mg, 0.15 mmol) was added and the mixture was stirred for 16 h. The reaction was quenched with aq sat NH₄Cl, diluted with EtOAc and washed with brine. The organic layer was dried over anh Na₂SO₄, filtered and the solvent was removed under vacuum to give the title compound (40 mg, Yield: 86%).

HPLC-MS (Method C): Rt. 2.03 min; ESI⁺-MS m/z: 306.

### Step e. 1-(7-Bromo-2-ethylquinolin-3-yl)butan-1-ol.

*p*-Tosylhydrazide (608 mg, 3.3 mmol) and sodium acetate (268 mg, 3.3 mmol) were added to a solution of the compound obtained in step d (250 mg, 0.8 mmol) in 1,4-dioxane (6 mL) and the reaction was heated at 110 °C for 24 h. The crude material was dissolved in EtOAc and washed with aq sat NaHCO₃. The organic layer was dried over anh Na₂SO₄, filtered and concentrated to dryness. The crude product was purified by flash chromatography, silica gel, Chx:EtOAc to give the title compound (188 mg, Yield: 74%).

HPLC-MS (Method C): Rt. 2.14 min; ESI⁺-MS m/z: 308.

### Step f. 7-Bromo-3-(1-bromobutyl)-2-ethylquinoline.

Bromine (0.04 mL, 0.78 mmol) was added dropwise to a solution of triphenylphosphine (204 mg, 0.78 mmol) in DCM (5 mL) at 0 °C, resulting in a colorless suspension. After fifteen minutes the compound obtained in step e (200 mg, 0.65 mmol) was added, and the mixture stirred during 3 h. The solvent was partially evaporated and hexane was added to precipitate triphenylphosphine oxide. The precipitate was filtered through a plug of silica gel and the filtrate was evaporated to give the title compound (240 mg, Yield: 99%).

HPLC-MS (Method C): Rt. 2.75 min; ESI⁺-MS m/z: 370.

### Step g. 7-Bromo-3-(1-((3S,5R)-3,5-dimethylpiperazin-1-yl)butyl)-2-ethylquinoline.

Starting from the compound obtained in step f (240 mg, 0.65 mmol) and following the procedure described in step c of EX 14, the title compound was obtained (158 mg, Yield: 60%).

HPLC-MS (Method C): Rt. 2.15 min; ESI⁺-MS m/z: 404.

### Step h. Title compound.

Starting from the compound obtained in step g (100 mg, 0.25 mmol) and following the procedure described in step d of EX 1, the title compound was obtained (14 mg, Yield: 10%).

HPLC-MS (Method C): Rt. 1.86 min; ESI⁺-MS m/z 544.

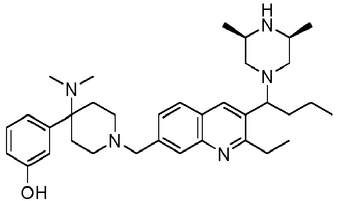

### Example 21. 3-(4-(Dimethylamino)-1-((3-(1-((3S,5R)-3,5-dimethylpiperazin-1-yl)butyl)-2-ethylquinolin-7-yl)methyl)piperidin-4-yl)phenol

Starting from the compound obtained in step g of EX 20 (100 mg, 0.25 mmol) and following the procedure described in step c of EX 6, the title compound was obtained (65 mg, Yield: 47%).

HPLC-MS (Method C):Rt. 1.77 min: ESI⁺-MS m/z 558.

This method was used for the preparation of EX 22 using suitable starting materials:

| **EX** | **STRUCTURE** | **CHEMICAL NAME** | **Rt (min)** | **MS (M+*H)*** | **HPLC Method** |
|---|---|---|---|---|---|
| **22** | 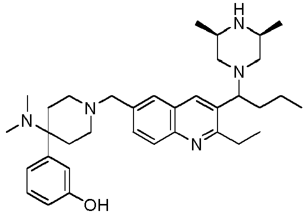 | 3-(4-(Dimethylamino)-1-((3-(1-((3*S*,5*R*)-3,5-dimethylpiperazin-1-yl)butyl)-2-ethylquinolin-6-yl)methyl)piperidin-4-yl)phenol | 1.69 | 558 | C |

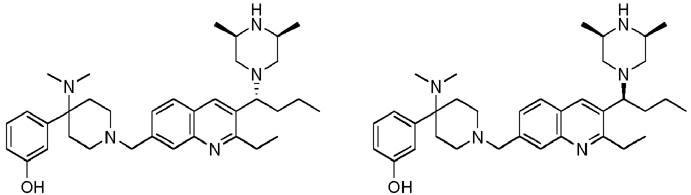

### Examples 23 and 24. 3-(4-(Dimethylamino)-1-((3-((R)-1-((3S,5R)-3,5-dimethylpiperazin-1-yl)butyl)-2-ethylquinolin-7-yl)methyl)piperidin-4-yl)phenol and 3-(4-(dimethylamino)-1-((3-((S)-1-((3S,5R)-3,5-dimethylpiperazin-1-yl)butyl)-2-ethylquinolin-7-yl)methyl)piperidin-4-yl)phenol

Starting from the compound obtained in EX 21, a chiral preparative HPLC (Column Chiralpak IC 4.6 x 250 mm, 5 µm; temperature: r.t.; eluent: n-Hexane/EtOH/Isopropyl Amine 80/20/0.1 v/v/v; flow rate: 1 mL/min; Rt1: 22.8 min, Rt2: 27.1 min) was carried out to give the title compounds.

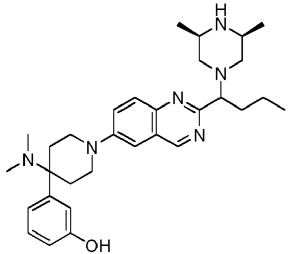

### Example 25. 3-(4-(Dimethylamino)-1-(2-(1-((3S,5R)-3,5-dimethylpiperazin-1-yl)butyl)quinazolin-6-yl)piperidin-4-yl)phenol

### Step a. N-(4-Bromo-2-formylphenyl)pentanamide.

TEA (2.45 mL, 17.6 mmol) was added to a solution of 2-amino-5-bromobenzaldehyde (2.2 g, 11 mmol) in DCM (50 mL) under argon atmosphere and the mixture was stirred for 10 min. The solution was cooled at 0 °C and pentanoyl chloride (2 mL, 16.5 mmol) was added. The mixture was allowed to reach r.t. and stirred for 1 h. The resulting mixture was diluted with DCM (200 mL), washed with aq sat NaHCO₃ and the organic layer was dried over anh Na₂SO₄ and filtered. The solvent was removed under vacuum to give the title compound (3.1 g, Yield: 99%).

HPLC-MS (Method C): Rt. 2.27 min; ESI⁺-MS m/z: 284.

### Step b. 6-Bromo-2-butylquinazoline.

A 30% aq NH₃ solution (525 mL) was added to a solution of the compound obtained in step a (3.1 g, 11 mmol) in IPA (50 mL) and the solution was stirred at 65 °C for 4 h. The mixture was partially concentrated under vacuum and the residue was dissolved in EtOAc and washed with brine. The organic layer was dried over anh Na₂SO₄, filtered and concentrated to dryness. The crude product was purified by flash chromatography, silica gel, Chx:EtOAc to give the title compound (1.25 g, Yield: 43%).

HPLC-MS (Method C): Rt. 2.23 min; ESI⁺-MS m/z: 265.

### Step c. 6-Bromo-2-(1-bromobutyl)quinazoline.

To a solution of the compound obtained in step b (1.25 g, 4.7 mmol) in acetic acid (50 mL), NaOAc (464 mg, 5.6 mmol) was added in portions and the reaction was stirred for 15 min at r.t. Bromine (0.3 mL, 5.6 mmol) was slowly added and the reaction was heated at 100 °C for 3 h. The mixture was concentrated under vacuum and the residue was dissolved in EtOAc and washed twice with 10% NaHSO₃ aq sol and brine. The organic layer was dried over anh Na₂SO₄, filtered and the solvent was removed under vacuum. The crude product was purified by flash chromatography, silica gel, Chx:EtOAc to give the title compound (1.6 g, Yield: 99%).

HPLC-MS (Method C): Rt. 2.47 min; ESI⁺-MS m/z: 343.

### Step d. 6-Bromo-2-(1-((3S,5R)-3,5-dimethylpiperazin-1-yl)butyl)quinazoline.

Starting from the compound obtained in step c (640 mg, 1.8 mmol) and following the procedure described in step c of EX 1, the title compound was obtained (458 mg, Yield: 65%).

HPLC-MS (Method C): Rt. 1.77 min; ESI⁺-MS m/z: 377.

### Step e. Title compound.

Starting from the compound obtained in step d (410 mg, 1.09 mmol) and following the procedure described in step d of EX 1, the title compound was obtained (91 mg, Yield: 16%).

HPLC-MS (Method E): Rt. 2.26 min; ESI⁺-MS m/z 517.

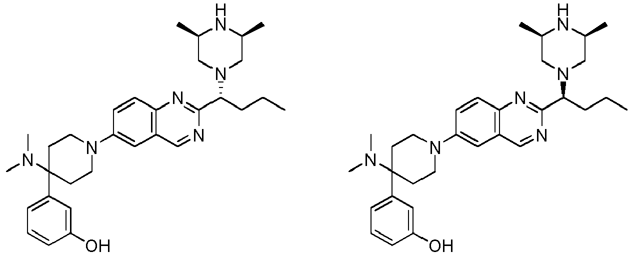

### Examples 26 and 27. 3-(4-(Dimethylamino)-1-(2-((R)-1-((3S,5R)-3,5-dimethylpiperazin-1-yl)butyl)quinazolin-6-yl)piperidin-4-yl)phenol and 3-(4-(dimethylamino)-1-(2-((S)-1-((3S,5R)-3,5-dimethylpiperazin-1-yl)butyl)quinazolin-6-yl)piperidin-4-yl)phenol

Starting from the compound obtained in EX 25, a chiral preparative HPLC (Column Chiralcel OD-H 20x250 mm, 5 µm; temperature: r.t.; eluent: n-Heptane/IPA/Et₂NH 90/10/0.03 v/v/v; flow rate 13 mL/min; Rt1: 23.6 min, Rt2: 29.7 min) was carried out to give the title compounds.

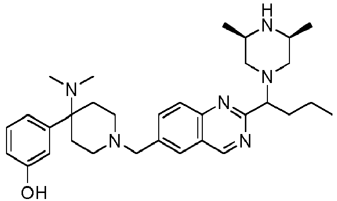

### Example 28. 3-(4-(Dimethylamino)-1-((2-(1-((3S,5R)-3,5-dimethylpiperazin-1-yl)butyl)quinazolin-6-yl)methyl)piperidin-4-yl)phenol

Starting from the compound obtained in step d of EX 25 (388 mg, 1 mmol) and following the procedure described in step c of EX 6, the title compound was obtained (155 mg, Yield: 28%).

HPLC-MS (Method C): Rt: 1.52 min; ESI⁺-MS m/z 531.

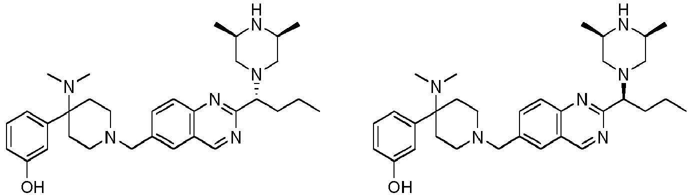

### Examples 29 and 30. 3-(4-(Dimethylamino)-1-((2-((R)-1-((3S,5R)-3,5-dimethylpiperazin-1-yl)butyl)quinazolin-6-yl)methyl)piperidin-4-yl)phenol and 3-(4-(dimethylamino)-1-((2-((S)-1-((3S,5R)-3,5-dimethylpiperazin-1-yl)butyl)quinazolin-6-yl)methyl)piperidin-4-yl)phenol

Starting from the compound obtained in EX 28, a chiral preparative HPLC (Column Chiralpal AD-H 20x250 mm, 5 µm; temperature: r.t.; eluent: n-Heptane/IPA/Et₂NH 80/20/0.07 v/v/v; flow rate 12 mL/min; Rt1: 9.5 min, Rt2: 13.5 min) was carried out to give the title compounds.

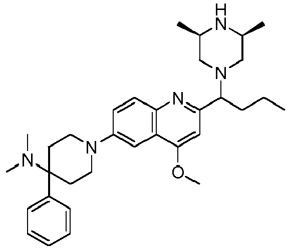

### Example 31. 1-(2-(1-((3S,5R)-3,5-Dimethylpiperazin-1-yl)butyl)-4-methoxyquinolin-6-yl)-N,N-dimethyl-4-phenylpiperidin-4-amine

### Step a. N-(2-acetyl-4-bromophenyl)pentanamide.

Starting from 1-(2-amino-5-bromophenyl)ethan-1-one (4.03 g, 18.83 mmol) and following the procedure described in step a of EX 25, the title compound was obtained (5.33 g, Yield: 95%).

HPLC (Method A): Rt, 11.07 min; ESI⁺-MS *m*/*z*, 298-300 (M+H).

### Step b. 6-Bromo-2-butylquinolin-4-ol.

TMSOTf (18.7 mL, 107 mmol) was slowly added to a solution of Et₃N (7.4 mL, 53.3 mmol) and the compound obtained in step a (5.30 g, 17.8 mmol) in 1,2-DCE (80 mL) (Caution, fume evolution is observed; exothermic). The resulting red solution was heated under reflux for 40 h; the reaction was allowed to reach r.t. and was cooled at 0 °C (ice bath) before adding MeOH (50 mL). Volatiles were removed and the resulting residue was dissolved in EtOAc and washed with NaOH (10% aq solution). The aqueous layer was extracted with EtOAc and the combined organic layers were dried over anh Na₂SO₄, filtered and concentrated. The crude residue was purified by flash chromatography, silica gel, MeOH:DCM followed by a second purification EtOAc:hexanes, to give the title compound (4.24 g, Yield: 85%).

HPLC (Method A): Rt, 9.56 min; ESI⁺-MS *m*/*z,* 280-282 (M+H).

### Step c. 6-Bromo-2-butyl-4-methoxyquinoline.

KOH (2.32 g, 41.35 mmol) and iodomethane (8.60 mL, 138 mmol) were added to a solution of the compound obtained in step b (3.86 g, 13.78 mmol) in MeOH (45 mL) and the mixture was stirred at r.t. for 6 days. Volatiles were removed and the resulting residue was dissolved in DCM and water. The aqueous layer was extracted with DCM and the combined organic layers were dried over anh Na₂SO₄, filtered and concentrated. The crude residue was purified by flash chromatography, silica gel, MeOH:DCM to give the title compound (0.49 g, pale yellow solid, Yield: 12%).

HPLC (Method A): Rt, 11.62 min; ESI⁺-MS *m*/*z*, 294-296 (M+H).

### Step d. 6-Bromo-2-(1-((3S,5R)-3,5-dimethylpiperazin-1-yl)butyl)-4-methoxyquinoline.

Starting from the compound obtained in step c (0.47 g, 1.60 mmol), and following the procedure described in steps b and c of EX 14, the title compound was obtained (0.26 g, pale yellow oil, Yield: 43%).

HPLC (Method B): Rt, 19.02 min; ESI⁺-MS *m*/*z*, 406-408 (M+H).

### Step e. Title compound.

Starting from the compound obtained in step d (55 mg, 0.12 mmol) and *N*,*N*-dimethyl-4-phenylpiperidin-4-amine (80 mg, 0.29 mmol), and following the procedure described in step d of EX 1, the title compound was obtained (48 mg, Yield: 80%).

HPLC-MS (Method B): Rt, 18.69 min; ESI⁺-MS *m*/*z,* 530 (M+H).

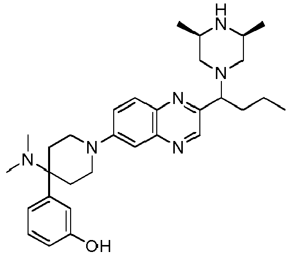

### Example 32. 3-(4-(Dimethylamino)-1-(2-(1-((3S,5R)-3,5-dimethylpiperazin-1-yl)butyl)quinoxalin-6-yl)piperidin-4-yl)phenol

### Step a. 6-Bromoquinoxalin-2-yl trifluoromethanesulfonate.

A 1 M solution of trifluoromethanesulfonic anhydride in DCM (25 mL, 25 mmol) was added to a solution of 6-bromoquinoxalin-2-ol (2.8 g, 12.5 mmol) and pyridine (4 mL, 50 mmol) in DCM at 0 °C and the mixture was stirred at r.t. for 3 h. The reaction was quenched with sat NH₄Cl and extracted with EtOAc. The organic layer was dried over anh Na₂SO₄, filtered and solvent was removed under vacuum to give the title compound (4.4 g, Yield: 98%).

HPLC-MS (Method C): Rt. 2.51 min; ESI⁻-MS m/z: 355.

### Step b. 6-Bromo-2-butylquinoxaline.

A 0.5 M solution of butylzinc bromide in THF (6.1 mL, 3.05 mmol) was added to a degassed solution of the compound obtained in step a (1 g, 2.8 mmol) and Ni(dppp)Cl₂ (151 mg, 0.28 mmol) in THF (30 mL) and the mixture was stirred at r.t. for 16 h. The mixture was dissolved in EtOAc and washed with aq sat NaHCO₃ and brine. The organic layer was dried over anh Na₂SO₄, filtered and concentrated to dryness. The crude product was purified by flash chromatography, silica gel, Chx:EtOAc to give the title compound (631 mg, Yield: 85%).

HPLC-MS (Method C): Rt. 2.45 min; ESI⁺-MS m/z: 265.

### Step c. 6-Bromo-2-(1-bromobutyl)quinoxaline.

Starting from the compound obtained in step b (795 mg, 3 mmol) and following the procedure described in step c of EX 25, the title compound was obtained (972 mg, 94%).

HPLC-MS (Method C): Rt. 2.66 min; ESI⁺-MS m/z: 343.

### Step d. 6-Bromo-2-(1-((3S,5R)-3,5-dimethylpiperazin-1-yl)butyl)quinoxaline.

Starting from the compound obtained in step c (972 mg, 2.8 mmol) and following the procedure described in step c of EX 1, the title compound was obtained (230 mg, 22%).

HPLC-MS (Method C): Rt. 1.93 min; ESI⁺-MS m/z: 377.

### Step e. Title compound.

Starting from the compound obtained in step d (115 mg, 0.3 mmol) and following the procedure described in step d of EX 1, the title compound was obtained (39 mg, Yield: 25%).

HPLC-MS (Method C): Rt. 166 min; ESI⁺-MS m/z 517.

This method was used for the preparation of EX 33 using suitable starting materials:

| **EX** | **STRUCTURE** | **CHEMICAL NAME** | **Rt (min)** | **MS (M+*H)*** | **HPLC Method** |
|---|---|---|---|---|---|
| **33** | 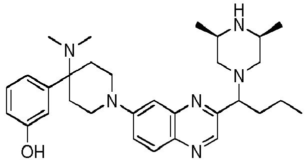 | 3-(4-(Dimethylamino)-1-(3-(1-((3*S*,5*R*)-3,5-dimethylpiperazin-1-yl)butyl)quinoxalin-6-yl)piperidin-4-yl)phenol | 1.78 | 517 | C |

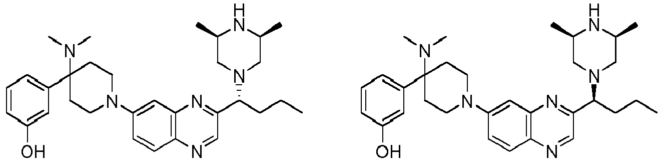

### Examples 34 and 35. 3-(4-(Dimethylamino)-1-(3-((R)-1-((3S,5R)-3,5-dimethylpiperazin-1-yl)butyl)quinoxalin-6-yl)piperidin-4-yl)phenol and 3-(4-(dimethylamino)-1-(3-((S)-1-((3S,5R)-3,5-dimethylpiperazin-1-yl)butyl)quinoxalin-6-yl)piperidin-4-yl)phenol

Starting from the compound obtained in EX 33, a chiral preparative SFC (Column LUX C4 21.2 mm x 250 mm, 5 µm; temperature: 40 °C; eluent: MeOH/CO₂/Et₂NH 35/65/0.5 v/v/v; Rt1: 7.9 min, Rt2: 9.8 min) was carried out to give the title compounds.

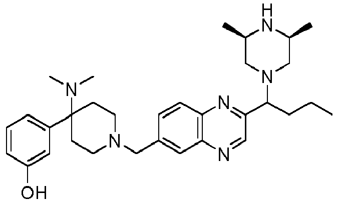

### Example 36. 3-(4-(Dimethylamino)-1-((2-(1-((3S,5R)-3,5-dimethylpiperazin-1-yl)butyl)quinoxalin-6-yl)methyl)piperidin-4-yl)phenol

Starting from the compound obtained in step d of example 32 (50 mg, 0.13 mmol) and following the procedure described in step c of example 6, the title compound was obtained (28 mg, Yield: 40%).

HPLC-MS (Method C): Rt 1.64 min; ESI⁺-MS m/z 351.

This method was used for the preparation of EX 37 using suitable starting materials:

| **EX** | **STRUCTURE** | **CHEMICAL NAME** | **Rt (min)** | **MS (M+*H)*** | **HPLC Method** |
|---|---|---|---|---|---|
| **37** | 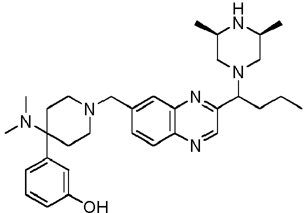 | 3-(4-(Dimethylamino)-1-((3-(1-((3*S*,5*R*)-3,5-dimethylpiperazin-1-yl)butyl)quinoxalin-6-yl)methyl)piperidin-4-yl)phenol | 1.74 | 531 | C |

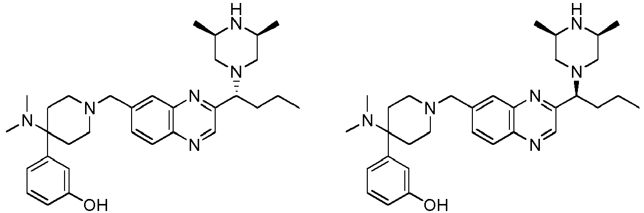

### Examples 38 and 39. 3-(4-(Dimethylamino)-1-((3-((R)-1-((3S,5R)-3,5-dimethylpiperazin-1-yl)butyl)quinoxalin-6-yl)methyl)piperidin-4-yl)phenol and 3-(4-(dimethylamino)-1-((3-((S)-1-((3S,5R)-3,5-dimethylpiperazin-1-yl)butyl)quinoxalin-6-yl)methyl)piperidin-4-yl)phenol

Starting from the compound obtained in EX 37, a chiral preparative HPLC [Column Chiralpak AD-H 20x250 mm, 5 µm; temperature: r.t.; eluent: n-Heptane/EtOH/Et₂NH 95/5/0.02 v/v/v; flow rate 13 mL/min; Rt1: 19.5 min, Rt2: 22.9 min] was carried out to give the title compounds.

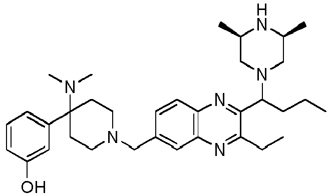

### Example 40. 3-(4-(Dimethylamino)-1-((2-(1-((3S,5R)-3,5-dimethylpiperazin-1-yl)butyl)-3-ethylquinoxalin-6-yl)methyl)piperidin-4-yl)phenol

### Step a. (2S,6R)-tert-Butyl 4-(1-(6-bromoquinoxalin-2-yl)butyl)-2,6-dimethylpiperazine-1-carboxylate.

TEA (0.30 mL, 2 mmol) and di-*tert*-butyl dicarbonate (288 mg, 1.3 mmol) were added in portions to a solution of the compound obtained in step d of EX 32 (200 mg, 0.53 mmol) in DCM (8 mL) and the reaction mixture was stirred at r.t. for 16 h. The reaction mixture was washed with aq sat Na₂CO₃, water and brine. The organic layer was dried over anh Na₂SO₄, filtered and concentrated to dryness. The crude product was purified by flash chromatography, silica gel, DCM:MeOH to give the title compound (172 mg, Yield: 68%).

HPLC-MS (Method C): Rt. 3.14 min; ESI⁺-MS m/z: 477.

### Step b. (2S,6R)-tert-Butyl 4-(1-(6-bromo-3-ethylquinoxalin-2-yl)butyl)-2,6-dimethylpiperazine-1-carboxylate.

A 0.5 M solution of ethyllithium in Chx (16 mL, 8 mmol) was added to a solution of the compound obtained in step a (1.5 g, 3.2 mmol) in THF (59 mL) at 0 °C and the solution was allowed to reach r.t and stirred for 16 h. The reaction was quenched with aq sat NH₄Cl, diluted with EtOAc and washed with brine. The organic layer was dried over Na₂SO₄, filtered and concentrated to dryness. The crude material was dissolved in acetone, CAN (2.3 g, 4.2 mmol) was added in portions and the reaction mixture was stirred for 30 min. The solvent was concentrated under vacuum and the crude product was dissolved in Et₂O and washed with aq sat NaHCO₃. The organic layer was dried over anh Na₂SO₄, filtered and concentrated to dryness to give the title compound (178 mg, Yield: 12%).

HPLC-MS (Method C): Rt. 3.44 min; ESI⁺-MS m/z: 505.

### Step c. 6-Bromo-2-(1-((3S,5R)-3,5-dimethylpiperazin-1-yl)butyl)-3-ethylquinoxaline.

TFA (2.5 mL) was added to a solution of the compound obtained in step b (178 mg, 0.35 mmol) in DCM (10 mL) and the mixture was stirred at r.t. for 16 h. The reaction mixture was basified with 20% aq NaOH and extracted with DCM. The combined organic layers were dried over anh Na₂SO₄, filtered and concentrated to dryness. The crude product was purified by flash chromatography, silica gel, DCM:MeOH to give the title compound (83 mg, Yield: 58%).

HPLC-MS (Method C): Rt. 2.48 min; ESI⁺-MS m/z: 405.

### Step d. Title compound.

Starting from the compound obtained in step c (184 mg, 0.45 mmol) and following the procedure described in step c of EX 6, the title compound was obtained (62 mg, Yield: 24%).

HPLC-MS (Method C): Rt. 1.87 min; ESI⁺-MS m/z 559.

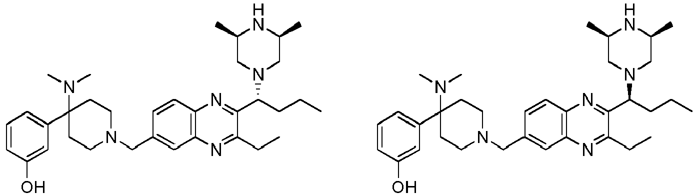

### Examples 41 and 42. 3-(4-(Dimethylamino)-1-((2-((R)-1-((3S,5R)-3,5-dimethylpiperazin-1-yl)butyl)-3-ethylquinoxalin-6-yl)methyl)piperidin-4-yl)phenol and 3-(4-(dimethylamino)-1-((2-((S)-1-((3S,5R)-3,5-dimethylpiperazin-1-yl)butyl)-3-ethylquinoxalin-6-yl)methyl)piperidin-4-yl)phenol

Starting from the compound obtained in EX 40, a chiral preparative HPLC (Column Chiralpak AD-H 20x250 mm, 5 µm; temperature: r.t.; eluent: n-Heptane/IPA/Et₂NH 85/15/0.05 v/v/v; flow rate 11 mL/min; Rt1: 6.8 min, Rt2: 16.3 min) was carried out to give the title compounds.

### Table of Examples with binding to the u-opioid receptor and the α₂δ-1 subunit of the voltage-gated calcium channel:

### BIOLOGICAL ACTIVITY

### Pharmacological study

### Human α₂δ-1 subunit of Caᵥ2.2 calcium channel assay

Human α₂δ-1 enriched membranes (2.5 µg) were incubated with 15 nM of radiolabeled [3H]-Gabapentin in assay buffer containing Hepes-KOH 10mM, pH 7.4. NSB (non specific binding) was measured by adding 10 µM pregabalin. The binding of the test compound was measured at either one concentration (% inhibition at 1 or 10 µM) or five different concentrations to determine affinity values (Ki). After 60 min incubation at 27 °C, binding reaction was terminated by filtering through Multiscreen GF/C (Millipore) presoaked in 0.5 % polyethyleneimine in Vacuum Manifold Station, followed by 3 washes with ice-cold filtration buffer containing 50 mM Tris-HCl, pH 7.4. Filter plates were dried at 60 °C for 1 hour and 30 µl of scintillation cocktail were added to each well before radioactivity reading. Readings were performed in a Trilux 1450 Microbeta radioactive counter (Perkin Elmer).

### Human µ-opioid receptor radioligand assay

Transfected CHO-K1 cell membranes (20 µg) were incubated with [³H]-DAMGO (1 nM) in assay buffer containing Tris-HCl 50 mM, MgCl₂ 5 mM at pH 7.4. NBS (non-specific binding) was measured by adding 10 µM Naloxone. The binding of the test compound was measured at either one concentration (% inhibition at 1 or 10 µM) or five different concentrations to determine affinity values (Ki). Plates were incubated at 27 °C for 60 min. After the incubation period, the reaction mixture was then transferred to MultiScreen HTS, FC plates (Millipore), filtered and plates were washed 3 times with ice-cold 10 mM Tris-HCl (pH 7.4). Filters were dried and counted at approximately 40% efficiency in a MicroBeta scintillation counter (Perkin-Elmer) using EcoScint liquid scintillation cocktail.

### Results:

As this invention is aimed at providing a compound or a chemically related series of compounds which act as dual ligands of the α₂δ subunit of voltage-gated calcium channels and the µ-opioid receptor it is a very preferred embodiment in which the compounds are selected which act as dual ligands of the α₂δ subunit of voltage-gated calcium channels and the µ-opioid receptor and especially compounds which have a binding expressed as Kᵢ responding to the following scales:
Kᵢ(µ) is preferably < 1000 nM, more preferably < 500 nM, even more preferably < 100 nM.

Kᵢ(α₂δ-1) is preferably < 10000 nM, more preferably < 5000 nM, or even more preferably < 500 nM.

The following scale has been adopted for representing the binding to µ-opioid receptor expressed as Kᵢ:
+ Kᵢ (µ) > 500 nM
++ 100 nM <= Kᵢ(µ) <= 500 nM
+++ Kᵢ(µ) < 100 nM

Preferably, when Kᵢ (µ) > 500 nM, the following scale has been adopted for representing the binding to the µ -receptor:
+ Kᵢ (µ) > 500 nM or inhibition ranges between 1% and 50 %.

The following scale has been adopted for representing the binding to the α₂δ-1 subunit of voltage-gated calcium channels expressed as Kᵢ:
+ Kᵢ(α₂δ-1) >5000 nM
++ 500nM <= Kᵢ(α₂δ-1) <=5000 nM
+++ Kᵢ(α₂δ-1) <500 nM

Preferably, when Kᵢ(α₂δ-1) > 5000 nM, the following scale has been adopted for representing the binding to the α₂δ-1 subunit of voltage-gated calcium channels:
+ Kᵢ(α₂δ-1) > 5000 nM or inhibition ranges between 1 % and 50 %

All compounds prepared in the present application exhibit binding to the α₂δ-1 subunit of voltage-gated calcium channels and the µ-opioid receptor, in particular the following binding results are shown:

| **Example** | **µ-binding** | **α2δ-1 binding** |
|---|---|---|
| **1** | +++ | + |
| **2** | +++ | ++ |
| **3** | +++ | ++ |
| **4** | +++ | ++ |
| **5** | +++ | + |
| **6** | +++ | +++ |
| **7** | +++ | +++ |
| **8** | +++ | +++ |
| **9** | +++ | ++ |
| **10** | +++ | +++ |
| **11** | +++ | ++ |
| **12** | ++ | +++ |
| **13** | ++ | + |
| **14** | + | +++ |
| **15** | +++ | +++ |
| **16** | +++ | +++ |
| **17** | +++ | + |
| **18** | ++ | ++ |
| **19** | +++ | + |
| **20** | +++ | +++ |
| **21** | +++ | +++ |
| **22** | +++ | ++ |
| **23** | +++ | +++ |
| **24** | +++ | ++ |
| **25** | +++ | + |
| **26** | +++ | + |
| **27** | +++ | + |
| **28** | +++ | + |
| **29** | +++ | ++ |
| **30** | +++ | + |
| **31** | ++ | +++ |
| **32** | +++ | ++ |
| **33** | +++ | +++ |
| **34** | +++ | +++ |
| **35** | ++ | ++ |
| **36** | +++ | ++ |
| **37** | +++ | +++ |
| **38** | +++ | +++ |
| **39** | +++ | ++ |
| **40** | +++ | +++ |
| **41** | +++ | +++ |
| **42** | +++ | ++ |

## Claims

1. Compound of general formula (I), 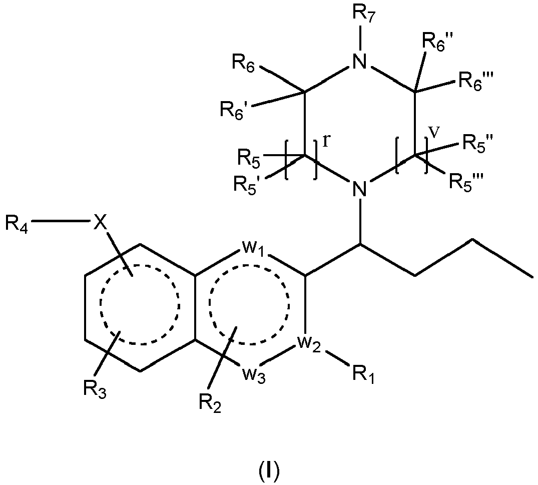 wherein
X is selected from the group consisting of a bond and -[C(RₐR_{b})]ₚ-;
Rₐ is selected from the group consisting of hydrogen, substituted or unsubstituted C₁₋₆ alkyl, substituted or unsubstituted C₂₋₆ alkenyl and substituted or unsubstituted C₂₋₆ alkynyl;
R_{b} is selected from the group consisting of hydrogen, substituted or unsubstituted C₁₋₆ alkyl, substituted or unsubstituted C₂₋₆ alkenyl and substituted or unsubstituted C₂₋₆ alkynyl;
alternatively, Rₐ and R_{b}, taken together with the carbon atom to which they are attached, form a substituted or unsubstituted cycloalkyl;
p is 0, 1, 2, 3, 4 or 5;
w₁ is nitrogen or carbon;
w₂ is nitrogen or - carbon;
w₃ is nitrogen or carbon;
with the proviso that when w₃ is =CH- substituted with -OR₂₁ with R₂₁ being hydrogen, then w₂ is carbon;
r is 0, 1 or 2;
v is 0, 1 or 2;
R₁ is selected from the group consisting of hydrogen, substituted or unsubstituted C₁₋₆ alkyl, substituted or unsubstituted C₂₋₆ alkenyl and substituted or unsubstituted C₂₋₆ alkynyl;
R₂ is selected from the group consisting of -R₂₁, -OR₂₁, -NO₂, halogen, -NR₂₁R₂₁',-NR₂₁C(O)R₂₁', -NR₂₁S(O)₂R₂₁', -S(O)₂NR₂₁R₂₁', - NR₂₁C(O)NR₂₁'R₂₁", -SR₂₁, -S(O)R₂₁, -S(O)₂R₂₁, -CN, haloalkyl, haloalkoxy, -C(O)OR₂₁, -C(O)NR₂₁R₂₁', -OCH₂CH₂OR₂₁,-NR₂₁S(O)₂NR₂₁'R₂₁" and -C(CH₃)₂OR₂₁;
wherein R₂₁, R₂₁' and R₂₁" are independently selected from the group consisting of hydrogen, substituted or unsubstituted C₁₋₆ alkyl, substituted or unsubstituted C₂₋₆ alkenyl, substituted or unsubstituted C₂₋₆ alkynyl and substituted or unsubstituted alkylcycloalkyl;
R₃ is selected from the group consisting of -R₃₁, -OR₃₁, -NO₂, halogen, -NR₃₁R₃₁',-NR₃₁C(O)R₃₁', -NR₃₁S(O)₂R₃₁', -S(O)₂NR₃₁R₃₁', - NR₃₁C(O)NR₃₁'R₃₁", -SR₃₁ , -S(O)R₃₁, -S(O)₂R₃₁, -CN, haloalkyl, haloalkoxy, -C(O)OR₃₁, -C(O)NR₃₁R₃₁', -OCH₂CH₂OR₃₁,-NR₃₁S(O)₂NR₃₁'R₃₁" and -C(CH₃)₂OR₃₁;
wherein R₃₁, R₃₁' and R₃₁" are independently selected from the group consisting of hydrogen, substituted or unsubstituted C₁₋₆ alkyl, substituted or unsubstituted C₂₋₆ alkenyl and substituted or unsubstituted C₂₋₆ alkynyl;
R₄ is 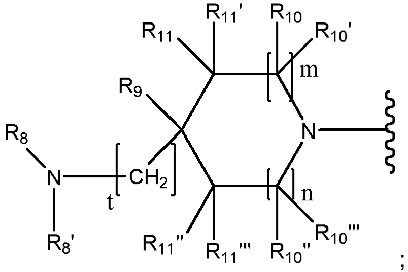
m is 0, 1 or 2;
n is 0, 1 or 2;
t is 0, 1 or 2;
R₅, R₅', R₅" and R₅'" are independently selected from the group consisting of hydrogen, halogen, substituted or unsubstituted C₁₋₆ alkyl, substituted or unsubstituted C₂₋₆ alkenyl and substituted or unsubstituted C₂₋₆ alkynyl;
alternatively, R₅ and R₅' and/or R₅" and R₅'" taken together with the carbon atom to which they are attached form a carbonyl group;
R₆, R₆', R₆" and R₆'" are independently selected from the group consisting of hydrogen, substituted or unsubstituted C₁₋₆ alkyl, substituted or unsubstituted C₂₋₆ alkenyl and substituted or unsubstituted C₂₋₆ alkynyl;
R₇ is selected from the group consisting of hydrogen, substituted or unsubstituted C₁₋₆ alkyl, substituted or unsubstituted C₂₋₆ alkenyl and substituted or unsubstituted C₂₋₆ alkynyl;
R₈ is selected from the group consisting of substituted or unsubstituted C₁₋₆ alkyl, substituted or unsubstituted C₂₋₆ alkenyl, substituted or unsubstituted C₂₋₆ alkynyl, substituted or unsubstituted cycloalkyl and substituted or unsubstituted alkylcycloalkyl;
R₈' is selected from the group consisting of hydrogen, substituted or unsubstituted C₁₋₆ alkyl, substituted or unsubstituted C₂₋₆ alkenyl, substituted or unsubstituted C₂₋₆ alkynyl, substituted or unsubstituted cycloalkyl and substituted or unsubstituted alkylcycloalkyl;
alternatively, R₈ and R₈' taken together with the nitrogen atom to which they are attached form a substituted or unsubstituted heterocyclyl;
Rg is selected from the group consisting of substituted or unsubstituted C₁₋₆ alkyl, substituted or unsubstituted C₂₋₆ alkenyl, substituted or unsubstituted C₂₋₆ alkynyl, substituted or unsubstituted cycloalkyl, substituted or unsubstituted aryl, substituted or unsubstituted heterocyclyl, substituted or unsubstituted alkylcycloalkyl, substituted or unsubstituted alkylaryl and substituted or unsubstituted alkylheterocyclyl;
R₁₀, R₁₀', R₁₀" and R₁₀'" are independently selected from the group consisting of hydrogen, halogen, substituted or unsubstituted C₁₋₆ alkyl, substituted or unsubstituted C₂₋₆ alkenyl and substituted or unsubstituted C₂₋₆ alkynyl;
alternatively, R₁₀ and R₁₀' and/or R₁₀" and R₁₀'" taken together with the carbon atom to which they are attached form a substituted or unsubstituted cycloalkyl;
alternatively, R₁₀ and R₁₀' and/or R₁₀" and R₁₀'" taken together with the carbon atom to which they are attached form a carbonyl group;
R₁₁, R₁₁', R₁₁" and R₁₁'" are independently selected from the group consisting of hydrogen, halogen, substituted or unsubstituted C₁₋₆ alkyl, substituted or unsubstituted C₂₋₆ alkenyl and substituted or unsubstituted C₂₋₆ alkynyl;
alternatively, R₁₁ and R₁₁' and/or R₁₁" and R₁₁'" taken together with the carbon atom to which they are attached form a substituted or unsubstituted cycloalkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

2. Compound according to claim 1, wherein the compound of Formula (I) is a compound of Formula (I')

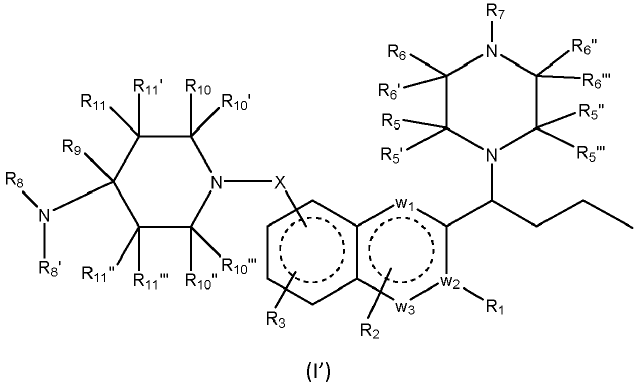

3. Compound according to claim 1, wherein the compound of Formula (I) is a compound of Formula (I²')

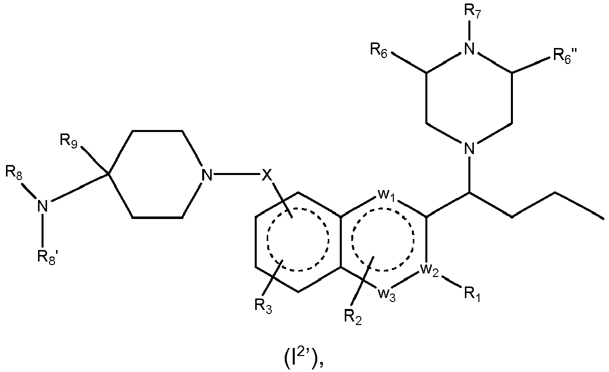

4. Compound according to claim 1, wherein the compound of Formula (I) is a compound of Formula (I³') 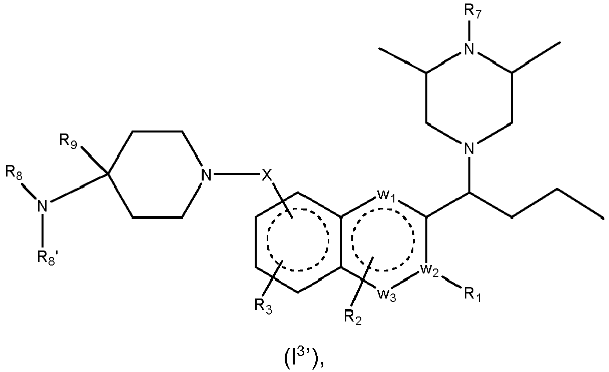 preferably, a compound of Formula (I⁴')

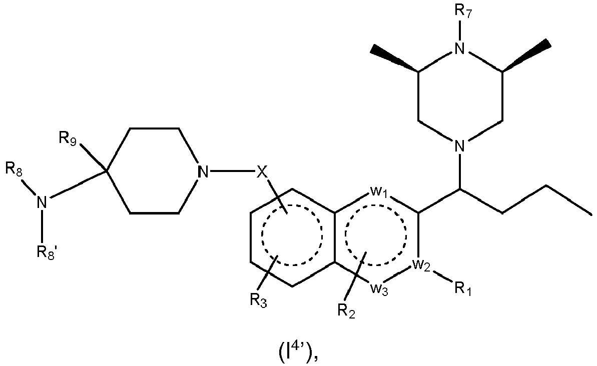

5. Compound according to claim 1, wherein the compound of Formula (I) is a compound of Formula (I⁵') 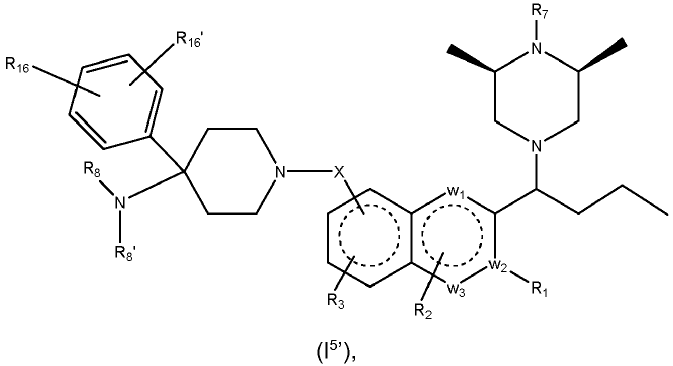 wherein R₁₆ and R₁₆' are independently selected from halogen, -R₉₁, -OR₉₁,-NO₂, -NR₉₁R₉₁', -NR₉₁C(O)R₉₁', -NR₉₁S(O)₂R₉₁', -S(O)₂NR₉₁R₉₁',-NR₉₁C(O)NR₉₁'R₉₁", -SR₉₁, -S(O)R₉₁, -S(O)₂R₉₁, -CN, haloalkyl, haloalkoxy,-C(O)OR₉₁, -OC(O)R₉₁, -C(O)NR₉₁R₉₁', -OCH₂CH₂OR₉₁, -NR₉₁S(O)₂NR₉₁'R₉₁" and -C(CH₃)₂OR₉₁;
wherein R₉₁, R₉₁' and R₉₁" are independently selected from the group consisting of the group consisting of hydrogen, substituted or unsubstituted C₁₋₆ alkyl, substituted or unsubstituted C₂₋₆ alkenyl, and substituted or unsubstituted C₂₋₆ alkynyl;

6. Compound according to claim 5, wherein R₁₆ is selected from hydrogen and - OR₉₁, preferably from hydrogen and -OH, while R₁₆' is hydrogen.

7. Compound according to claim 5 or 6, wherein R₁₆ is selected from hydrogen and -OR₉₁ in meta position, preferably from hydrogen and -OH in meta position, while R₁₆' is hydrogen.

8. Compound according to any one of claims 5 to 7, wherein the compound of Formula (I) is a compound of Formula (I⁶') or (I⁷')

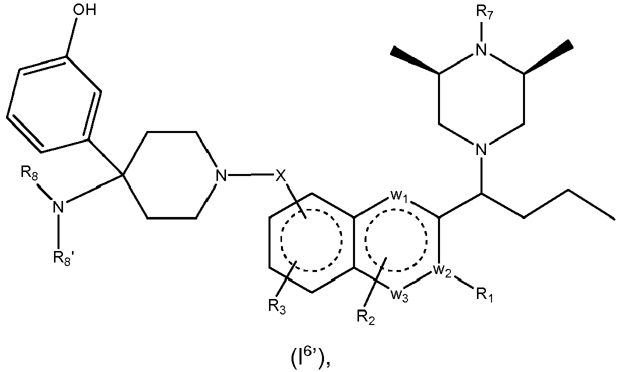

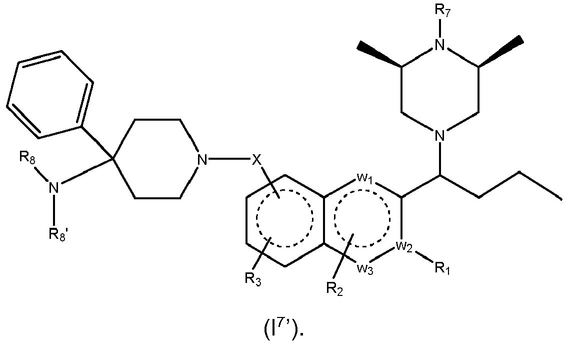

9. Compound according to any one of claims 1 to 8, wherein X is selected from the group consisting of a bond and -[C(RₐR_{b})]ₚ-; preferably X is a bond or -CH₂-.

10. Compound according to any one of claims 1 to 9 wherein the compound is selected from
| | |
|---|---|
| **1** | 3-(4-(Dimethylamino)-1-(6-(1-((3*S*,5*R*)-3,5-dimethylpiperazin-1-yl)butyl)naphthalen-2-yl)piperidin-4-yl)phenol |
| **2** | 3-(4-(Dimethylamino)-1-(3-(1-((3*S*,5*R*)-3,5-dimethylpiperazin-1-yl)butyl)quinolin-7-yl)piperidin-4-yl)phenol |
| **3** | 3-(4-(Dimethylamino)-1-(2-(1-((3*S*,5*R*)-3,5-dimethylpiperazin-1-yl)butyl)quinolin-6-yl)piperidin-4-yl)phenol |
| **4** | 3-(4-(Dimethylamino)-1-(3-((*R*)-1-((3*S*,5*R*)-3,5-dimethylpiperazin-1-yl)butyl)quinolin-7-yl)piperidin-4-yl)phenol |
| **5** | 3-(4-(Dimethylamino)-1-(3-((*S*)-1-((3*S*,5*R*)-3,5-dimethylpiperazin-1-yl)butyl)quinolin-7-yl)piperidin-4-yl)phenol |
| **6** | 3-(4-(Dimethylamino)-1-((3-(1-((3*S*,5*R*)-3,5-dimethylpiperazin-1-yl)butyl)quinolin-7-yl)methyl)piperidin-4-yl)phenol |
| **7** | 3-(4-(Dimethylamino)-1-((2-(1-((3*S*,5*R*)-3,5-dimethylpiperazin-1-yl)butyl)quinolin-6-yl)methyl)piperidin-4-yl)phenol |
| **8** | 3-(4-(Dimethylamino)-1-((3-((*R*)-1-((3*S*,5*R*)-3,5-dimethylpiperazin-1-yl)butyl)quinolin-7-yl)methyl)piperidin-4-yl)phenol |
| **9** | 3-(4-(Dimethylamino)-1-((3-((*S*)-1-((3*S*,5*R*)-3,5-dimethylpiperazin-1-yl)butyl)quinolin-7-yl)methyl)piperidin-4-yl)phenol |
| **10** | 3-(4-(Dimethylamino)-1-((2-((*R*)-1-((3*S*,5*R*)-3,5-dimethylpiperazin-1-yl)butyl)quinolin-6-yl)methyl)piperidin-4-yl)phenol |
| **11** | 3-(4-(Dimethylamino)-1-((2-((*S*)-1-((3*S*,5*R*)-3,5-dimethylpiperazin-1-yl)butyl)quinolin-6-yl)methyl)piperidin-4-yl)phenol |
| **12** | 1-((3-((*R*)-1-((3*S*,5*R*)-3,5-Dimethylpiperazin-1-yl)butyl)quinolin-7-yl)methyl)-*N*,*N*-dimethyl-4-phenylpiperidin-4-amine |
| **13** | 1-((3-((*S*)-1-((3*S*,5*R*)-3,5-Dimethylpiperazin-1-yl)butyl)quinolin-7-yl)methyl)-*N*,*N*-dimethyl-4-phenylpiperidin-4-amine |
| **14** | 3-(4-(Dimethylamino)-1-(3-(1-((3*S*,5*R*)-3,5-dimethylpiperazin-1-yl)butyl)quinolin-6-yl)piperidin-4-yl)phenol |
| **15** | 3-(4-(Dimethylamino)-1-((3-(1-((3*S*,5*R*)-3,5-dimethylpiperazin-1-yl)butyl)quinolin-6-yl)methyl)piperidin-4-yl)phenol |
| **16** | 3-(4-(Dimethylamino)-1-((3-((*R*)-1-((3*S*,5*R*)-3,5-dimethylpiperazin-1-yl)butyl)quinolin-6-yl)methyl)piperidin-4-yl)phenol |
| **17** | 3-(4-(Dimethylamino)-1-((3-((*S*)-1-((3*S*,5*R*)-3,5-dimethylpiperazin-1-yl)butyl)quinolin-6-yl)methyl)piperidin-4-yl)phenol |
| **18** | 1-((3-((*R*)-1-((3*S*,5*R*)-3,5-Dimethylpiperazin-1-yl)butyl)quinolin-6-yl)methyl)-*N*,*N*-dimethyl-4-phenylpiperidin-4-amine |
| **19** | 1-((3-((*S*)-1-((3*S*,5*R*)-3,5-Dimethylpiperazin-1-yl)butyl)quinolin-6-yl)methyl)-*N*,*N*-dimethyl-4-phenylpiperidin-4-amine |
| **20** | 3-(4-(Dimethylamino)-1-(3-(1-((3*S*,5*R*)-3,5-dimethylpiperazin-1-yl)butyl)-2-ethylquinolin-7-yl)piperidin-4-yl)phenol |
| **21** | 3-(4-(Dimethylamino)-1-((3-(1-((3*S*,5*R*)-3,5-dimethylpiperazin-1-yl)butyl)-2-ethylquinolin-7-yl)methyl)piperidin-4-yl)phenol |
| **22** | 3-(4-(Dimethylamino)-1-((3-(1-((3*S*,5*R*)-3,5-dimethylpiperazin-1-yl)butyl)-2-ethylquinolin-6-yl)methyl)piperidin-4-yl)phenol |
| **23** | 3-(4-(Dimethylamino)-1-((3-((*R*)-1-((3*S*,5*R*)-3,5-dimethylpiperazin-1-yl)butyl)-2-ethylquinolin-7-yl)methyl)piperidin-4-yl)phenol |
| **24** | 3-(4-(Dimethylamino)-1-((3-((*S*)-1-((3*S*,5*R*)-3,5-dimethylpiperazin-1-yl)butyl)-2-ethylquinolin-7-yl)methyl)piperidin-4-yl)phenol |
| **25** | 3-(4-(Dimethylamino)-1-(2-(1-((3*S*,5*R*)-3,5-dimethylpiperazin-1-yl)butyl)quinazolin-6-yl)piperidin-4-yl)phenol |
| **26** | 3-(4-(Dimethylamino)-1-(2-((*R*)-1-((3*S*,5*R*)-3,5-dimethylpiperazin-1-yl)butyl)quinazolin-6-yl)piperidin-4-yl)phenol |
| **27** | 3-(4-(Dimethylamino)-1-(2-((*S*)-1-((3*S*,5*R*)-3,5-dimethylpiperazin-1-yl)butyl)quinazolin-6-yl)piperidin-4-yl)phenol |
| **28** | 3-(4-(Dimethylamino)-1-((2-(1-((3*S*,5*R*)-3,5-dimethylpiperazin-1-yl)butyl)quinazolin-6-yl)methyl)piperidin-4-yl)phenol |
| **29** | 3-(4-(Dimethylamino)-1-((2-((*R*)-1-((3*S*,5*R*)-3,5-dimethylpiperazin-1-yl)butyl)quinazolin-6-yl)methyl)piperidin-4-yl)phenol |
| **30** | 3-(4-(Dimethylamino)-1-((2-((*S*)-1-((3*S*,5*R*)-3,5-dimethylpiperazin-1-yl)butyl)quinazolin-6-yl)methyl)piperidin-4-yl)phenol |
| **31** | 1-(2-(1-((3*S*,5*R*)-3,5-Dimethylpiperazin-1-yl)butyl)-4-methoxyquinolin-6-yl)-*N*,*N*-dimethyl-4-phenylpiperidin-4-amine |
| **32** | 3-(4-(Dimethylamino)-1-(2-(1-((3*S*,5*R*)-3,5-dimethylpiperazin-1-yl)butyl)quinoxalin-6-yl)piperidin-4-yl)phenol |
| **33** | 3-(4-(Dimethylamino)-1-(3-(1-((3*S*,5*R*)-3,5-dimethylpiperazin-1-yl)butyl)quinoxalin-6-yl)piperidin-4-yl)phenol |
| **34** | 3-(4-(Dimethylamino)-1-(3-((*R*)-1-((3*S*,5*R*)-3,5-dimethylpiperazin-1-yl)butyl)quinoxalin-6-yl)piperidin-4-yl)phenol |
| **35** | 3-(4-(Dimethylamino)-1-(3-((*S*)-1-((3*S*,5*R*)-3,5-dimethylpiperazin-1-yl)butyl)quinoxalin-6-yl)piperidin-4-yl)phenol |
| **36** | 3-(4-(Dimethylamino)-1-((2-(1-((3*S*,5*R*)-3,5-dimethylpiperazin-1-yl)butyl)quinoxalin-6-yl)methyl)piperidin-4-yl)phenol |
| **37** | 3-(4-(Dimethylamino)-1-((3-(1-((3*S*,5*R*)-3,5-dimethylpiperazin-1-yl)butyl)quinoxalin-6-yl)methyl)piperidin-4-yl)phenol |
| **38** | 3-(4-(Dimethylamino)-1-((3-((*R*)-1-((3*S*,5*R*)-3,5-dimethylpiperazin-1-yl)butyl)quinoxalin-6-yl)methyl)piperidin-4-yl)phenol |
| **39** | 3-(4-(Dimethylamino)-1-((3-((*S*)-1-((3*S*,5*R*)-3,5-dimethylpiperazin-1-yl)butyl)quinoxalin-6-yl)methyl)piperidin-4-yl)phenol |
| **40** | 3-(4-(Dimethylamino)-1-((2-(1-((3*S*,5*R*)-3,5-dimethylpiperazin-1-yl)butyl)-3-ethylquinoxalin-6-yl)methyl)piperidin-4-yl)phenol |
| **41** | 3-(4-(Dimethylamino)-1-((2-((*R*)-1-((3*S*,5*R*)-3,5-dimethylpiperazin-1-yl)butyl)-3-ethylquinoxalin-6-yl)methyl)piperidin-4-yl)phenol |
| **42** | 3-(4-(Dimethylamino)-1-((2-((*S*)-1-((3*S*,5*R*)-3,5-dimethylpiperazin-1-yl)butyl)-3-ethylquinoxalin-6-yl)methyl)piperidin-4-yl)phenol |

11. Process for the preparation of compounds of Formula (I) as defined in any one of claims 1 to 10, said process comprises:
a) reacting a compound of formula VII wherein T is -X-R₄ 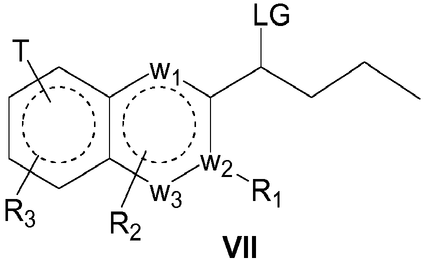 with a suitable amine of formula XI, 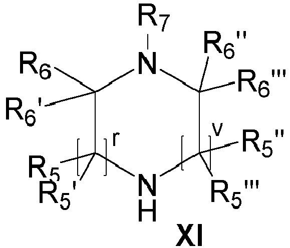 in a suitable solvent, such as acetonitrile, dichloromethane or dimethylformamide, in the presence of a base such as triethylamine, K₂CO₃ or *N*,*N*-diisopropylethylamine, at a suitable temperature comprised between room temperature and the reflux temperature, preferably heating, wherein R₁, R₂, R₃, R₄, R₅, R₅', R₅", R₅"', R₆, R₆', R₆", R₆'", R₇, X, w₁, w₂, w₃, r and v have the meanings as defined in the preceding claims;
or
b) wherein w₁ is nitrogen and w₂ and w₃ are carbon, said process comprises reacting a compound of formula XIV 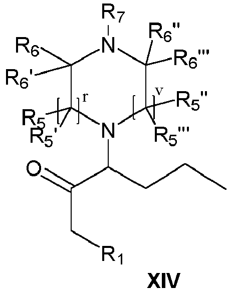 with an aminoaldehyde of formula XIII, 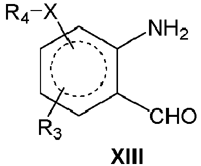 in the presence of a base, such as potassium hydroxide, in a suitable solvent, such as ethanol, at a suitable temperature, preferably heating, wherein R₁, R₂, R₃, R₄, R₅, R₅', R₅", R₅"', R₆, R₆', R₆", R₆'", R₇, X, w₁, w₂, w₃, r and v have the meanings as defined in the preceding claims;
or
c) when X is a bond, said process comprises reacting a compound of formula XII wherein T is halogen, 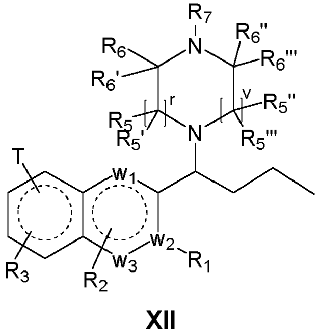 with a compound of formula XVI 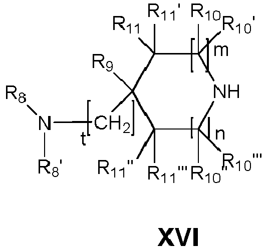 under Buchwald-Hartwig arylation conditions, using a Pd catalyst such as tris(dibenzylideneacetone)dipalladium(0) or palladium acetate, and a suitable ligand, preferably a phosphine ligand such as DavePhos, XPhos or BINAP, using a suitable base such as sodium *tert*-butoxide or cesium carbonate, in a suitable solvent such as toluene or 1,4-dioxane, at a suitable temperature, preferably heating, wherein R₁, R₂, R₃, R₄, R₅, R₅', R₅", R₅"', R₆, R₆', R₆", R₆'", R₇, R₈, R₈', R₉, R₁₀, R₁₀', R₁₀", R₁₀"', R₁₁, R₁₁', R₁₁", R₁₁", X, w₁, w₂, w₃, n, m, t, r and v have the meanings as defined in the preceding claims;
or
d) wherein X is -CH₂-, said process comprises reacting a compound of formula XII wherein T is halogen, 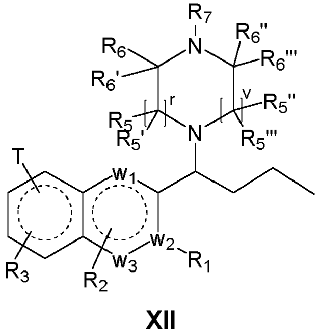 with a compound of formula XVII 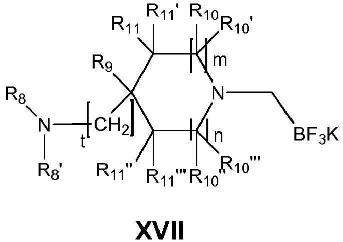 by coupling using a Pd catalyst such as palladium acetate, using a suitable base such as cesium carbonate, and a suitable ligand, preferably a phosphine ligand such as XPhos, in a suitable solvent such as mixtures of dioxane and water, at a suitable temperature, preferably heating and optionally under microwave irradiation, wherein R₁, R₂, R₃, R₄, R₅, R₅', R₅", R₅"', R₆, R₆', R₆", R₆'", R₇, R₈, R₈', R₉, R₁₀, R₁₀', R₁₀", R₁₀"', R₁₁, R₁₁', R₁₁", R₁₁"', X, w₁, w₂, w₃, n, m, t, r and v have the meanings as defined in the preceding claims.

12. Use of the compounds of Formula II, III, IV, V, VI, VII, VIII, IX, X, XI, XII, XIII, XIV, XV, XVI or XVII 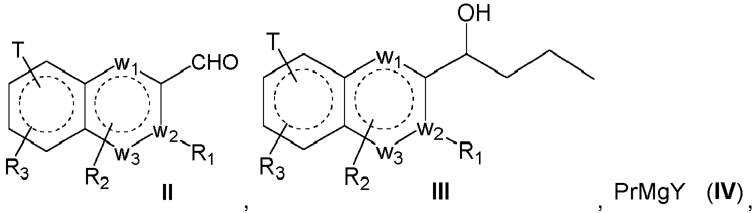 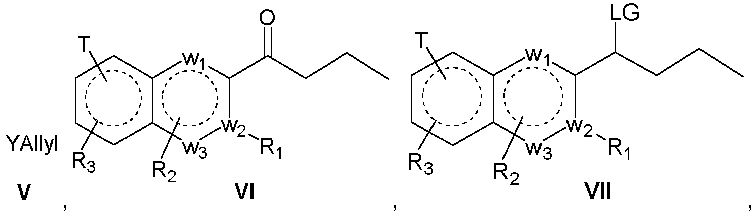 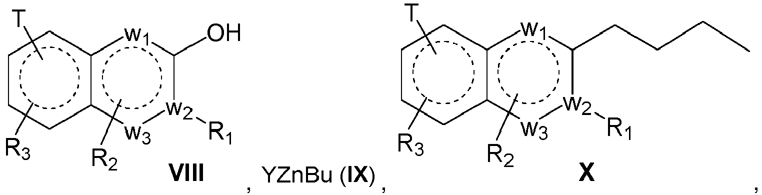 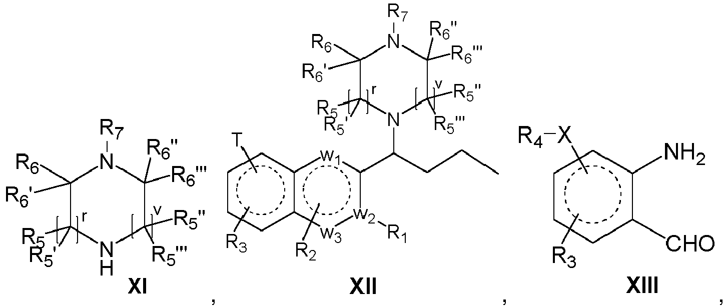 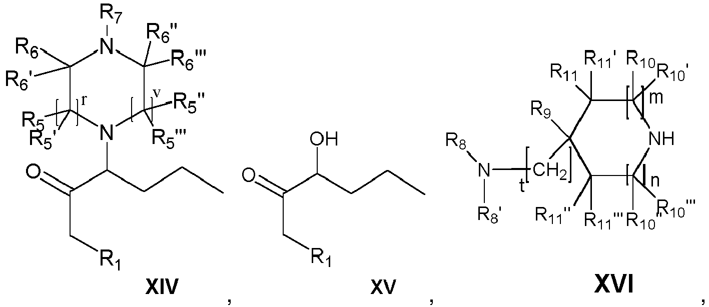 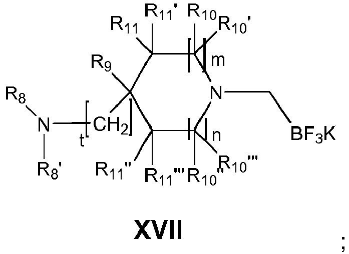 wherein LG represents a leaving group (such as chloro, bromo, iodo, mesylate, tosylate, nosylate or triflate), Y represents a halogen atom, T represents a halogen atom or a group XR₄, and R₁, R₂, R₃, R₄, R₅, R₅', R₅", R₅"', R₆, R₆', R₆", R₆'", R₇, R₈, R₈', R₉, R₁₀, R₁₀', R₁₀", R₁₀''' R₁₁, R₁₁', R₁₁", R₁₁"', X, w₁, w₂, w₃, n, m, t, r and v have the meanings as defined in the preceding claims, for the preparation of compounds of Formula (I).

13. A pharmaceutical composition which comprises a compound of Formula (I) as defined in any one of claims 1 to 10 or a pharmaceutically acceptable salt thereof, and a pharmaceutically acceptable carrier, adjuvant or vehicle.

14. A compound of Formula (I) as defined in any one of claims 1 to 10 or a pharmaceutical composition as defined in claim 13 for use as a medicament.

15. A compound of Formula (I) as defined in any one of claims 1 to 10 or a pharmaceutical composition as defined in claim 13 for use as a medicament for the treatment of pain, especially medium to severe pain, visceral pain, chronic pain, cancer pain, migraine, inflammatory pain, acute pain or neuropathic pain, allodynia or hyperalgesia.
